# EUROPEAN PATENT APPLICATION

(11) **EP 2 801 619 A1**
(43) Date of publication of application: **12.11.2014**
(21) Application number: 13167340.2
(22) Date of filing: 10.05.2013
(51) Int. Cl.: C12N 15/82, C07K 14/415, A01H 5/00

(54) **Method of enhancing photosynthesis using Curvature thylakoid 1 (CURT1) polypeptide**

(71) Applicant: Fondazione Edmund Mach, 38010 San Michele all'Adige (IT)
(72) Inventor: Leister, Dario Michael, 82319 Starnberg (DE); Armbruster, Ute, El Cerrito, CA 94530 (US)
(74) Representative: Lahrtz, Fritz

(57) **Abstract**

The present invention refers to a method of enhancing photosynthesis of a photosynthetic eukaryote by transforming a cell of the photosynthetic eukaryote or the photosynthetic eukaryote with a polynucleotide encoding a CURT1 polypeptide or by increasing the expression of an endogenous CURT1 polynucleotide. The present invention also relates to an organism transformed with a polynucleotide encoding a CURT1 polypeptide or having an increased expression of an endogenous CURS1 polynucleotide. Such an organism may be a photosynthetic eukaryote or a cyanobacterium.

SEQUENCE LISTING

<110>  Fondazione Edmund Mach

<120>  Method of enhancing photosynthesis using Curvature thylakoid 1
       (CURT1) polypeptide

<130>  M68359EP

<160>  79

<170>  PatentIn version 3.5

<210>  1
<211>  495
<212>  DNA
<213>  Arabidopsis thaliana

<400>  1
atggcgatat cagtagcagc ttcgtcctct atggccgtga tggttccacg tgttcccgcc      60

gtatccaccc gttgctccgc cgtcccttat cttcctcctc gctcctttgg ccgatcctct     120

ttcaccgttc cgttgaagct tgtttcaggg aatggattgc aaaaagttga attgttgaag     180

acaagagctt cttcagaaga gacctcgtcc attgatacca acgaactcat cacagatttg     240

aaggaaaagt gggatggtct tgagaacaaa tctactgtac ttatatatgg aggaggagcc     300

attgttgctg tttggttatc ttccattgtt gttggtgcca tcaactctgt tcctctgctt     360

ccgaaagtta tggaacttgt cggtctcggg tacactggat ggtttgtcta cagatacctt     420

ctcttcaagt caagcagaaa ggaattggct gaggatattg aatccttgaa gaagaagatc     480

gcaggaagcg aatag                                                      495


<210>  2
<211>  525
<212>  DNA
<213>  Arabidopsis thaliana

<400>  2
atggcttctc tttcagtctc ttcctcttca accatcatcg attcaagagc tcctccttct      60

cgactagcct ccgcctccgc ctcttctccg tcgtgcattt cacttcccac acttccgatt     120

cagtctcata cccgtgccgc taaagccact gcttactgtc ggaagattgt gaggaacgtt     180

gtgacgagag ctactactga agttggtgaa gctcctgcca ctactaccga agctgagact     240

actgagttac ctgaaatcgt caagactgct caagaagctt gggagaaagt ggatgacaag     300

tacgctattg gttctcttgc ctttgctggt gtagtggctc tttggggttc tgctggaatg     360

atttcggcaa tcgataggct tccattggtt cctggtgttc ttgaacttgt aggcatcggt     420

tacacaggat ggttcactta caagaacctg gtcttcaaac cagacaggga ggctttgttt     480

gagaaggtca agagcacata caaagacata ttagggagca gctga                     525

<210>  3
<211>  471
<212>  DNA
<213>  Arabidopsis thaliana

<400>  3
atggcttcaa tttctgcaac tttgccttcg ccattgttac tcacacagag aaaatccaat      60

ctcacatcga ttcaaaaact cccattttct ctaactcgag gtacgaatga tctttctcca     120

ttatctctta ctcgaaaccc tagcagcatc agtctgatgg tgaaagctag tggagaaagc     180

tcagattcat cgactgatct cgacgttgtt agtacgattc agaatgtttg ggataagtct     240

gaagataggt taggtcttat tggtttgggt tttgctggta ttgtagctct ttgggcatca     300

ttgaatctca tcacggcaat cgacaaattg cccgttatct cgagcggatt cgaactagtt     360

ggtatcttgt tctccacgtg gttcacatat cgatatctct tgttcaaacc ggacagacag     420

gagctttcga aaattgtcaa gaaatcagta gcggatatac ttggccagtg a              471


<210>  4
<211>  582
<212>  DNA
<213>  Arabidopsis thaliana

<400>  4
atggagctct gcaccaggtc taccacaatc atcactcatc tccctgcgag cttcaacggc      60

catggatatc tagcgggaaa atctgttgat agaatctcac ttcctctcca gcgaaacgtt     120

gcgtctctcg ttcttcaatc ccgcacgttg cgttgtagta gaaagtttcc aggagaaaca     180

gttactgaag aaacatcaac aggagtcaat gagtttggtg tggaggatcg tgatggagta     240

gttgttgcag ccgaagagaa aaactcgaat agtgaagctc ctcaagctga agatgaggaa     300

actcaagccc tagagttttt gaacgatatt aagctggact cagacaagac atattcaatc     360

ctgctctatg ggagtggtgc gatagttgct ctctacttaa cgtctgcaat cgtcagttca     420

ctcgaagcaa tccctctgtt tcctaagcta atggaagtag ttggtctcgg atacacactc     480

tggttcacta cccgttatct gctatttaag aggaacagag aagaactcaa gacaaaagtt     540

agtgaaatca aaaagcaggt tcttgggtct gatagtgagt ga                        582


<210>  5
<211>  164
<212>  PRT
<213>  Arabidposis thaliana

<400>  5

Met Ala Ile Ser Val Ala Ala Ser Ser Ser Met Ala Val Met Val Pro
1               5                   10                  15


Arg Val Pro Ala Val Ser Thr Arg Cys Ser Ala Val Pro Tyr Leu Pro
            20                  25                  30

Pro Arg Ser Phe Gly Arg Ser Ser Phe Thr Val Pro Leu Lys Leu Val
        35                  40                  45

Ser Gly Asn Gly Leu Gln Lys Val Glu Leu Leu Lys Thr Arg Ala Ser
    50                  55                  60

Ser Glu Glu Thr Ser Ser Ile Asp Thr Asn Glu Leu Ile Thr Asp Leu
65                  70                  75                  80

Lys Glu Lys Trp Asp Gly Leu Glu Asn Lys Ser Thr Val Leu Ile Tyr
                85                  90                  95

Gly Gly Gly Ala Ile Val Ala Val Trp Leu Ser Ser Ile Val Val Gly
            100                 105                 110

Ala Ile Asn Ser Val Pro Leu Leu Pro Lys Val Met Glu Leu Val Gly
        115                 120                 125

Leu Gly Tyr Thr Gly Trp Phe Val Tyr Arg Tyr Leu Leu Phe Lys Ser
    130                 135                 140

Ser Arg Lys Glu Leu Ala Glu Asp Ile Glu Ser Leu Lys Lys Lys Ile
145                 150                 155                 160

Ala Gly Ser Glu

<210>  6
<211>  174
<212>  PRT
<213>  Arabidopsis thaliana

<400>  6

Met Ala Ser Leu Ser Val Ser Ser Ser Ser Thr Ile Ile Asp Ser Arg
1               5                   10                  15

Ala Pro Pro Ser Arg Leu Ala Ser Ala Ser Ala Ser Ser Pro Ser Cys
            20                  25                  30

Ile Ser Leu Pro Thr Leu Pro Ile Gln Ser His Thr Arg Ala Ala Lys
        35                  40                  45

Ala Thr Ala Tyr Cys Arg Lys Ile Val Arg Asn Val Val Thr Arg Ala
    50                  55                  60

Thr Thr Glu Val Gly Glu Ala Pro Ala Thr Thr Thr Glu Ala Glu Thr
65                  70                  75                  80

Thr Glu Leu Pro Glu Ile Val Lys Thr Ala Gln Glu Ala Trp Glu Lys
                85                  90                  95

Val Asp Asp Lys Tyr Ala Ile Gly Ser Leu Ala Phe Ala Gly Val Val
            100                 105                 110

Ala Leu Trp Gly Ser Ala Gly Met Ile Ser Ala Ile Asp Arg Leu Pro
        115                 120                 125

Leu Val Pro Gly Val Leu Glu Leu Val Gly Ile Gly Tyr Thr Gly Trp
    130                 135                 140

Phe Thr Tyr Lys Asn Leu Val Phe Lys Pro Asp Arg Glu Ala Leu Phe
145                 150                 155                 160

Glu Lys Val Lys Ser Thr Tyr Lys Asp Ile Leu Gly Ser Ser
                165                 170

<210>  7
<211>  156
<212>  PRT
<213>  Arabidopsis thaliana

<400>  7

Met Ala Ser Ile Ser Ala Thr Leu Pro Ser Pro Leu Leu Leu Thr Gln
1               5                   10                  15

Arg Lys Ser Asn Leu Thr Ser Ile Gln Lys Leu Pro Phe Ser Leu Thr
            20                  25                  30

Arg Gly Thr Asn Asp Leu Ser Pro Leu Ser Leu Thr Arg Asn Pro Ser
        35                  40                  45

Ser Ile Ser Leu Met Val Lys Ala Ser Gly Glu Ser Ser Asp Ser Ser
    50                  55                  60

Thr Asp Leu Asp Val Val Ser Thr Ile Gln Asn Val Trp Asp Lys Ser
65                  70                  75                  80

Glu Asp Arg Leu Gly Leu Ile Gly Leu Gly Phe Ala Gly Ile Val Ala
                85                  90                  95

Leu Trp Ala Ser Leu Asn Leu Ile Thr Ala Ile Asp Lys Leu Pro Val
            100                 105                 110

Ile Ser Ser Gly Phe Glu Leu Val Gly Ile Leu Phe Ser Thr Trp Phe
        115                 120                 125

Thr Tyr Arg Tyr Leu Leu Phe Lys Pro Asp Arg Gln Glu Leu Ser Lys
    130                 135                 140

Ile Val Lys Lys Ser Val Ala Asp Ile Leu Gly Gln
145                 150                 155

<210>  8
<211>  193
<212>  PRT
<213>  Arabidopsis thaliana

<400>  8

Met Glu Leu Cys Thr Arg Ser Thr Thr Ile Ile Thr His Leu Pro Ala
1               5                   10                  15

Ser Phe Asn Gly His Gly Tyr Leu Ala Gly Lys Ser Val Asp Arg Ile
            20                  25                  30

Ser Leu Pro Leu Gln Arg Asn Val Ala Ser Leu Val Leu Gln Ser Arg
        35                  40                  45

Thr Leu Arg Cys Ser Arg Lys Phe Pro Gly Glu Thr Val Thr Glu Glu
    50                  55                  60

Thr Ser Thr Gly Val Asn Glu Phe Gly Val Glu Asp Arg Asp Gly Val
65                  70                  75                  80

Val Val Ala Ala Glu Glu Lys Asn Ser Asn Ser Glu Ala Pro Gln Ala
                85                  90                  95

Glu Asp Glu Glu Thr Gln Ala Leu Glu Phe Leu Asn Asp Ile Lys Leu
            100                 105                 110

Asp Ser Asp Lys Thr Tyr Ser Ile Leu Leu Tyr Gly Ser Gly Ala Ile
        115                 120                 125

Val Ala Leu Tyr Leu Thr Ser Ala Ile Val Ser Ser Leu Glu Ala Ile
    130                 135                 140

Pro Leu Phe Pro Lys Leu Met Glu Val Val Gly Leu Gly Tyr Thr Leu
145                 150                 155                 160

Trp Phe Thr Thr Arg Tyr Leu Leu Phe Lys Arg Asn Arg Glu Glu Leu
                165                 170                 175

Lys Thr Lys Val Ser Glu Ile Lys Lys Gln Val Leu Gly Ser Asp Ser
            180                 185                 190

Glu

<210> 9
<211> 450
<212> DNA
<213> Synechocystis sp. PCC 6803

<400> 9
gtgggccgta aacattcaat ccgaataata aaggactggt tgaagatgga agaacaaaag 60

acagcaactg ccgggattaa aaccgatgta ggaccaatca ctactcccaa tccccaaaag 120

tccccaatca ccgaccaggc ttggcaggaa tggctacaac cggtgtggga agtgctgggt 180

aaaattcctc aaatgaccgg ggagtttttc gaggacaaca agcaacccct catttccctg 240

ggcataatcc tgttgggcat tatcagcgtc aaaatcttga ttgctgtgct cgatgccatt 300

aatgatattc ctttattggc accaacttta cagttgatcg gcatgggcta caccgcttgg 360

tttatctggc gttacctttg gaaagctgaa aagcgccaag agttggcttc cgaatttggt 420

gcactaaagg agcaaatttt tggcggttag 450

<210> 10
<211> 149
<212> PRT
<213> Synechocystis sp. PCC 6803

<400> 10

Met Gly Arg Lys His Ser Ile Arg Ile Ile Lys Asp Trp Leu Lys Met
1               5                   10                  15

Glu Glu Gln Lys Thr Ala Thr Ala Gly Ile Lys Thr Asp Val Gly Pro
            20                  25                  30

Ile Thr Thr Pro Asn Pro Gln Lys Ser Pro Ile Thr Asp Gln Ala Trp
        35                  40                  45

Gln Glu Trp Leu Gln Pro Val Trp Glu Val Leu Gly Lys Ile Pro Gln
    50                  55                  60

Met Thr Gly Glu Phe Phe Glu Asp Asn Lys Gln Pro Leu Ile Ser Leu
65                  70                  75                  80

Gly Ile Ile Leu Leu Gly Ile Ile Ser Val Lys Ile Leu Ile Ala Val
                85                  90                  95

Leu Asp Ala Ile Asn Asp Ile Pro Leu Leu Ala Pro Thr Leu Gln Leu
            100                 105                 110

Ile Gly Met Gly Tyr Thr Ala Trp Phe Ile Trp Arg Tyr Leu Trp Lys
        115                 120                 125

Ala Glu Lys Arg Gln Glu Leu Ala Ser Glu Phe Gly Ala Leu Lys Glu
    130                 135                 140

Gln Ile Phe Gly Gly
145

<210>  11
<211>  15
<212>  PRT
<213>  Artificial Sequence

<220>
<223>  Description of Artificial Sequnece: Part of synCURT1

<400>  11

Cys Thr Asp Val Gly Pro Ile Thr Thr Pro Asn Pro Gln Lys Ser
1               5                   10                  15

<210>  12
<211>  21
<212>  DNA
<213>  Artificial Sequence

<220>
<223>  Description of Artificial Sequence: CURT1A forward primer

<400>  12
cgtcccggga tggcgatatc a                                                21

<210>  13
<211>  21
<212>  DNA
<213>  Artificial Sequence

<220>
<223>  Description of Artificial Sequence: CURT1A reverse primer

<400>  13
gccagatctt tcgcttcctg c                                                21

<210>  14
<211>  24
<212>  DNA
<213>  Artificial Sequence

<220>
<223>  Description of Artificial Sequence: CURT1A forward primer

<400>  14
cggaattcat ggcttcttca gaag                                             24

<210>  15
<211>  24
<212>  DNA
<213>  Artificial Sequence

<220>
<223>  Description of Artificial Sequence: CURT1A reverse primer

<400>  15
gctctagact attcgcttcc tgcg                                              24


<210>  16
<211>  24
<212>  DNA
<213>  Artificial Sequence

<220>
<223>  Description of Artificial Sequence: CURT1A forward primer

<400>  16
cgccatggcg atatcagtag cagc                                              24


<210>  17
<211>  26
<212>  DNA
<213>  Artificial Sequence

<220>
<223>  Description of Artificial Sequence: CURT1A reverse primer

<400>  17
cgccatggcg cttcctgcga tcttct                                            26


<210>  18
<211>  21
<212>  DNA
<213>  Artificial Sequence

<220>
<223>  Description of Artificial Sequence: CURT1A forward primer

<400>  18
aacaccactg agttggattt c                                                 21


<210>  19
<211>  21
<212>  DNA
<213>  Artificial Sequence

<220>
<223>  Description of Artificial Sequence: CURT1A reverse primer

<400>  19
aacatacaga gagaaagctg g                                                 21


<210>  20
<211>  20
<212>  DNA

<213>  Artificial Sequence

<220>
<223>  Description of Artificial Sequence: CURT1A forward primer

<400>  20
aagaagaagt tctcggcgtg                                                   20


<210>  21
<211>  20
<212>  DNA
<213>  Artificial Sequence

<220>
<223>  Description of Artificial Sequence: CURT1A reverse primer

<400>  21
gacgaggtct cttctgaaga                                                   20


<210>  22
<211>  16
<212>  DNA
<213>  Artificial Sequence

<220>
<223>  Description of Artificial Sequence: CURT1A forward primer

<400>  22
ggatggtctt gagaac                                                       16


<210>  23
<211>  20
<212>  DNA
<213>  Artificial Sequence

<220>
<223>  Description of Artificial Sequence: CURT1A reverse primer

<400>  23
gcttcctgcg atcttcttct                                                   20


<210>  24
<211>  20
<212>  DNA
<213>  Artificial Sequence

<220>
<223>  Description of Artificial Sequence: CURT1A forward primer

<400>  24
ctcgaatagt gaagctcctc                                                   20


<210>  25
<211>  20
<212>  DNA
<213>  Artificial Sequence

<220>

<223>  Description of Artificial Sequence: CURT1A reverse primer

<400>  25
cactcactat cagacccaag                                                    20


<210>  26
<211>  21
<212>  DNA
<213>  Artificial Sequence

<220>
<223>  Description of Artificial Sequence: CURT1B forward primer

<400>  26
cggaattcat ggccgctaaa g                                                  21


<210>  27
<211>  20
<212>  DNA
<213>  Artificial Sequence

<220>
<223>  Description of Artificial Sequence: CURT1B reverse primer

<400>  27
cctctagact agctgctccc                                                    20


<210>  28
<211>  20
<212>  DNA
<213>  Artificial Sequence

<220>
<223>  Description of Artificial Sequence: CURT1B forward primer

<400>  28
actccaagat cgtctctacg                                                    20


<210>  29
<211>  21
<212>  DNA
<213>  Artificial Sequence

<220>
<223>  Description of Artificial Sequence: CURT1B reverse primer

<400>  29
catgcatggt tagcttagct t                                                  21


<210>  30
<211>  21
<212>  DNA
<213>  Artificial Sequence

<220>
<223>  Description of Artificial Sequence: CURT1B forward primer

<400>  30

aaagccactg cttactgtcg g                                                21

<210>  31
<211>  21
<212>  DNA
<213>  Artificial Sequence

<220>
<223>  Description of Artificial Sequence: CURT1B reverse primer

<400>  31
gctgctccct aatatgtctt t                                                21

<210>  32
<211>  22
<212>  DNA
<213>  Artificial Sequence

<220>
<223>  Description of Artificial Sequence: CURT1C forward primer

<400>  32
cggaattcat ggctagtgga ga                                               22

<210>  33
<211>  22
<212>  DNA
<213>  Artificial Sequence

<220>
<223>  Description of Artificial Sequence: CURT1C reverse primer

<400>  33
gctctagatc actggccaag ta                                               22

<210>  34
<211>  22
<212>  DNA
<213>  Artificial Sequence

<220>
<223>  Description of Artificial Sequence: CURT1C forward primer

<400>  34
cgccatggct tcaatttctg ca                                               22

<210>  35
<211>  25
<212>  DNA
<213>  Artificial Sequence

<220>
<223>  Description of Artificial Sequence: CURT1C reverse primer

<400>  35
cgccatggcc tggccaagta tatcc                                            25

<210>  36
<211>  21
<212>  DNA
<213>  Artificial Sequence

<220>
<223>  Description of Artificial Sequence: CURT1C forward primer

<400>  36
caactttgcc ttcgccattg t                                                21


<210>  37
<211>  20
<212>  DNA
<213>  Artificial Sequence

<220>
<223>  Description of Artificial Sequence: CURT1C reverse primer

<400>  37
ctggccaagt atatccgcta                                                  20


<210>  38
<211>  21
<212>  DNA
<213>  Artificial Sequence

<220>
<223>  Description of Artificial Sequence: CURT1C forward primer

<400>  38
caactttgcc ttcgccattg t                                                21


<210>  39
<211>  20
<212>  DNA
<213>  Artificial Sequence

<220>
<223>  Description of Artificial Sequence: CURT1C reverse primer

<400>  39
gctgctaggg tttcgagtaa                                                  20


<210>  40
<211>  21
<212>  DNA
<213>  Artificial Sequence

<220>
<223>  Description of Artificial Sequence: CURT1C forward primer

<400>  40
tgggcatcat tgaatctcat c                                                21


<210>  41
<211>  20
<212>  DNA

<213>  Artificial Sequence

<220>
<223>  Description of Artificial Sequence: CURT1C reverse primer

<400>  41
ctggccaagt atatccgcta                                                    20


<210>  42
<211>  24
<212>  DNA
<213>  Artificial Sequence

<220>
<223>  Description of Artificial Sequence: CURT1D forward primer

<400>  42
cgcccgggat ggagctctgc acca                                               24


<210>  43
<211>  23
<212>  DNA
<213>  Artificial Sequence

<220>
<223>  Description of Artificial Sequence: CURT1D reverse primer

<400>  43
gcagatctct cactatcaga ccc                                                23


<210>  44
<211>  23
<212>  DNA
<213>  Artificial Sequence

<220>
<223>  Description of Artificial Sequence: CURT1D forward primer

<400>  44
cggaattcat gtgtagtaga aag                                                23


<210>  45
<211>  22
<212>  DNA
<213>  Artificial Sequence

<220>
<223>  Description of Artificial Sequence: CURT1D reverse primer

<400>  45
gctctagatc actcactatc ag                                                 22


<210>  46
<211>  20
<212>  DNA
<213>  Artificial Sequence

<220>

<223>  Description of Artificial Sequence: CURT1D forward primer

<400>  46
cgccatggag ctctgcacca                                                    20


<210>  47
<211>  24
<212>  DNA
<213>  Artificial Sequence

<220>
<223>  Description of Artificial Sequence: CURT1D reverse primer

<400>  47
cgccatggcc tcactatcag accc                                               24


<210>  48
<211>  20
<212>  DNA
<213>  Artificial Sequence

<220>
<223>  Description of Artificial Sequence: CURT1D forward primer

<400>  48
tgcaccaggt ctaccacaat                                                    20


<210>  49
<211>  21
<212>  DNA
<213>  Artificial Sequence

<220>
<223>  Description of Artificial Sequence: CURT1D reverse primer

<400>  49
ttgagtttcc tcatcttcag c                                                  21


<210>  50
<211>  20
<212>  DNA
<213>  Artificial Sequence

<220>
<223>  Description of Artificial Sequence: CURT1D forward primer

<400>  50
tgcaccaggt ctaccacaat                                                    20


<210>  51
<211>  20
<212>  DNA
<213>  Artificial Sequence

<220>
<223>  Description of Artificial Sequence: CURT1D reverse primer

<400>  51

gcaacaacta ctccatcacg                                                    20

<210>  52
<211>  20
<212>  DNA
<213>  Artificial Sequence

<220>
<223>  Description of Artificial Sequence: CURT1D forward primer

<400>  52
ctcgaatagt gaagctcctc                                                    20

<210>  53
<211>  20
<212>  DNA
<213>  Artificial Sequence

<220>
<223>  Description of Artificial Sequence: CURT1D reverse primer

<400>  53
cactcactat cagacccaag                                                    20

<210>  54
<211>  23
<212>  DNA
<213>  Artificial Sequence

<220>
<223>  Description of Artificial Sequence: PetC forward primer

<400>  54
gcgaattcgc gtcgagtatt cca                                                23

<210>  55
<211>  47
<212>  DNA
<213>  Artificial Sequence

<220>
<223>  Description of Artificial Sequence: PetC reverse primer

<400>  55
gcgaattcgc gtcgagtatt ccagctctag attaagacca ccatgga                      47

<210>  56
<211>  23
<212>  DNA
<213>  Artificial Sequence

<220>
<223>  Description of Artificial Sequence: PsaD1 forward primer

<400>  56
gcgaattcat ggccgagaaa aca                                                23

<210>  57
<211>  26
<212>  DNA
<213>  Artificial Sequence

<220>
<223>  Description of Artificial Sequence: PsaD1 reverse primer

<400>  57
gctctagatc acaaatcata actttg                                          26


<210>  58
<211>  21
<212>  DNA
<213>  Artificial Sequence

<220>
<223>  Description of Artificial Sequence: ACTIN1 forward primer

<400>  58
tgcgacaatg gaactggaat g                                               21


<210>  59
<211>  20
<212>  DNA
<213>  Artificial Sequence

<220>
<223>  Description of Artificial Sequence: ACTIN1 reverse primer

<400>  59
agcatgtgga agtgcatacc                                                 20


<210>  60
<211>  34
<212>  DNA
<213>  Artificial Sequence

<220>
<223>  Description of Artificial Sequence: PG UR forward primer

<400>  60
tttggtctct aggttgctca gcagtgacct attc                                 34


<210>  61
<211>  33
<212>  DNA
<213>  Artificial Sequence

<220>
<223>  Description of Artificial Sequence: PG UR reverse primer

<400>  61
tttggtctct tggggccact gttattttga ttg                                  33


<210>  62
<211>  33
<212>  DNA

<213>  Artificial Sequence

<220>
<223>  Description of Artificial Sequence: psbA2P forward primer

<400>  62
tttggtctct cccatggaaa aaacgacaat tac                                    33


<210>  63
<211>  35
<212>  DNA
<213>  Artificial Sequence

<220>
<223>  Description of Artificial Sequence: COE_P reverse primer

<400>  63
tttggtctct ttggttataa ttccttatgt atttg                                  35


<210>  64
<211>  37
<212>  DNA
<213>  Artificial Sequence

<220>
<223>  Description of Artificial Sequence: CURT1A forward primer

<400>  64
tttggtctct ccaaatggct tcttcagaag agacctc                                37


<210>  65
<211>  36
<212>  DNA
<213>  Artificial Sequence

<220>
<223>  Description of Artificial Sequence: CURT1A2 reverse primer

<400>  65
tttggtctct aacgctattc gcttcctgcg atcttc                                 36


<210>  66
<211>  36
<212>  DNA
<213>  Artificial Sequence

<220>
<223>  Description of Artificial Sequence: selection cassette forward
      primer

<400>  66
tttggtctca cgttggaatt cgattgatcc gtcgac                                 36


<210>  67
<211>  34
<212>  DNA
<213>  Artificial Sequence

<220>
<223>  Description of Artificial Sequence: selection cassette reverse
      primer

<400>  67
tttggtctcc catactttag gcccgtagtc tgca                                34


<210>  68
<211>  35
<212>  DNA
<213>  Artificial Sequence

<220>
<223>  Description of Artificial Sequence: PG DR2 forward primer

<400>  68
tttggtctct tatgcaattt cgtttgcgaa tttac                               35


<210>  69
<211>  32
<212>  DNA
<213>  Artificial Sequence

<220>
<223>  Description of Artificial Sequence: PG DR2 reverse primer

<400>  69
tttggtctct aagcataaaa taccttccca tc                                  32


<210>  70
<211>  36
<212>  DNA
<213>  Artificial Sequence

<220>
<223>  Description of Artificial Sequence: CURT UR forward primer

<400>  70
tttggtctct aggtttttgg acctatctgg gtgaag                               36


<210>  71
<211>  32
<212>  DNA
<213>  Artificial Sequence

<220>
<223>  Description of Artificial Sequence: CURT UR reverse primer

<400>  71
tttggtctct aacgagcttc ccatattggg gc                                  32


<210>  72
<211>  36
<212>  DNA
<213>  Artificial Sequence

<220>
<223>  Description of Artificial Sequence: selection cassette forward

primer

<400>  72
tttggtctca cgttggaatt cgattgatcc gtcgac                                36


<210>  73
<211>  34
<212>  DNA
<213>  Artificial Sequence

<220>
<223>  Description of Artificial Sequence: selection cassette reverse
       primer

<400>  73
tttggtctcc catactttag gcccgtagtc tgca                                  34


<210>  74
<211>  37
<212>  DNA
<213>  Artificial Sequence

<220>
<223>  Description of Artificial Sequence: COE_P forward primer

<400>  74
tttggtctct tatgcccatg gaaaaaacga caattac                               37


<210>  75
<211>  35
<212>  DNA
<213>  Artificial Sequence

<220>
<223>  Description of Artificial Sequence: COE_P reverse primer

<400>  75
tttggtctct ttggttataa ttccttatgt atttg                                 35


<210>  76
<211>  37
<212>  DNA
<213>  Artificial Sequence

<220>
<223>  Description of Artificial Sequence: CURT1A forward primer

<400>  76
tttggtctct ccaaatggct tcttcagaag agacctc                               37


<210>  77
<211>  36
<212>  DNA
<213>  Artificial Sequence

<220>
<223>  Description of Artificial Sequence: CURT1A reverse primer

<400>  77
tttggtctct gacactattc gcttcctgcg atcttc                                36

<210>  78
<211>  29
<212>  DNA
<213>  Artificial Sequence

<220>
<223>  Description of Artificial Sequence: CURT DR forward primer

<400>  78
tttggtctca tgtctccaga ccgccccag                                        29

<210>  79
<211>  33
<212>  DNA
<213>  Artificial Sequence

<220>
<223>  Description of Artificial Sequence: CURT DR reverse primer

<400>  79
tttggtctct aagccaaatg ccattcctgg gcg                                   33

## Description

The present invention relates to a method of enhancing photosynthesis of a photosynthetic eukaryote by transforming a polynucleotide encoding a CURT1 polypeptide into the photosynthetic eukaryote or enhancing the expression of an endogenous polynucleotide encoding a CURT1 polypeptide in the photosynthetic eukaryote. The present invention also relates to an organism or a part thereof having enhanced photosynthesis.

Photosynthesis is a process used by plants and other organisms to convert the light energy captured from the sun into chemical energy that can be used to fuel the organism's activities. Photosynthesis occurs in plants, algae, and many species of bacteria. In plants, algae, and cyanobacteria, photosynthesis uses carbon dioxide and water, releasing oxygen as a waste product. Although photosynthesis can happen in different ways in different species, some features are always the same. For example, the process always begins when energy from light is absorbed by proteins called photosynthetic reaction centers that contain chlorophylls. In plants, these proteins are held inside organelles called chloroplasts, while in bacteria they are embedded in the plasma membrane. Some of the light energy gathered by chlorophylls is stored in the form of adenosine triphosphate (ATP). The rest of the energy is used to remove electrons from a substance such as water. These electrons are then used in the reactions that turn carbon dioxide into organic compounds. In plants, algae and cyanobacteria, this is done by a sequence of reactions called the Calvin cycle.

Photosynthetic organisms are photoautotrophs, which means that they are able to synthesize glucose directly from carbon dioxide and water using energy from light. The overall reaction of photosynthesis is the light-driven conversion of carbon dioxide and water to glucose and oxygen:

6 CO₂+6 H₂O→C₆H₁₂O₆+6O₂

In plants, algae and cyanobacteria, photosynthesis releases oxygen. This is called *oxygenic photosynthesis.* Although there are some differences between oxygenic photosynthesis in plants, algae, and cyanobacteria, the overall process is quite similar in these organisms.

In plants and algae, photosynthesis takes place in organelles called chloroplasts. A typical plant cell contains about 10 to 100 chloroplasts. The chloroplast is enclosed by a membrane system, the outer and inner chloroplast envelope. Within the membrane is an aqueous fluid called the stroma. Thylakoids, the internal membranes of chloroplasts and cyanobacteria, accommodate the light reactions of photosynthesis. Chloroplasts of land plants contain grana, which are characteristic cylindrical stacks with a typical diameter of 300-600 nm comprising ∼5-20 layers of thylakoid membrane. A single granum consists of a central core of appressed membranes, two stroma-exposed membranes at the top and bottom of the cylindrical structure, and the highly curved margins that merge two grana membranes at their periphery. Grana stacks are interconnected by stroma-exposed membrane pairs of up to few µm in length, the "stroma lamellae". All thylakoid membranes within one chloroplast form a continuous network that encloses a single lumenal space. The topography of this network, as well as the precise three-dimensional structure of grana themselves, remains a much debated issue. Although grana are ubiquitous in land plants, the fraction of thylakoid membrane found in stroma lamellae appears to be remarkably constant among species.

Grana and stroma thylakoids differ strikingly in their protein composition (lateral heterogeneity). Photosystem II (PSII) and its light-harvesting complex (LHCII) performing the light reaction of photosynthesis are concentrated in grana, whereas photosystem I (PSI) and the chloroplast ATP synthase, which protrude extensively from the membrane into the stroma, are excluded from the grana core and reside in the stroma-facing regions. The primary purpose of grana is debated and suggested functions include prevention of spillover of excitation energy through physical separation of photosystems, fine-tuning of photosynthesis, facilitation of state transitions and switching between linear and cyclic electron flow and, in particular, enhancing light harvesting under low-light conditions through the formation of large arrays of PSII-LHCII supercomplexes. However, grana formation also imposes constraints on photosynthesis, such as the requirement for long-range diffusion of electron carriers between PSII and PSI and the relocation of PSII between appressed and non-appressed regions during its repair.

The formation of the intricate network of thylakoid membranes involves forces that mediate the stacking of membranes, as well as mechanisms that promote the curvature of the membranes at the sites of cylindrical grana stacks. Whereas the latter mechanisms are as yet unknown, it is generally accepted that stacking results from the interplay of physicochemical forces of attraction and repulsion between adjacent membranes. Moreover, lipid composition and lipid-protein interactions are also thought to play a role. Thus, plants adapted to shade and low-light conditions have many more layers of thylakoid membranes per granum than those that prefer bright sunlight. Furthermore, because the diameter of grana increases when thylakoid proteins are less phosphorylated, light-induced PSII phosphorylation has been proposed to increase repulsion between adjacent thylakoid membrane layers.

In photosynthetic bacteria, the proteins that gather light for photosynthesis are embedded within cell membranes. These membranes may be tightly folded into cylindrical sheets called thylakoids or bunched up into round vesicles called intracytoplasmic membranes. These structures can fill most of the interior of a cell, giving the membrane a very large surface area and therefore increasing the amount of light that the bacteria can absorb.

To meet the increasing world food demands as well as for economic reasons, enhancing the rate of plant growth and hence of biomass production is becoming more and more important. To achieve this goal, it is of central importance to generate plant varieties with improved growth rate under suboptimal conditions, such as the conditions of low light found in some greenhouses.

Enhancing photosynthetic activity by increasing stacking of thylakoid membranes would be one approach to adapt plants to specific conditions of light. However, so far there has been little success to increase the efficiency of photosynthesis by targeted gene technological approaches which influence the stacking of thylakoid membranes. In particular, there has been no success in identifying the molecular structures which are involved in thylakoid stacking and membrane curvature.

Therefore, there is a need in the art for a method allowing the enhancement of photosynthesis in particular in plants under cultivation growing in the shade or under low-light conditions. This problem has been solved by the present invention by the provision of the claims. In a first aspect, the present invention provides a method of enhancing photosynthesis in a cell of a photosynthetic eukaryote or in a photosynthetic eukaryote comprising the step of transforming the cell of the photosynthetic eukaryote or the photosynthetic eukaryote with a polynucleotide encoding a CURT1 polypeptide, whereby the cell of the photosynthetic eukaryote or the photosynthetic eukaryote expresses the polynucleotide.

Chloroplasts of land plants characteristically contain grana, cylindrical stacks of thylakoid membranes. Multiple forces contribute to grana stacking, but it is not known how the extreme curvature at margins is generated and maintained. The present inventors succeeded in assigning a function in grana stacking to the CURVATURE THYLAKOID1 (CURT1) protein family. The CURT1 protein family of *Arabidopsis* comprises four CURT1 polypeptides, CURT1A, B, C and D. Polypeptides of the CURT1 protein family are conserved in plants, algae and cyanobacteria. Polypeptides of this family have been found to be highly enriched at grana margins. Grana architecture is correlated with CURT1 polypeptide level.

The CURT1 polypeptides are involved in membrane curvature of thylakoid membranes and thus in grana stacking. In vitro, CURT1 polypeptides oligomerise and induce tubulation of liposomes, implying that CURT1 polypeptides suffice to induce membrane curvature by an evolutionarily conserved mechanism. Heterologous expression of *Arabidopsis* CURT1A modifies the shape of cyanobacterial thylakoids. Thus, CURT1 polypeptides contribute to thylakoid architecture by inducing membrane curvature at grana margins.

*Arabidopsis* CURT1 polypeptides are characterised by a chloroplast transit sequence and four α-helix regions, whereby the N-terminal α-helix region is tentatively amphiphatic. The four CURT1 polypeptides show a high homology, especially in the two C-terminal α-helix regions. The polynucleotide and amino acid sequences of CURT1A, B, C and D of *Arabidopsis thaliana* have been published under the following GenBank accession numbers as available from the NCBI (National Centre for Biotechnology Information; National Library of Medicine 38A, Bethesda, MD20894; USA; www.ncbi.nih.gov). The sequences are indicated in the following.

The polynucleotide sequence of CURT1A is available under the GenBank Accession No. NM_116345.3:

ATGGCGATAT CAGTAGCAGC TTCGTCCTCT ATGGCCGTGA TGGTTCCACG
TGTTCCCGCC GTATCCACCC GTTGCTCCGC CGTCCCTTAT CTTCCTCCTC
GCTCCTTTGG CCGATCCTCT TTCACCGTTC CGTTGAAGCT TGTTTCAGGG
AATGGATTGC AAAAAGTTGA ATTGTTGAAG ACAAGAGCTT CTTCAGAAGA
GACCTCGTCC ATTGATACCA ACGAACTCAT CACAGATTTG AAGGAAAAGT
GGGATGGTCT TGAGAACAAA TCTACTGTAC TTATATATGG AGGAGGAGCC
ATTGTTGCTG TTTGGTTATC TTCCATTGTT GTTGGTGCCA TCAACTCTGT
TCCTCTGCTT CCGAAAGTTA TGGAACTTGT CGGTCTCGGG TACACTGGAT
GGTTTGTCTA CAGATACCTT CTCTTCAAGT CAAGCAGAAA GGAATTGGCT
GAGGATATTG AATCCTTGAA GAAGAAGATC GCAGGAAGCG AATAG
(SEQ ID NO. 1)

The polynucleotide sequence of CURT1B is available under the GenBank Accession No. NM_130248.4:

ATGGCTTCTC TTTCAGTCTC TTCCTCTTCA ACCATCATCG ATTCAAGAGC

TCCTCCTTCT CGACTAGCCT CCGCCTCCGC CTCTTCTCCG TCGTGCATTT

CACTTCCCAC ACTTCCGATT CAGTCTCATA CCCGTGCCGC TAAAGCCACT

GCTTACTGTC GGAAGATTGT GAGGAACGTT GTGACGAGAG CTACTACTGA

AGTTGGTGAA GCTCCTGCCA CTACTACCGA AGCTGAGACT ACTGAGTTAC

CTGAAATCGT CAAGACTGCT CAAGAAGCTT GGGAGAAAGT GGATGACAAG
TACGCTATTG GTTCTCTTGC CTTTGCTGGT GTAGTGGCTC TTTGGGGTTC
TGCTGGAATG ATTTCGGCAA TCGATAGGCT TCCATTGGTT CCTGGTGTTC
TTGAACTTGT AGGCATCGGT TACACAGGAT GGTTCACTTA CAAGAACCTG
GTCTTCAAAC CAGACAGGGA GGCTTTGTTT GAGAAGGTCA AGAGCACATA
CAAAGACATA TTAGGGAGCA GCTGA
(SEQ ID NO. 2)

The polynucleotide sequence of CURT1C is available under the GenBank Accession No. NM_104101.1:

ATGGCTTCAA TTTCTGCAAC TTTGCCTTCG CCATTGTTAC TCACACAGAG
AAAATCCAAT CTCACATCGA TTCAAAAACT CCCATTTTCT CTAACTCGAG
GTACGAATGA TCTTTCTCCA TTATCTCTTA CTCGAAACCC TAGCAGCATC
AGTCTGATGG TGAAAGCTAG TGGAGAAAGC TCAGATTCAT CGACTGATCT
CGACGTTGTT AGTACGATTC AGAATGTTTG GGATAAGTCT GAAGATAGGT
TAGGTCTTAT TGGTTTGGGT TTTGCTGGTA TTGTAGCTCT TTGGGCATCA
TTGAATCTCA TCACGGCAAT CGACAAATTG CCCGTTATCT CGAGCGGATT
CGAACTAGTT GGTATCTTGT TCTCCACGTG GTTCACATAT CGATATCTCT
TGTTCAAACC GGACAGACAG GAGCTTTCGA AAATTGTCAA GAAATCAGTA
GCGGATATAC TTGGCCAGTG A
(SEQ ID NO. 3)

The polynucleotide sequence of CURT1D is available under the GenBank Accession No. NM_119971.3:

ATGGAGCTCT GCACCAGGTC TACCACAATC ATCACTCATC TCCCTGCGAG
CTTCAACGGC CATGGATATC TAGCGGGAAA ATCTGTTGAT AGAATCTCAC
TTCCTCTCCA GCGAAACGTT GCGTCTCTCG TTCTTCAATC CCGCACGTTG
CGTTGTAGTA GAAAGTTTCC AGGAGAAACA GTTACTGAAG AAACATCAAC
AGGAGTCAAT GAGTTTGGTG TGGAGGATCG TGATGGAGTA GTTGTTGCAG
CCGAAGAGAA AAACTCGAAT AGTGAAGCTC CTCAAGCTGA AGATGAGGAA
ACTCAAGCCC TAGAGTTTTT GAACGATATT AAGCTGGACT CAGACAAGAC
ATATTCAATC CTGCTCTATG GGAGTGGTGC GATAGTTGCT CTCTACTTAA
CGTCTGCAAT CGTCAGTTCA CTCGAAGCAA TCCCTCTGTT TCCTAAGCTA
ATGGAAGTAG TTGGTCTCGG ATACACACTC TGGTTCACTA CCCGTTATCT
GCTATTTAAG AGGAACAGAG AAGAACTCAA GACAAAAGTT AGTGAAATCA
AAAAGCAGGT TCTTGGGTCT GATAGTGAGT GA

(SEQ ID NO. 4)

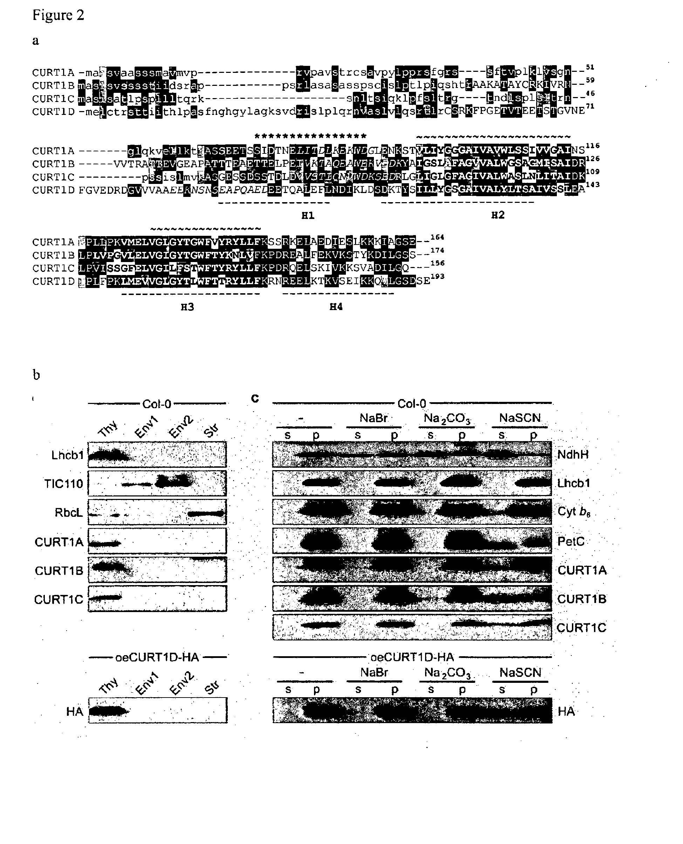

The corresponding amino acid sequences of CURT1A, B, C and D are indicated in Fig. 2 and have been assigned the numbers SEQ ID NO: 5 for CURT1A, SEQ ID NO: 6 for CURT1B, SEQ ID NO: 7 for CURT1C and SEQ ID NO: 8 for CURT1D in the sequence listing.

The polynucleotide sequence of slr0483, a *Synechocystis* CURT1 homolog, is available under the GenBank Accession No. BA000022.2 from nucleotides 2617440-2617889 of the Synechocystis sp. PCC 6803 complete genome.

GTGGGCCGTA AACATTCAAT CCGAATAATA AAGGACTGGT TGAAGATGGA
AGAACAAAAG ACAGCAACTG CCGGGATTAA AACCGATGTA GGACCAATCA
CTACTCCCAA TCCCCAAAAG TCCCCAATCA CCGACCAGGC TTGGCAGGAA
TGGCTACAAC CGGTGTGGGA AGTGCTGGGT AAAATTCCTC AAATGACCGG
GGAGTTTTTC GAGGACAACA AGCAACCCCT CATTTCCCTG GGCATAATCC
TGTTGGGCAT TATCAGCGTC AAAATCTTGA TTGCTGTGCT CGATGCCATT
AATGATATTC CTTTATTGGC ACCAACTTTA CAGTTGATCG GCATGGGCTA
CACCGCTTGG TTTATCTGGC GTTACCTTTG GAAAGCTGAA AAGCGCCAAG
AGTTGGCTTC CGAATTTGGT GCACTAAAGG AGCAAATTTT TGGCGGTTAG
(SEQ ID No. 9)

The amino acid sequence of slr0483 is available under the GenBank Accession No. BAA10316.1

MGRKHSIRII KDWLKMEEQK TATAGIKTDV GPITTPNPQK SPITDQAWQE
WLQPVWEVLG KIPQMTGEFF EDNKQPLISL GIILLGIISV KILIAVLDAI
NDIPLLAPTL QLIGMGYTAW FIWRYLWKAE KRQELASEFG ALKEQIFGG
(SEQ ID No. 10)

The polynucleotide encoding a CURT1 polypeptide, also designated CURT1 polynucleotide, may be any polynucleotide encoding a CURT1 polypeptide. There are four CURT1 polynucleotides in the plant of the genus *Arabidopsis* encoding CURT1A, B, C and D polypeptides. Homologous genes of *Arabidopsis* CURT1 genes exist in the nuclear genomes of other plants, algae, and, more distantly related, also in cyanobacteria. In the context of the present invention, a polynucleotide encoding a CURT1 polypeptide as comprised by the plant genus *Arabidopsis* is a polynucleotide comprising or consisting of SEQ ID NO: 1 coding for CURT1A, SEQ ID NO: 2 coding for CURT1B, SEQ ID NO: 3 coding for CURT1C and/or SEQ ID NO: 4 coding for CURT1D. SEQ ID NO: 1, SEQ ID NO: 2, SEQ ID NO: 3 and SEQ ID NO: 4 encode CURT1A, B, C and D polypeptides having the amino acid sequences of SEQ ID NO: 5, SEQ ID NO: 6, SEQ ID NO: 7 or SEQ ID NO: 8, respectively. The term "polynucleotide encoding a CURT1 polypeptide" or "CURS1 polynucleotide" or "polynucleotide" as used herein encompasses polynucleotides which are defined by SEQ ID NOs: 1 to 4 as well as naturally occurring or non-naturally occurring variants or fragments thereof (as defined herein). If no specific CURT1 polynucleotide is referred to, the above terms are directed to the CURT1A, B, C and/or D polynucleotide(s) either alone or combined in any possible combination such as CURT1A and B, CURT1A and C, CURT1A and D, CURT1B and C, CURT1B and D, CURT1C and D, CURT1A, B and C, CURT1A, B and D, CURT1A, C and D, CURT1B, C and D or CURT1A, B, C and D.

The most common (and therefore preferred) polynucleotides are deoxyribonucleic acid (DNA) and ribonucleic acid (RNA). The polynucleotide can be a DNA molecule such as a genomic DNA molecule or a cDNA molecule which can be single- or double-stranded as well as a synthetic DNA such as a synthesized single-stranded polynucleotide. The polynucleotide of the present invention can also be an RNA molecule. Preferably, the term also relates to non-coding regions of a gene, wherein these sections are of a relevant size in order to be specific for that gene. Examples of those regions are regulatory elements such as a promoter. Most preferably, the term "polynucleotide" relates to gene, open reading frame (ORF), promoter, DNA, cDNA or mRNA. The polynucleotide encoding the desired genetic information, preferably DNA, may comprise the gene of interest, a promoter region, a start codon and a stop codon and possibly further regions which may be used for regulation of expression of the gene.

The polynucleotide, as referred to above, may encode functionally active variants of the polypeptide of SEQ ID NOs: 5 to 8. Such functionally active variants have a sequence identity with SEQ ID NOs: 5 to 8 of more than 50%, of more than 60%, preferably more than 70%, more preferably of more than 80%, still more preferably more than 85%, even more preferably more than 90%, even more preferably more than 95%, most preferably more than 97% and/or have an activity of more than 50%, more than 60%, more than 70%, more than 80%, more than 90%, more than 95%, more than 97% or more than 100 % , e.g. more than 150 %, 200 %, 300 % 400 % or 500 % of the activity of the polypeptide of SEQ ID NOs: 5 to 8. Consequently, the polynucleotides encoding such variants may contain deletions, insertions, substitutions and/or additions within and/or at the 5' and/or 3' termini of SEQ ID NOs: 1 to 4 and show an identity to the polynucleotides of SEQ ID NOs: 1 to 4 of more than 50%, more than 60%, more than 70%, preferably more than 80%, more preferably more than 85%, even more preferably more than 90%, even more preferably more than 95%, most preferably more than 97%. In the context of the present invention, a variant polynucleotide encoding a functionally active variant of the polypeptide of SEQ ID NOs: 5 to 8 means a sequence encoding a variant polypeptide which has the activity of inducing membrane curvature resulting in enhanced photosynthesis.

The polynucleotide fragments, as referred to above, may encode functionally active fragments of the polypeptides of SEQ ID NOs: 5 to 8, respectively. This may include fragmental polypeptides with short internal and/or C- and/or N-terminal deletions whereby the activity of the resulting polypeptides as identified herein is maintained to an extent of more than 50 %, more than 60 %, more than 70 %, more than 80 %, more than 90 %, more than 95 %, more than 97 % or more than 100 % , e.g. more than 150 %, 200 %, 300 % 400 % or 500 %, of the activity of the wild-type proteins as encoded by SEQ ID NOs: 1 to 4. Consequently, the respective polynucleotide encoding such fragments contains deletions within and/or at the 5' and/or 3' termini resulting in the polypeptides of SEQ ID NOs: 5 to 8 with, e. g., deletions of at the most 50%, 45%, 40%, 35%, 30%, 25%, 20%, 15%, 10 %, 5%, or less. In the context of the present invention, a fragmental polynucleotide encoding a functionally active fragment of the polypeptide of SEQ ID NOs: 5 to 8 means a sequence encoding a fragment which has the activity of inducing membrane curvature resulting in enhanced photosynthesis.

It is noted that the above mentioned modifications may be combined. For example, a polynucleotide as comprised by the present invention may be a fragment comprising one or more variations. It should also be noted that fragments or variants include fragments or variants, as defined herein, of promoter or regulatory sequences with which the polynucleotide of SEQ ID NOs: 1 to 4 or fragments or variants thereof are associated in nature. These variants are functionally active in that they regulate the transcription or translation of the genes associated therewith.

In a further embodiment, the CURT1 polynucleotide encodes a CURT1 polypeptide of SEQ ID NO: 5, 6, 7 or 8. Due to the degeneracy of the genetic code, the polynucleotide sequences of SEQ ID NO: 1, 2, 3 or 4 may be modified by replacing nucleotides in such a way that the same amino acid is encoded.

In a further embodiment, the CURT1 polynucleotide hybridizes under stringent conditions to the homologous polynucleotide of SEQ ID NOs: 1 to 4 or a functionally active variant or fragment thereof. In the present invention, the term "hybridizes under stringent conditions" refers to the formation of a hybrid between two polynucleotide molecules under conditions which allow the formation of a so-called specific hybrid, while a non-specific hybrid is substantially not formed. An example of such conditions includes conditions under which a complementary strand of a highly identical nucleic acid, namely a DNA composed of a nucleotide sequence having 70 % or more, preferably 80% or more, more preferably 85% or more, still more preferably 90% or more and even more preferably 95% or more identity with the polynucleotide of SEQ ID NOs: 1 to 4, hybridizes, while a less complementary strand of a nucleic acid less identical than the above does not hybridize. More specifically, such conditions refer to conditions in which the sodium salt concentration is 15 to 750 mM, preferably 50 to 750 mM, and more preferably 300 to 750 mM, the temperature is 25 to 70°C, preferably 50 to 70°C, and more preferably 55 to 65°C, and the formamide concentration is 0 to 50%, preferably 20 to 50%, and more preferably 35 to 45%. Furthermore, under stringent conditions, conditions for washing a filter after hybridization normally comprise the following: the sodium salt concentration is 15 to 600 mM, preferably 50 to 600 mM, and more preferably 300 to 600 mM, and the temperature is 50 to 70°C, preferably 55 to 70°C, and more preferably 60°C. Stringency, and thus specificity, can e.g. be increased by increasing the reaction temperature and/or lowering the ion strength of the reaction buffer. For example, low stringent conditions comprise hybridization in 3 x SSC at room temperature to 65°C and highly stringent conditions comprise hybridization in 0.1 x SSC at 68°C. Exemplary moderately stringent conditions (nucleic acids hybridize under moderately stringent conditions, if they are maximally degenerate with respect to their codon composition) comprise 50% formamide, 5 x SSC and 1% SDS at 42°C and washing in 1 x SSC at 45°C. Highly stringent conditions comprise incubation at 42°C, 50% formamide, 5 x SSC and 1% SDS (e.g. 50% formamide, 5 x SSC and 1% SDS, 50 mM sodium phosphate, 5 x Denhardt's solution, 10 x dextran sulphate, 20 mg/ml sheared salmon sperm DNA) or 5 x SSC and 1% SDS at 65°C and washing in 0.2 x SSC and 0.1% SDS at about 65°C (1 x SSC stands for 0.15 M sodium chloride and 0.015 M trisodium citrate buffer). Preferred in the present invention are moderately or highly stringent conditions, most preferred are highly stringent conditions. In the context of the present invention, a "hybridizing" sequence means a sequence which encodes a polypeptide which has the activity of inducing membrane curvature resulting in enhanced photosynthesis. It should be noted that the hybridizing sequence is the complementary non-coding strand of a putative hybridizing CURT1 polynucleotide which hybridizes to the coding strand of SEQ ID NOs: 1, 2, 3 or 4.

In a further embodiment, the polynucleotide is complementary to a polynucleotide of SEQ ID NOs: 1 to 4 or a polynucleotide encoding a polypeptide of SEQ ID NOs: 5 to 8 or functionally active fragments or variants thereof.

In one embodiment, the polynucleotide comprises one, two, three or all four polynucleotides of SEQ ID NOs: 1 to 4 in any combinations, as referred to above.

The polynucleotide of the present invention may be provided by any methods known in the art. Using the sequence information provided herein, primers suitable for amplification/isolation of the polynucleotide of SEQ ID NOs: 1 to 4 can be determined according to standard methods well known to those of skill in the art. Primers suitable for amplification/isolation of the polynucleotide of SEQ ID NOs: 1 to 4 are designed according to the nucleotide sequence information provided in the sequence listing. The procedure is as follows: a primer is selected which may consist of 10 to 40, preferably 15 to 25 nucleotides. It is advantageous to select primers containing C and G nucleotides in a proportion sufficient to ensure efficient hybridization; i.e., an amount of C and G nucleotides of at least 40%, preferably 50% of the total nucleotide content. A standard PCR reaction will be performed which typically contains 0.5 to 5 Units of Taq DNA polymerase per 100 µl, 20 to 200 µM deoxynucleotide each, preferably at equivalent concentrations, 0.5 to 2.5 mM magnesium over the total deoxynucleotide concentration, 105 to 106 target molecules, and about 20 pmol of each primer. About 25 to 50 PCR cycles are performed. A more stringent annealing temperature improves discrimination against incorrectly annealed primers and reduces incorporation of incorrect nucleotides at the 3' end of primers. A denaturation temperature of 95°C to 97°C is typical, although higher temperatures may be appropriate for denaturation of G+C-rich targets. The number of cycles performed depends on the starting concentration of target molecules, though typically more than 40 cycles are not recommended as non-specific background products tend to accumulate. An alternative method for retrieving polynucleotides encoding variant polypeptides as defined herein is by hybridization screening of a DNA or RNA library using the primers and probes as defined herein. Hybridization procedures are well-known and are described in the art and herein.

Alternatively or additionally to the above, the polynucleotide may be provided by cloning and thereby introducing it into and amplifying it in a cell. The procedure of introducing a gene into a recipient cell is called transformation. The genes can be introduced into the cells by a variety of means known in the art and adapted to each cell type. The term "cell" refers to the cell in which the gene is expressed irrespective of whether it is a prokaryotic cell or a eukaryotic cell and of whether the cell naturally expresses the respective genes or not. Recombinant DNA cloning techniques well known in the art for introducing and expressing a nucleic acid molecule can be used to introduce and express the gene which is either endogenous if the cell harbors the respective gene or is heterologous if the gene is not endogenous to the cell. Cells can be transformed using any appropriate means, including viral or bacteriophage based vectors, chemical agents, electroporation, calcium phosphate co-precipitation or direct diffusion of DNA.

The regulatory regions for regulating expression of the CURT1 polynucleotide in a vector are promoters and possibly enhancers or other regulatory sequences. They may be heterologous to the respective gene or may be associated therewith in nature. The genetic information may be expressed permanently or under the control of a repressor and/or a promoter region in a cell into which the nucleic acid of the present invention is introduced. The obtained cells may be either used directly or used for tissue cultures or the cells may be harvested and samples comprising the respective polypeptide are obtained by disrupting the cells. Alternatively, DNA or RNA may be used either in cells or in cell-free expression systems such as, e.g., microarray systems in which the DNA or RNA is immobilized and is translated and/or transcribed by the addition of functional cell lysate, comprising the factors required for transcription and/or translation (enzymes, ribosomes, tRNA, amino acids, nucleotides, ATP etc.). Also included are artificial nucleic acids which include peptide nucleic acid (PNA), morpholino and locked nucleic acid (LNA), as well as glycol nucleic acid (GNA) and threose nucleic acid (TNA). Each of these is distinguished from naturally-occurring DNA or RNA by changes to the backbone of the molecule.

The term "comprising" as used herein is meant to "include or encompass" the desired feature and further features which must not be specifically mentioned. The term "comprising" is also meant to "consist of" the desired feature and not to include further features except the desired feature.

The term "heterologous", as it relates to polynucleotide sequences or polypeptides denotes sequences that are normally not associated with a region of a recombinant construct and/or a particular cell. A "heterologous" region is an identifiable segment of a polynucleotide within or attached to another polynucleotide that is not found in association with the other molecule in nature. For example, a heterologous region of a construct could be a regulatory region not found to be associated with a gene as identified herein in nature. Similarly, a heterologous sequence could be a coding sequence which is itself not found in nature as it contains e.g. synthetic sequences with codons different from the native gene. Moreover, a cell transformed with a construct which is not normally present in the cell would be considered heterologous for the purposes of the present invention.

The term exogenous, as used herein, refers to polynucleotides or polypeptides that are not normally present in a cell. Transforming a cell with an exogenous polynucleotide may also mean that the polynucleotide is introduced into a cell, regardless of whether the same polynucleotide is already present in the cell.

A homologous polynucleotide sequence is a variant sequence as defined herein. The term "homologous" may be used interchangeably with variant. The term "homologous" may also refer to a polynucleotide or polypeptide that is from another species and that has the same physiological function in said other species. The term "homologous" may also refer to an identical sequence.

Vectors are agents that transport an endogenous or heterologous gene into the cell. Vectors typically consist of a number of genetic components, including but not limited to regulatory elements such as promoters, leaders, introns, and terminator sequences. Transcription of DNA into mRNA is regulated by a region of DNA usually referred to as the "promoter". Vectors can be a plasmid, a cosmid, a virus (e. g. bacteriophage) a phagemide, an artificial chromosome or a retro-viral vector or others as known in the art. Vectors are able to autonomously replicate in a host cell or can be incorporated into chromosomal DNA. The term "vectors" includes those that function primarily for insertion of a polynucleotide into a cell, those that function primarily for replication of a polynucleotide (replication vector) in a cell of a photosynthetic eukaryote or those that function primarily for transcription and/or translation of DNA or RNA in a cell.

The promoter region contains a sequence of bases that signals RNA polymerase to associate with the DNA and to initiate the transcription into mRNA using one of the DNA strands as a template to make a corresponding complementary strand of RNA.

As generally used herein, transformation of a cell or tissue with a polynucleotide is the introduction of a polynucleotide into the cell or tissue by any means.

The CURT1 polypeptides as comprised by the present invention encompass the polypeptides of SEQ ID NOs: 5 to 8 as well as functionally active variants or fragments thereof, as defined above, which may be natural or non-natural. Preferably, the variants or fragments differ from the sequences of SEQ ID NOs: 5 to 8 e.g. by addition, deletion, substitution and/or insertion of amino acids and have a sequence identity with SEQ ID NOs: 5 to 8 of more than 50%, of more than 60%, more than 70%, preferably of more than 80%, more preferably more than 85%, even more preferably more than 90%, even more preferably more than 95%, most preferably more than 97% and/or have an activity of more than 50%, more than 60%, more than 70%, more than 80%, more than 90%, more than 95%, more than 97% or more than 100 % , e.g. more than 150 %, 200 %, 300 % 400 % or 500 % of the activity of the polypeptides of SEQ ID NOs: 5 to 8.

In the context of the present invention, the variant of the proteins of SEQ ID NOs: 5 to 8 as comprised by the present invention is a functionally active protein in that it maintains the biological function of the reference protein of SEQ ID NOs: 5 to 8, i.e. it has the activity of inducing membrane curvature resulting in enhanced photosynthesis.

Non-naturally occurring variants or naturally occurring variants of the polypeptides of SEQ ID NOs: 5 to 8 may comprise a limited number of amino acid deletions, insertions and/or substitutions, particularly deletions, insertions and/or substitutions of, e.g., at most 100, 50, 30, 20, 10, 9, 8, 7, 6, 5, 4, 3, 2 or 1 amino acid(s) thereby obtaining a sequence identity or activity of the respective wild-type proteins, e.g. with respect to SEQ ID NOs: 5 to 8, as mentioned above.

The variant may be a modified protein or a modified protein variant which comprises a further component. In one embodiment, the variant may be a fusion protein comprising (i) a polypeptide of SEQ ID NOs: 5 to 8 or functionally active variant and (ii) a further protein or peptide component. For example, the polypeptide may be coupled to a marker, such as a tag used for purification purposes (e.g. 6 His (or HexaHis) tag, Strep tag, HA tag, c-myc tag or glutathione S-transferase (GST) tag). If e.g. a highly purified polypeptide of SEQ ID NOs: 5 to 8 or variant should be required, double or multiple markers (e.g. combinations of the above markers or tags) may be used. In this case the proteins are purified in two or more separation chromatography steps, in each case utilizing the affinity of a first and then of a second tag. Examples of such double or tandem tags are the GST-His-tag (glutathione-S-transferase fused to a polyhistidine-tag), the 6xHis-Strep-tag (6 histidine residues fused to a Strep-tag), the 6xHis-tag100-tag (6 histidine residues fused to a 12-amino-acid protein of mammalian MAP-kinase 2), 8xHis-HA-tag (8 histidine residues fused to a hemagglutinin-epitope-tag), His-MBP (His-tag fused to a maltose-binding protein, FLAG-HA-tag (FLAG-tag fused to a hemagglutinin-epitope-tag), and the FLAG-Strep-tag. The marker could be used in order to detect the tagged protein, wherein specific antibodies could be used. Suitable antibodies include anti-HA (such as 12CA5 or 3F10), anti-6 His, anti-c-myc and anti-GST. Furthermore, the protein could be linked to a marker of a different category, such as a fluorescence marker such as green fluorescent protein, to a binding protein such as steptavidin, one or more small molecular dyes such as Cy dye, or a radioactive marker, which allows for the detection of a protein as comprised by the present invention. In a further embodiment, the proteins of SEQ ID NOs: 5 to 8 could be part of a fusion protein, wherein the second part could be used for detection, such as a protein component having enzymatic activity.

Alternatively or additionally, the polypeptides of SEQ ID NOs: 5 to 8 or variants or fragments thereof as described above may comprise one or more amino acid substitution(s). However, semi-conservative and especially conservative amino acid substitutions, wherein an amino acid is substituted with a chemically related amino acid are preferred. Typical substitutions are among the aliphatic amino acids, among the amino acids having aliphatic hydroxyl side chain, among the amino acids having acidic residues, among the amide derivatives, among the amino acids with basic residues, or the amino acids having aromatic residues.

Several technologies for transforming a cell of a photosynthetic eukaryote or a plant with a polynucleotide are well known to those of skill in the art. For transformation of a cell of a photosynthetic eukaryote in accordance with the present invention, suitable genetic components to be inserted into the cells have to be selected.

In a preferred embodiment, the genetic components are incorporated into a vector comprising at least the following types of genetic components:
(a) a promoter that functions in the cells of the photosynthetic eukaryote to cause the production of an RNA sequence,
(b) a CURT1 polynucleotide sequence that causes the production of an RNA sequence, and
(c) possibly a 3' non-translated DNA sequence that functions in the cells of the photosynthetic eukaryote to cause the addition of polyadenylated nucleotides to the 3' end of the RNA sequence.

The CURT1 polynucleotide may heterologous to the cell or may be already present in the cell.

The vector may contain a number of genetic components to facilitate transformation of the cell or tissue of the photosynthetic eukaryote and regulate expression of the CURT1 polynucleotide. Means for preparing vectors containing the desired genetic components are well known in the art. For example, vectors useful to transform plants and methods of making those vectors are described in U.S. Patent Nos. 4,971908, 4,940,835, 4,769,061 and 4,757,011. The vectors may be plasmids, cosmids, viral vectors, phagemides, artificial chromosomes or retro-viral vectors.

The promoter for transformation of a cell or tissue of a photosynthetic eukaryote may be constitutive, inducible, developmental stage-preferred, cell type-preferred, tissue-preferred, organ-preferred such as nuclear or cytoplasmic specific, temporally regulated, spatially regulated, spatio-temporally regulated, tissue-enhanced or tissue-specific. The promoter may also be a viral promoter. Constitutive promoters are active under most conditions. Examples of constitutive promoters active in plant cells include caulimovirus promoters such as the cauliflower mosaic virus (CaMV) 19S and 35S promoters, the sX CaMV 35S promoter, the Sep1 promoter, the rice actin promoter, the *Arabidopsis* actin promoter, the ubiquitin promoter, pEmu, the figfigwort mosaic virus 35S promoter, the Smas promoter, the super promoter (U.S. Patent No. 5, 955,646 ), the GRP1-8 promoter, the cinnamyl alcohol dehydrogenase promoter (U.S. Patent No. 5,683,439 ), promoters from the T-DNA of Agrobacterium, such as mannopine synthase, nopaline synthase, octopine synthase or the small subunit of ribulose biphosphate carboxylase (ssu-RUBISCO) promoter, the CaMV-35Somega promoter, *Arabidopsis* ubiquitin *UBQ1* promoter or barley leaf thionin *BTH6* promoter (Holtorf, 1995).

Promoter hybrids can also be constructed to enhance transcriptional activity (U. S. Patent No. 5,106,739) or to combine desired transcriptional activity, inducibility, and tissue or developmental specificity. The particular promoter selected should be capable of causing sufficient expression to result in the production of an effective amount of the CURT1 protein. The promoters used for expression of the CURT1 polynucleotide may be modified, if desired, to affect their control characteristics. Promoters can be derived by means of ligation with operator regions, random or controlled mutagenesis, etc. Furthermore, the promoters may be altered to contain multiple "enhancer sequences" to assist in elevating gene expression. Examples of such enhancer sequences have been reported by Kay et al. (1987). Enhancer sequences may be used to increase or alter the transcriptional or translational efficiency of the gene. The enhancer sequence may be derived from the sequence that accompanies the selected promoter sequence or from unrelated promoters or genes. Other genetic components that serve to enhance expression or affect transcription or translational of a gene are also envisioned as genetic components. This sequence can be derived from the promoter selected to express the gene and can be specifically modified so as to increase translation of the mRNA. The regulatory sequences can also be obtained from viral RNAs, from suitable eukaryotic genes, or from a synthetic gene sequence.

Inducible promoters are induced by the presence or absence of biotic or abiotic factors. Inducible promoters are a very powerful tool in genetic engineering because the expression of genes operably linked to them can be turned on or off as desired, for example at certain stages of development of an organism or in a particular tissue. Inducible promoters are preferentially active under certain environmental conditions such as the presence or absence of a nutrient or metabolite, heat or cold, light, pathogen attack, anaerobic conditions, and the like. Examples of inducible promoters are cis-Jasmone-, tetracycline-, dexamethasone-, copper-, salicyclic acid-, heavy-metal-, benzene sulfonamide-, abscisic acid-, ethanol-, cyclohexanone-inducible promoters or soybean heat-shock promoters. The Gmshp17.3 promoter is induced by heat shock (Holtorf, 1995); the hsp80 promoter from Brassica is induced by heat shock; the PPDK promoter is induced by light; the PR-1 promoters from tobacco, *Arabidopsis* and maize are inducible by infection with a pathogen and the Adh1 promoter is induced by hypoxia and cold stress. Chemically inducible promoters are especially suitable if gene expression is wanted to occur in a time specific manner. Examples of such promoters are a salicylic acid inducible promoter (PCT Application No. WO 95/19443), a tetracycline inducible promoter (Gatz, (1992) or an ethanol inducible promoter (PCT Application No. WO 93/21334). The skilled person will be aware of many other promoters which are inducible and can be used in the present invention.

Tissue-specific promoters control gene expression in a tissue-dependent manner and according to the developmental stage of the plant. Tissue-specific promoters include generally those that are specifically expressed in certain tissues such as leaves, roots, seeds, or xylem. The transgenes driven by these type of promoters will only be expressed in tissues where the transgene product is desired, leaving the rest of the tissues in the plant unmodified by transgene expression. Tissue-specific promoters may be induced by endogenous or exogenous factors, so they can be classified as inducible promoters as well. For example, tissue-specific promoters may be root-specific promoters, seed-specific promoters or ovule-specific promoters (Nain, 2008) or the Gh-sp-promoter derived from a seed protein gene, or Gh-rbcS-promoter obtained from a nuclear encoded chloroplast gene (Song, 2000). Tissue-specific promoters as used in the present invention are typically specific for leaves of plants as photosynthesis occurs mainly in leaves. Examples of leaf-specific promoters are the light-harvesting chlorophyll a/b binding protein (*CAB*) of photosystem-II (*CAB2*) promoter (*CAB2*-p) (Song, 2007) or the *Gh-rbcS* promoter (Song, 2000). The skilled person will be aware of many other promoters which are leaf specific and can be used in the present invention.

Any type of promoter can be used, as long as it functions in the cells. Selection of promoters, vectors and other elements is a matter of routine design. Many such elements are described in literature and are available through commercial suppliers.

The regulatory regions may be heterologous to the respective gene or may be associated therewith in nature. The genetic information may be expressed permanently or under the control of a repressor and/or a promoter region in a cell into which the nucleic acid of the present invention is introduced.

The 3' non-translated region of the constructs comprising a CURT1 gene and a regulatory sequence may or should contain a transcriptional terminator, or an element having equivalent function, and a polyadenylation signal, which functions in plants to cause the addition of polyadenylated nucleotides to the 3' end of the RNA. Examples of suitable 3' regions are the 3' transcribed, non-translated regions containing the polyadenylation signal of Agrobacterium tumor-inducing (Ti) plasmid genes, such as the nopaline synthase (NOS) gene, plant genes such as the soybean storage protein genes and the small subunit of the ribulose-1,5-bisphosphate carboxylase (ssRUBISCO) gene or the 3' region from the ssRUBISCO E9 gene from pea (EP 385,962).

In another embodiment, the vector contains a selectable, screenable, or scoreable marker gene. The polynucleotide that serves as a selection device functions in a regenerable plant cell or tissue to produce a compound that would confer upon the plant tissue resistance to an otherwise toxic compound. Examples include beta-glucuronidase (GUS), green fluorescent protein (GFP), luciferase (LUX), antibiotic or herbicide tolerance genes such as the glyphosate gene. Examples of transposons and associated antibiotic resistance genes include the transposons Tns (bla), Tn5 (nptII), Tn7 (dhfr), penicillin, kanamycin, neomycin, G418, bleomycin, methotrexate, trimethoprim, chloramphenicol or tetracycline.

After the construction of the plant transformation vector, said nucleic acid molecule can be transformed into the cell of a photosynthetic eukaryote by any method known in the art. While it is preferred that the CURT1 polynucleotide is comprised within a vector which is transformed into a cell, it may also be transformed into a cell in plain form without being comprised by a vector or any other polynucleotide construct.

Suitable transformation methods include chemical methods, physical methods such as microinjection, electroporation or the gene gun method or receptor-mediated mechanisms. For some monocot species, direct DNA transfer into isolated protoplasts and microprojectile-mediated DNA delivery has been developed. Another method is the protoplast method, where DNA is delivered to the protoplasts through liposomes, PEG and electroporation. The protoplast methods require the establishment of long-term embryogenic suspension cultures. Also usable are microprojectile bombardment method for the transformation and regeneration of monocots and dicots. Another often used method is the Agrobacterium-mediated transformation or agroinfiltration wherein plants are infected by Agrobacterium or virus-based vectors and recombinant plasmids are transiently produced in the plant. Preferred strains include Agrobacterium tumefaciens strain C58, a nopaline strain that is used to mediate the transfer of DNA into a plant cell; octopine strains, such as LBA4404; or agropine strains, e. g., EHA101, EHA105, or EHA109. A further transformation method is transformation of protoplasts using polyethylene glycol.

Transformation of cells can be stable, when the DNA is stably integrated into the genome, thereby creating a transgenic photosynthetic eukaryote, or transient, where the DNA is not stably integrated into the genome. In a preferred embodiment, the cells are stably transformed with the exogenous polynucleotide.

Transformation of algae, especially diatoms and green algae, is also known in the art. In principle, the above transformation methods may be used, as far as they are applicable to algal cells. The transformation may be by a conventional method. For example, an endogenous promoter is separated, a gene is conjugated with a promoter, and the promoter and the gene are introduced into algae. Alternatively, for transformation of algae, a promoter derived from a virus rather than an endogenous promoter may be used. For example, algae may be transformed using a viral promoter such as a CaMV35S promoter derived from a cauliflower mosaic virus. Another method for transformation of algae is by the use of a promoter located upstream from a gene considered as encoding a replication-associated protein of a Chaetoceros debilis DNA virus (CdebDNAV) as disclosed in US 2011/0300633. This promoter is able to transform several algal species. Moreover, transformation of algae may be performed by the other methods as mentioned above such as chemical, physical or receptor-mediated transformation.

The term "eukaryote" refers to an organism whose cells contain complex structures enclosed within membranes. The defining membrane-bound structure that sets eukaryotic cells apart from prokaryotic cells is the nucleus, or nuclear envelope, within which the genetic material is carried. Most eukaryotic cells also contain other membrane-bound organelles such as mitochondria, chloroplasts and the Golgi apparatus. All large complex organisms, but also many unicellular organisms are eukaryotes, including animals, plants, algae and fungi.

"Photosynthetic" organisms are organisms which are able to perform photosynthesis and to synthesize glucose directly from carbon dioxide and water using energy from light.

A "photosynthetic eukaryote" as used in the present invention is a eukaryotic organism which is able to perform photosynthesis. Comprised by the term photosynthetic eukaryote are plants and the most algae.

In one embodiment, the plant is selected from monocotyledons, dicotyledons or eudicotyledons. In another embodiment, the plant is useful for nutrition, in particular human nutrition or animal nutrition. In another embodiment, said plant is from a species selected from flowering plants. In a further embodiment, said plant is from a species selected from greenhouse plants. In a still further embodiment, said plant is useful for the production of biomass. Algae are a very large and diverse group of simple, typically autotrophic organisms, ranging from unicellular to multicellular forms. Most algae are photosynthetic like plants, however are "simple" because they lack the many distinct cell and organ types found in land plants. Comprised by the present invention are any algae which are photosynthetic. Algae conduct photosynthesis within membrane-bound organelles also called chloroplasts. Chloroplasts contain circular DNA and are similar in structure to cyanobacteria. The exact nature of the chloroplasts is different among separate lineages of algae, reflecting different endosymbiotic events. In one embodiment, the alga is useful for nutrition, in particular human nutrition or animal nutrition. In a further embodiment, said alga is useful for the production of biomass. As used herein, a cell of a photosynthetic eukaryote may refer to an isolated cell which is free from surrounding cells, however, may also refer to a cell within a tissue (which is a group of one or several types of differentiated cells functioning together such as meristematic tissue, protective tissue which covers the surface of organs such as e. g. leaves or living cells of e. g. roots and stems, parenchyma tissue, sclerenchyma tissue, collenchyma tissue, xylem tissue etc.) or an organ (which is a collection of tissues representing a functional and structural unit such as vegetative organs such as root, stem or leaf or reproductive organs such as flower, seed, fruit or cone), a part (which is any part of a photosynthetic eukaryote) or an explant (tissue obtained from the plant for culturing) of a photosynthetic eukaryote. The term "cell" may also refer to a protoplast which is a cell without a cell wall. Thus, if a cell is transformed with a polynucleotide or if a plant is regenerated from a cell, this includes transformation or regeneration of an isolated cell as well as of a cell within a tissue, organ, part or explant of the photosynthetic eukaryote, so that usually not only a cell is transformed or regenerated, but also the surrounding cells and usually the whole tissue, organ, part or explant of the photosynthetic eukaryote or the photosynthetic eukaryote itself. The term "enhancing photosynthesis", as used herein, means that the rate of photosynthesis is increased in a cell into which a CURT1 polypeptide has been introduced or in which the expression of the CURT1 polynucleotide is increased versus the same cell into which the CURT1 polypeptide has not been introduced or in which the expression of the CURT1 polynucleotide is not increased (control cell). For example, a control cell may be a wildtype cell. An enhancement of photosynthesis results in the increase of growth and/or yield of the photosynthetic eukaryote. Consequently, the rate of photosynthesis can be measured by determining the growth and/or yield of the photosynthetic eukaryote, as expressed e.g. by an increased production of biomass, by an increased biovolume or an increased cell density in solution in case of unicellular organisms, e.g. unicellular algae. Measurement can be done by harvesting the photosynthetic eukaryote which has been transformed with a CURT1 polynucleotide or in which the expression of endogenous CURT1 polynucleotide is increased after a specific time of culturing such as hours, days or weeks, drying the mass to constant weight and determining the weight of the dry mass. Alternatively, the wet mass may be determined. Alternatively, the volume may be determined. A comparison with the control will indicate whether photosynthesis is enhanced. Analogously, a control in the context of "increasing the expression of an endogenous CURT1 gene" refers to a cell in which the expression of the particular endogenous CURT1 gene is not increased by the particular methods applied for increasing expression. Measurement of "increase of expression" may be performed by any method known in the art, for example by hybridization of probes to the expressed mRNA and quantification of the mRNA, by quantification of the expressed polypeptide etc.

Measurements of photosynthesis rate also include the determination of the fraction of QA (primary electron acceptor quinone molecule) present in the reduced state and measurements of the maximum quantum yield (FV/FM) and of the effective quantum yield ([Phi]II) of photosystem II. Such measurements are known to a person skilled in the art and can be performed without difficulties. Other methods for determining the rate of photosynthesis including a) measuring the uptake of CO₂; b) measuring the production of O₂; c) measuring the production of carbohydrates; or d) measuring the rate of decolorisation of DCPIP in the Hill Reaction may be used. These or further methods are known to the skilled person, such as a method using IRGA (Infra-Red Gas analyser) which can compare the CO₂ concentration in gas passing into a chamber surrounding the photosynthetic eukaryote and the CO₂ leaving the chamber or by measuring uptake of CO₂ labeled with ¹⁴C. Alternatively, the photosynthetic eukaryote can be given into a plastic bag and the CO₂ concentration can be monitored in the bag using a CO₂ monitor to measure the CO₂ concentration produced by the photosynthetic eukaryote; or by using a bicarbonate indicator solution in the bag. A well-known method for measuring the uptake of CO₂ in algae is the use of immobilized algae known as the 'algal balls' technique. Thereby, the algae are immobilized in a hydrogen carbonate indicator solution. Alternatively, a disc cut out from one side of a leaf can be dried and weighed and compared to a disk cut out from the same leaf some time later. This may be compared to a control leaf.

Enhancement of the photosynthesis rate may occur under light exposure of the photosynthetic eukaryote or a cell or tissue thereof to any photosynthetic active radiation (PAR), preferably in the range of 50 to 2000 µmol m⁻²·s⁻¹, more preferably 50 to 1000 µmol m⁻²·s⁻¹, still more preferably 50 to 500 µmol m⁻²·s⁻¹, still more preferably 50-200 µmol m⁻²·s⁻¹ and most preferably 80-100 µmol m⁻²·s⁻¹. PAR is meant to refer to the spectral range that photosynthetic organisms are able to use in the process of photosynthesis. Preferably such spectral range is in the range from 400 nm to 700 nm. This is measured with a quantum sensor in units of micromol per metre squared per second (µmol m⁻²·s⁻¹), which is usually called Photosynthetic Photon Flux Density (PPFD), relating to light in the 400 to 700 nanometer wavelength. For example, at midday in mid summer the sun can reach a PAR of around 2000 µmol m⁻²·s⁻1.

The increase of the photosynthesis rate is at least 5 %, preferably at least 10 %, more preferably at least 15 %, still more preferably at least 30 %, still more preferably at least 50 % or still more preferably at least 100 %.

In a second aspect, the present invention is directed to a method as referred to above, wherein the cell of a photosynthetic eukaryote is a regenerable cell and wherein the photosynthetic eukaryote is regenerated from the transformed regenerable cell of the photosynthetic eukaryote.

The method of the present invention can be used for enhancing photosynthesis of a regenerable plant cell. A regenerable plant cell refers to a cell that can form into a differentiated plant. Source examples of such a plant cell include callus tissue, somatic embryo, microspore, inflorescence, hypocotyl tissue, cotyledon tissue, root tissue or leave tissue.

In a first step, a regenerable cell or a regenerable tissue, organ, part or explant is generated which is transformed with a CURT1 polynucleotide. The skilled person knows how to isolate a cell, tissue, organ, part or explant from a plant and how to cultivate the cell, tissue, organ, part or explant in order to receive a regenerable cell, tissue, organ, part or explant. A cell, tissue, organ, part or explant isolated from a plant may be cultivated in a tissue culture system in order to grow and to result in the formation of a callus which is a friable lump of cells on a solid surface or to grow as individual or small clusters of cells in a suspension culture. A cell, tissue, organ, part or explant isolated from a plant may also be cultivated in a tissue culture system to result in the formation of a multicellular organism such as a whole plant. In principle, a regenerable cell, tissue, organ, part or explant for a plant tissue culture can be initiated from almost any part of a plant and maintained in a plant tissue culture system. Mostly, the meristematic ends of plants like stem tip, auxiliary bud tip or root tip are used. Plant tissue culture relies on the fact that many plant cells have the ability to regenerate into a whole plant. For example, a regenerable cell, tissue, organ, part or explant, as used within the present invention, may be embryogenic callus tissue. Embryogenic calli are produced from immature embryos. These calli can be produced by isolating and culturing immature embryos on a nutrient media containing carbohydrates and plant growth regulators. Embryogenic callus or other target tissue for transformation may be isolated by a number of methods known to those of skill in the art. For example, WO 97/48814 describes the isolation of wheat immature embryos and embryogenic callus. Similarly, immature embryos of maize may be precultured on a suitable culture medium. Alternatively, somatic embryos may be formed from plant cells or explants that are not normally involved in the development of embryos, i.e. ordinary plant tissue. Cells derived from competent source tissue are cultured to form an undifferentiated mass of cells called a callus. Plant growth regulators in the tissue culture medium can be manipulated to induce callus formation and subsequently changed to induce embryos to form from the callus. The ratio of different plant growth regulators required to induce callus or embryo formation varies with the type of plant. Alternatively, a regenerable cell, tissue, organ, part or explant can be developed from leaf tissue or hypocotyl sections as e.g. in soybean, wherein suspension cell cultures can be developed from leaf tissue and maintained for an extended period. Hypocotyl sections can be prepared from germinated seedlings by methods known to those of skill in the art. Explants for regenerating photosynthetic eukaryotes may be taken from seedlings. Alternatively, precultured cells or tissues may be used as starting material. Precultured means culturing the cells or tissues in an appropriate medium to support plant growth. The preculture of the regenerable cell, tissue, organ, part or explant can occur for an extended period of time, for example at least seven days or for a shorter period of time such as six days or less. Examples of suitable media for preculture would include, but are not limited to, MS-based media or N6-based media supplemented with additional nutrients and/or plant growth regulators. A variety of tissue culture media that, when supplemented appropriately, support plant tissue growth and development is known to those skilled in the art. Examples of such media include Gamborg's media, McCown's Woody plant media, Nitsch and Nitsch media or Schenk and Hildebrandt media which may supplemented accordingly. Such media and media supplements such as nutrients and growth regulators for use in transformation and regeneration are usually optimized for the particular target crop of interest.

Once the regenerable cell, tissue, organ, part or explant is developed and produced, the next step of the method is transforming the CURT1 polypeptide into the cell, tissue, organ, part or explant. A number of methods have been reported and can be used to transform, i.e. insert, genetic components into a regenerable plant cell, tissue, organ, part or explant, as referred to above.

Generation of a photosynthetic eukaryote or of a whole cell organism from a cell, tissue, organ, part or explant is performed in plant tissue culture in which plant cells, tissues, organs, parts or explants (tissue obtained from the plant for culturing) are maintained or grown under sterile conditions on a nutrient culture medium of known composition. The exact conditions required to regenerate intact plants from cultured cells, tissues, organs or explants may be different for plant species. Each species or variety of a species may have a particular set of cultural requirements. Such conditions are either state of the art or have to be identified through experimentation. Due to the knowledge about plant tissue culture, the skilled person will be able to identify by experimentation suitable conditions for culturing cells, tissues, organs, parts or explants and regenerate whole plants therefrom.

In a third aspect, the present invention refers to a method of enhancing photosynthesis in a cell of a photosynthetic eukaryote or in a photosynthetic eukaryote comprising the step of increasing the expression of a CURT1 polynucleotide endogenous to the cell of the photosynthetic eukaryote or to the photosynthetic eukaryote in said cell or photosynthetic eukaryote.

Methods for increasing the expression of an endogenous gene are known to those skilled in the art. Exposing plant cells to mutagenic substances, such as ethyl methane sulfonate (EMS), or exposure to mutagenic radiation may lead to point mutations in the CURT1 gene sequence(s) and may result in an enhanced expression of the CURT1 polypeptides. Such point mutations can be identified using, as an example, the TILLING-method ("targeting induced local lesions in genomes"), which is based on the recognition of mismatch hybridization via HPLC. Gene expression may also be increased by the administration or modulation, i.e. activation or inhibition, of factors which directly or indirectly influence the expression of endogenous CURT1 genes such as transcription or translation factors such as regulatory proteins. Such factors may be screened for in screening assays using for example chemical libraries. Other factors such as heat or cold, light, certain environmental conditions, anaerobic conditions or the presence or absence of chemical molecules such as nutrients may prove useful in enhancing expression of (an) endogenous CURT1 gene(s). Other methods may involve the modulation of regulatory polynucleotide sequences which direct transcription or translation of CURT1 polynucleotides by replacing the regulatory sequences by more powerful sequences or by introducing mutations into the regulatory sequences for enhancing activity thereof. Enhancing the expression of endogenous (and exogenous) genes in plants may be achieved by supplying a virally encoded booster sequence comprising the 5' proximal region of the potyvirus genome to the plant (WO 1998/044097). Expression of an endogenous CURT1 polypeptide may be increased by at least 5 %, preferably at least 10 %, more preferably at least 15 %, still more preferably at least 30 %, still more preferably at least 50 % or still more preferably at least 100 %.

In a forth aspect, the present invention refers to the method above, wherein the enhancement of photosynthesis results in an increase of growth and/or yield of the photosynthetic eukaryote. The disclosures with respect to enhancing photosynthesis etc. apply.

In a fifth aspect, the present invention refers to an organism having an enhanced CURT1 expression as compared to a control organism. The disclosures with respect to enhancing expression and control organism etc., as referred to above, apply.

In a sixth aspect, the present invention refers to said organism, wherein the expression of an endogenous CURT1 polynucleotide in a cell is enhanced or wherein a cell is transformed with a polynucleotide encoding a CURT1 polypeptide. Enhanced CURT1 expression can be achieved by introducing a CURT1 polynucleotide into a cell such that the polynucleotide is expressed into a CURT1 polypeptide. The CURT1 polynucleotide may be heterologous to the organism or may be identical to an endogenous CURT1 polynucleotide. The presence of an additional CURT1 polynucleotide in the organism in addition to the endogenous CURT1 polynucleotide results in an enhanced expression of the CURT1 polynucleotide and therefore, in an enhanced production of CURT1 polypeptides. Depending on the choice of regulatory sequences, the expression of the exogenous CURT1 polynucleotide may be enhanced over that of the endogenous polynucleotide. Alternatively, enhanced CURT1 expression can be obtained by increasing the expression of an endogenous CURT1 polynucleotide. Transformation or enhancing expression of an endogenous gene is discussed above.

In a seventh aspect, the present invention refers to the above organism, wherein the cell is stably transformed with a CURT1 polynucleotide. Transformation of cells is stable, when the polynucleotide is stably integrated into the genome, thereby creating a transgenic organism. The advantage of stably integrated polynucleotides into the genome is that subsequent generations also stably incorporate the polynucleotide.

In an eighth aspect, the present invention refers to the above organism which is a photosynthetic eukaryote, preferably a plant or an alga, or a cyanobacterium. The definition of a photosynthetic eukaryote is discussed above. Cyanobacteria, also known as blue-green bacteria, blue-green algae or *Cyanophyta,* are a phylum of bacteria that obtain their energy through photosynthesis. Cyanobacteria possess cyanobacterial thylakoid structures, but do not form grana. Cyanobacteria are considered to be progenitors of chloroplasts. It could be shown by the present inventors, that CURT1 polypeptides are affecting thylakoid architecture in cyanobacteria. Cyanobacteria have been used increasingly to study diverse biological processes, including photosynthesis and its regulation; cell differentiation and N₂ fixation; metabolism of nitrogen, carbon, and hydrogen; resistance to environmental stresses; and molecular evolution. Therefore, many vectors and other genetic tools have been developed for unicellular and filamentous strains of cyanobacteria. For example, transformation by use of vectors, electroporation, and conjugation are used for gene transfer. Methods for preparing recombinant microorganisms are described inter alia, in Sambrook, 2001 and Thiel, 1994. For example, suitable vectors for transformation are the shuttle vectors pUF12, PUF311, pFCLV7 or pFC57 (GB2221908) or pAQ-EX1. Moreover, transformation of cyanobacteria may be performed by the other methods as mentioned above such as chemical, physical or receptor-mediated transformation.

In one embodiment, the cyanobacterium is from the group of unicellular cyanobacteria, such as, for example, *Synechocystis* or *Synechococcus.* Preferably, the cyanobacterium is from the genus *Synechocystis,* more preferably *Synechocystis sp. PCC 6803.*

Cyanobacteria, as used in the present invention, serve as a model system for investigating the function of CURT1 polypeptides. It can be taken from the experimental part that *Arabidopsis* CURT1 polypeptides can partially replace *Synechocystis* CURT1 polypeptides. The present inventors could assign a role to the CURT1 polypeptides in *Synechocystis* thylakoid architecture. Thus, *Synechocystis* represents a suitable model system for investigating the function of CURT1 polypeptides.

In a ninth aspect, the present invention refers to a cyanobacterium comprising a polynucleotide encoding a CURT1 polypeptide. The above disclosures with respect to transformation methods also apply to cyanobacteria.

In a tenth aspect, the present invention refers to an organism as discussed above, which is a dicotyledonous, monocotyledonous or eudicotyledonous plant.

In an eleventh aspect, the plant is selected from the group consisting of
(a) Leguminosae, particularly pea, alfalfa, soybean, bean or peanut;
(b) Umbelliferae, particularly carrot or celery;
(c) Solanaceae, particularly tomato, potato, aubergine, tobacco, or pepper;
(d) Cruciferae, particularly the genus Brassica, for example oilseed rape, beet, cabbage, cauliflower, broccoli or thale cress (*Arabidopsis thaliana*);
(e) Compositae, particularly lettuce;
(f) Malvaceae, particularly cotton;
(g) Amaranthaceae, particularly sugar beet or beet root;
(h) Cucurbitaceae, particularly zucchini, squash, melon or cucumber;
(i) wheat, barley, sorghum, millet, rye, triticale, maize, rice, oats and sugarcane;
(ji) apple, pear, quince, plum, cherry, peach, nectarine, apricot, papaya, mango, citrus species, poplar, pine, sequoia, cedar, or oak;
(k) herb species, particularly basil, thyme, sage, chives or parsley; and
(1) berry species, particularly strawberries, Ribes species, Vaccinium species or Rubus species.

In a twelfth aspect, the present invention refers to a method of producing the organism as referred to above, comprising the steps of transforming a cell of the organism or the organism with a polynucleotide encoding a CURT1 polypeptide, wherein the cell or the organism expresses the polynucleotide. The organism and methods of transformation are as mentioned above, including photosynthetic eukaryotes and cyanobacteria..

In a thirteenth aspect, the present invention refers to the method of the twelfth aspect, further comprising generating from the cell a whole organism, whereby the organism is a multicellular organism. Under the second aspect of the invention, reference is made to the generation of a whole organism such as a plant from a cell, tissue, organ, part or explant. This disclosure also applies to the thirteenth aspect of the present invention.

In a fourteenth aspect, the present invention refers to a seed with a stably transformed polynucleotide encoding a CURT1 polypeptide, wherein the seed is produced by the plant as referred to above, wherein a cell of the plant is stably transformed with a polynucleotide encoding a CURT1 polypeptide. Methods for producing seeds from a plant which is transformed with a CURT1 polynucleotide are known in the art. Otherwise, the disclosures with respect to plants, transformation, polynucleotides, polypeptides etc. as referred to above apply.

In a fifteenth aspect, the present invention refers to the use of a polynucleotide encoding a CURT1 polypeptide for enhancing photosynthesis of a cell of a photosynthetic eukaryote or of a photosynthetic eukaryote. The disclosures with respect to enhancing photosynthesis, photosynthetic eukaryote, plants, algae, transformation, polynucleotides, polypeptides etc. as referred to above apply.

In a sixteenth aspect, the CURT1 polypeptide is at least one of CURT1A, CURT1B, CURT1C or CURT1D, preferably wherein the CURT1 polypeptide is CURT1A, CURT1B, CURT1C and CURT1D.

In a seventeenth aspect, the CURT1 polypeptide is from the genus *Arabidopsis,* in particular *Arabidopsis thaliana.*

In an eighteenth aspect, the cell of a photosynthetic eukaryote (including a tissue, organ, part or explant of a photosynthetic eukaryote comprising such cell) or the photosynthetic eukaryote is grown under conditions of shade or low light. As used herein, the term "low-light" conditions refers to conditions with light exposure in the range 10-200 µmol m⁻²·s⁻¹ photosynthetic active radiation (PAR), preferably 10-100 µmol m⁻²·s⁻¹ and more preferably 80-100 µmol m⁻²·s⁻¹ PAR. By a cell of a photosynthetic eukaryote or photosynthetic eukaryote under the eighteenth aspect of the present invention is meant a cell of a photosynthetic eukaryote or a photosynthetic eukaryote which has an enhanced CURT1 expression, for example which is transformed with a CURT1 polynucleotide or which has an enhanced endogenous CURT1 expression.

In a nineteenth aspect, the polynucleotide encoding the CURT1 polypeptide is under the control of an inducible or tissue specific promoter. With respect to inducible and tissue-specific promoters, the above explanations and definitions apply.

### FIGURES

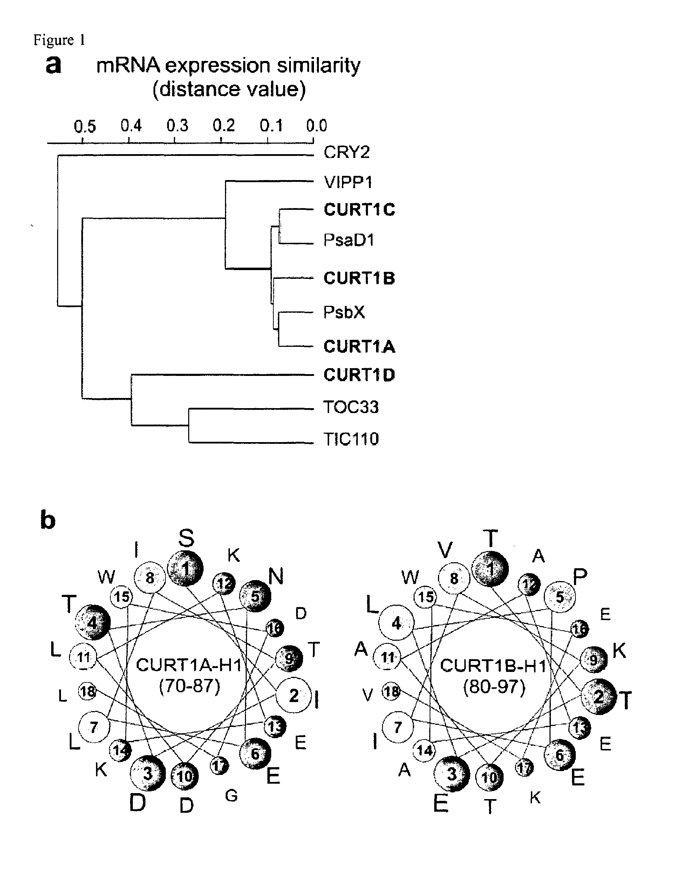

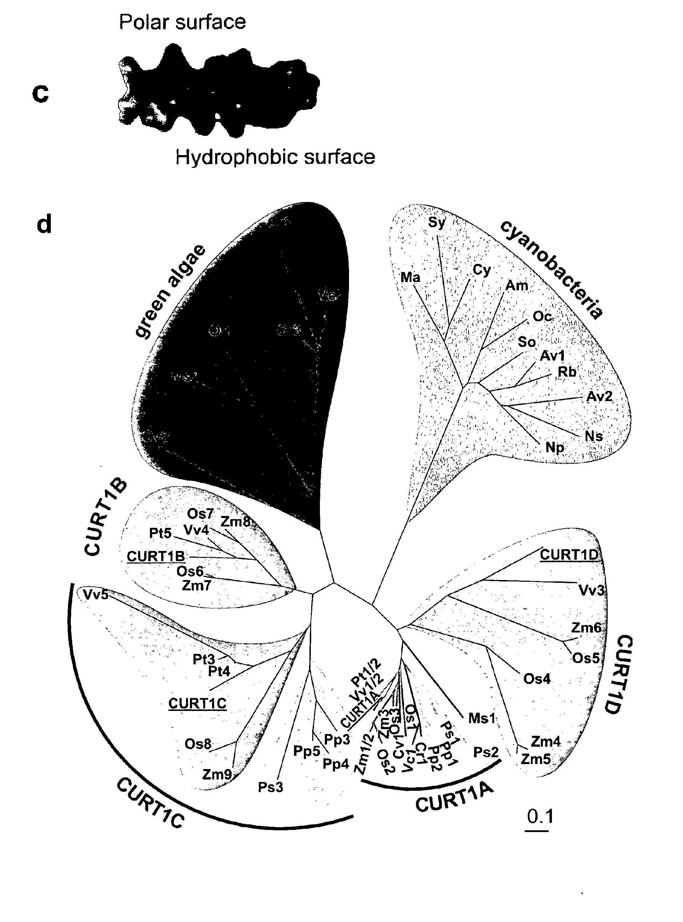

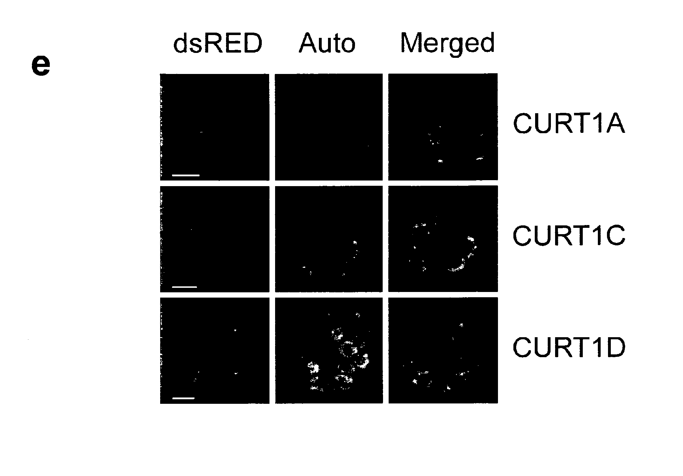

**Figure 1****. Characteristics of CURT1 proteins a,** Expression characteristics of CURT1 protein family genes. *CURT1A, B, C* and *D*, together with genes coding for the chloroplast proteins TIC110 (*At1g06950*), TOC33 (*At1g02280*), PSBX (*At2g06520*), PSAD1 (*At4g02770*) and VIPP1 (*At1g65260*) and the non-chloroplast protein CRY2 (*Atlg04400*) were hierarchically clustered according to expression profile similarity using the gene coexpression database ATTEDII (http://atted.jp/) (Obayashi, 2007).1. Low distance values indicate high expression similarities. **b**, CURT1 proteins contain a conserved N-terminal amphipathic helix. Helical wheel representations of helix H1 of CURT1A and CURT1B with the strongest amphipathic properties tentatively termed CURT1A-H1 and CURT1B-H1 are shown. In the helical wheel representation, the amino acids are coloured according to the physicochemical properties of the side chains (-yellow - hydrophobic; -blue - polar, positively charged; -pink - polar, negatively charged; -green - polar, uncharged). **c**, 3D-model of the putative amphipathic helix CURT1A-H1. The computed surfaces are gradually coloured according to their hydrophobicity from dark red (highly hydrophobic) to deep blue (highly polar). **d**, Phylogenetic analysis of the CURT1 protein family. For the phylogenetic analysis of CURT1 sequences, the C-terminal 99 amino acids of each protein were aligned, and the programs Phylip (v3.67; http://mobyle.pasteur.fr/) and TreeView (v1.6.6; http://taxonomy.zoology.gla.ac.uk/rod/treeview.html) were used for phylogenetic tree construction. The neighbour-joining method was employed for tree building. Standard settings were utilised for all phylogenetic analyses. Branch lengths reflect the estimated number of substitutions per 100 sites. The cyanobacterial and green algal clades are highlighted by blue and dark-green background/branch colour, respectively. Lower and higher plant clades are indicated by light-green and green background/branch colour. Sequence data from this figure can be found in the Arabidopsis Genome Initiative (http://www.arabidopsis.org/), GenBank (http://www.ncbi.nlm.nih.gov) or EMBL (http://www.ebi.ac.uk/embl/) databases under the following accession numbers: *Arabidopsis thaliana:* CURT1A, At4g01150; CURT1B, At2g46820; CURT1C, At1g52220; CURT1D, At4g38100. *Vitis vinifera:* Vv1, 225454426; Vv2, 225462673; Vv3, 225469778; Vv4, 225453634; Vv5, 225442615. *Populus trichocarpa:* Pt1, 224130462; Pt2, 224067952; Pt3, 224070909; Pt4, 224054368; Pt5, 224064380. *Zea mays*: Zm1, 226504766; Zm2, 226500194; Zm3, 226496830; Zm4, 226499766; Zm5, 226529623; Zm6, 226528453; Zm7, 212275151; Zm8, 226509242; Zm9: GRMZM2G302639. *Oryza sativa* subsp. *japonica:* Os1, 115476522; Os2, 115467478; Os3, 115448483; Os4, 115467106; Os5, 115479645; Os6, 115483154; Os7, 115472001; Os8, 297597647. *Picea sitchensis:* Ps1, 116782153; Ps2, 116781331; Ps3, 116783046. *Physcomitrella patens* subsp. *patens:* Pp1, 168018075; Pp2, 168048886; Pp3, 168030972; Pp4, 168019666; Pp5, 168004237. *Chlamydomonas reinhardtii:* Cr1, 159488417; Cr2, 159490118; Cr3, 159466615. *Volvox carteri;* Vc1, 302843342; Vc2, 302846248. *Ostreococcus tauri:* Ot, 308798659. *Micromonas spusilla :* Ms1, 303278702 ; Ms2, 303273852. *Chlorella variabilis :* Cv1, 307104959 ; Cv2, 307106927. *Synechocystis sp.* PCC 6803: Sy, 16331518. *Nostoc punctiforme* PCC 73102 : Np, 186683920. *Cyanothece sp.* CCY0110: Cy , 126659752. *Oscillatoria sp.* PCC 6506 : Oc, 300337383. *Arthrospira maxima* CS-328 : Am, 209493929. *Nodularia spumigena* CCY9414 : Ns, 119510999. *Raphidiopsis brookii* D9: Rb, 281196613. *Microcystis aeruginosa* PCC 7806: Ma, 159030779. *Anabaena variabilis :* Av1, 75907007; Av2, 75906820. *Synechococcus sp.* PCC7002 : So, 170079043. **e**, Full-length CURT1A-, CURT1C- and CURT1D-dsRED fusions were transiently introduced into *Arabidopsis* protoplasts by polyethylene glycol-mediated DNA uptake and analysed using fluorescence microscopy (Auto, chloroplasts revealed by chlorophyll autofluorescence; dsRED, fluorescence of the fusion protein; Merged, overlay of both images). Scale bar = 10 µm.
**Figure 2****. CURT1 proteins are conserved intrinsic thylakoid proteins a,** Sequence alignment *of Arabidopsis* CURT1 proteins. Lowercase letters indicate chloroplast transit peptides, H1-H4 are predicted α-helices (-), of which H1 is tentatively amphipathic (*). Sequences used for antibody generation are depicted in italics. Black/grey shading indicates identical/closely related amino acids conserved in ≥50% of the sequences. TM, transmembrane domain (-). **b,** Suborganellar localisation of CURT1 proteins. Thylakoid (Thy), envelope (Env) and stroma (Str) fractions were subjected to SDS-PAGE and 20 immunoblot analysis. **c,** Extraction of thylakoid-associated proteins with different salt solutions. Supernatants (s) and membrane fractions (p) were analysed as above.

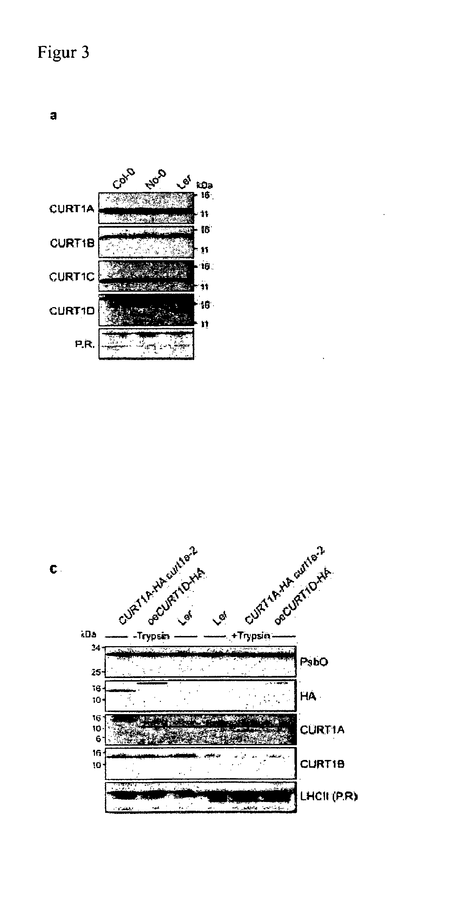

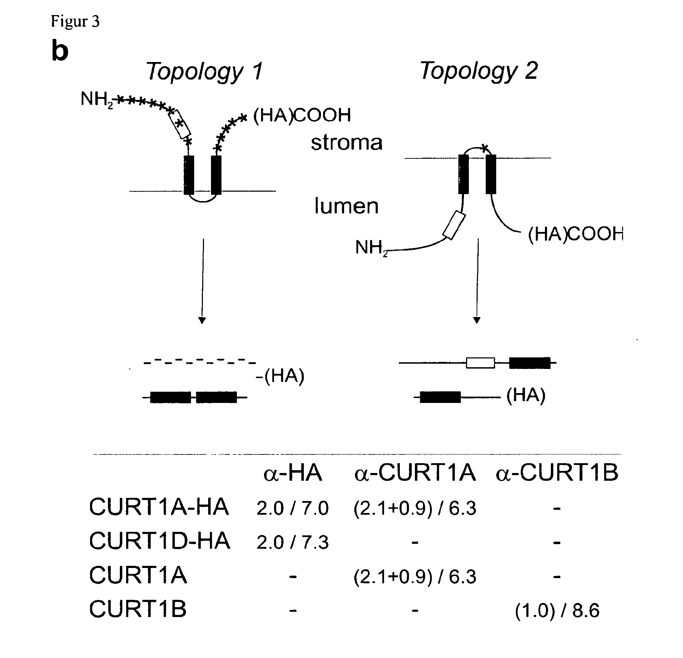

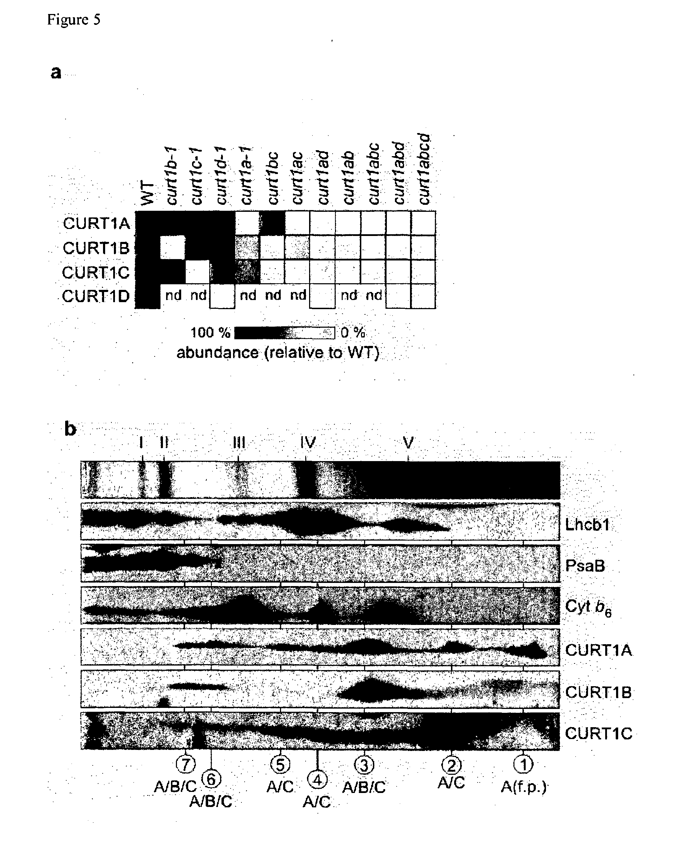

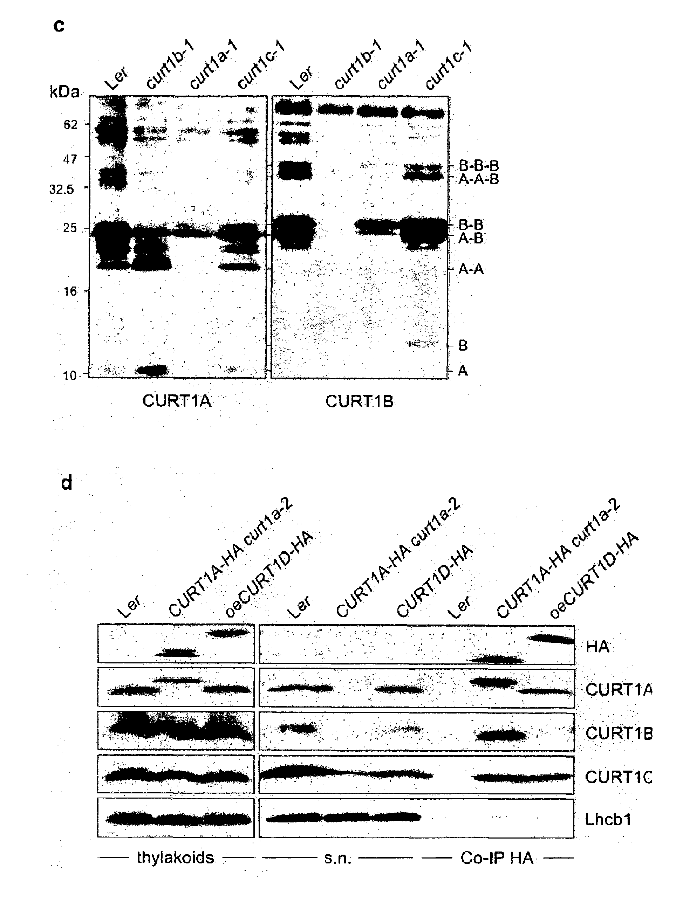

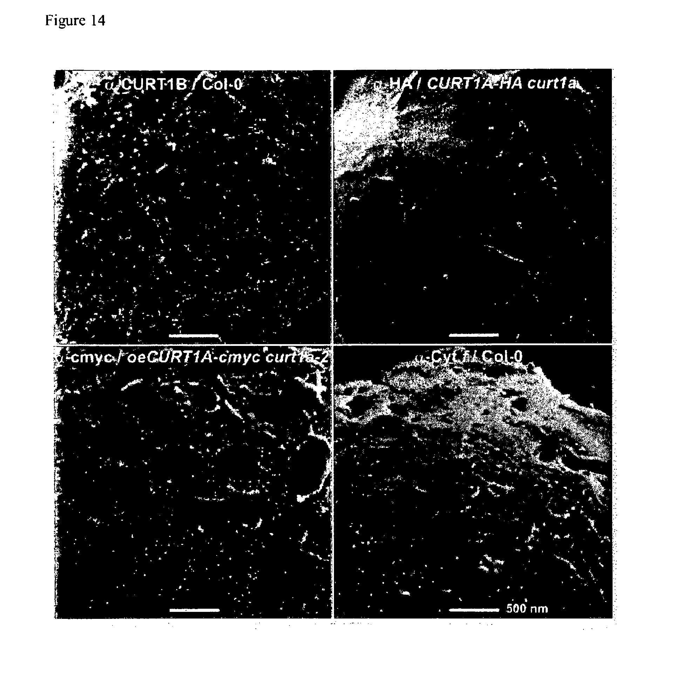

**Figure 3****. Analysis of accumulation and topology of CURT1 proteins a,** Accumulation of CURT1 proteins in different ecotypes of *A. thaliana.* Total protein extracts corresponding to 3 µg (CURT1A, B and C) or 10 µg (CURT1D) of Chl from three different *A. thaliana* ecotypes (Col-0, L*er*, and No-0) matching the background of the mutant lines used in this study were fractionated by SDS-PAGE, and blots were probed with antibodies raised against CURT1A, B, C and D. The Ponceau Red (P.R.) stained protein blot served as loading control. Note that for CURT1D no signal at the expected size of 15.7 kDa was obtained; the band at >16 kDa represents an unspecific signal. **b**, Schematic representation of the two possible topologies of CURT1 proteins, with the two TMs indicated by black boxes, the regions used to raise antibodies by grey boxes and the trypsin cleavage sites by asterisks. Below this, the expected sizes (in kDa) of the products of trypsin digestion of CURT1A, CURT1D-HA, CURT1B and CURT1A-HA recognised by the indicated antibodies in topology 1 / topology 2 are provided. Note that the regions used as epitopes for the CURT1A- and B-specific antibodies are predicted to contain trypsin cleavage sites, and therefore the corresponding products (provided in parentheses) should not be detectable. **c**, Immunoblot analysis ofthylakoid membrane preparations from WT (L*er*), oeCURT1D-HA and CURT1A-HA *curt1a-2* plants using antisera that recognise N-terminal epitopes of CURT1A and B, HA or PsbO (as a control for luminal thylakoid proteins). Samples were prepared before (-Trypsin) and after (+Trypsin) treatment with trypsin. In intact thylakoids, only stroma-exposed polypeptides are accessible to the enzyme. The behaviour of LHCII, visualised by Ponceau Red (P.R) staining, served as control for trypsin activity. If the N and C termini of the proteins faced the stroma (Topology 1 in panel B), tryptic digestion should cleave the segments of the proteins against which the antibodies were raised into fragments of less than 2.1 kDa. Tryptic cleavage of oppositely oriented CURT1 proteins (with the N- and C-termini facing the lumen; Topology 2 in panel B) at one site within the inter-TM region is expected to result in fragments between 6.3 and 8.6 kDa detectable by the antibodies specific for the HA-tag, CURT1A or B. As expected, PsbO on the lumenal side of the thylakoid membrane was not affected by trypsin, whereas Lhcblwas fragmented due to cleavage of its stroma-exposed regions. CURT1A-HA was completely digested without leaving any proteolytic fragment that could be detected by the HA antibody. Similarly, most of the CURT1D-HA and CURT1B proteins were digested without generating detectable fragments. CURT1A was only degraded to a minor degree, indicating that the protein might be protected from trypsin through interaction with other proteins. The fact that the fragments smaller than 2.1 kDa expected for Topology 1 are not detected by the CURT1A and B-specific antibodies can be explained by the destruction of their recognition sites, which contain predicted trypsin cleavage sites. In the case of the HA-tagged proteins, the expected small fragments may resist precipitation with acetone, which was used for trypsin inactivation, or be inefficiently transferred to the membrane during electroblotting. Nevertheless, the absence of fragments between 6.3 and 8.6 kDa clearly implies that CURT1 proteins adopt Topology 1. Moreover, the incomplete digestion of CURT1 proteins by trypsin indicates that they are protected, most likely because they form oligomers (see Fig. 5). Further support for the suggested CURT1 topology is the prerequisite of a stromal orientation of the antibody-binding domains for efficient Co-immunoprecipitation (see Fig. 5d) and immunogold-labelling (see Fig. 14).

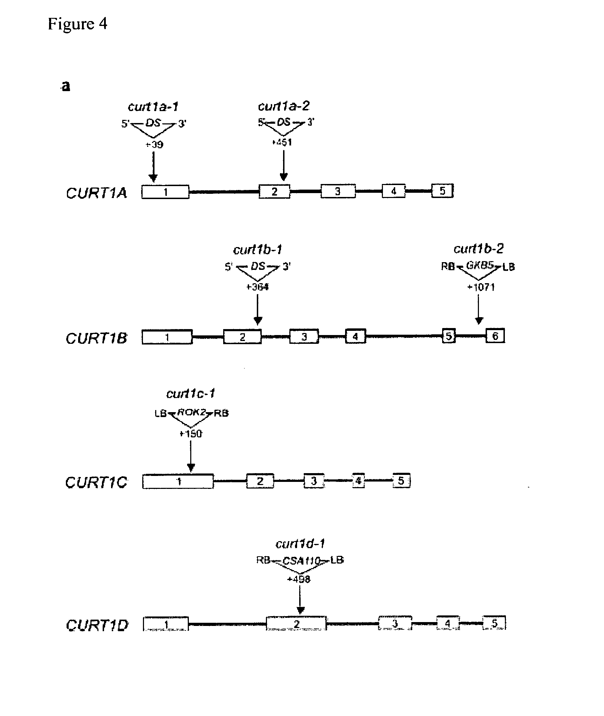

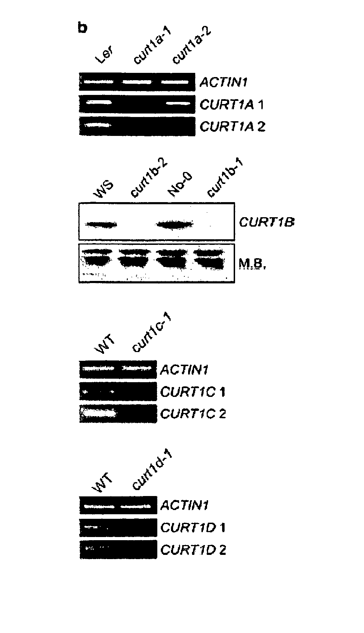

**Figure 4****. Mutations of *CURT1* genes and their effects on transcript accumulation a,** Insertion lines. Both *curt1a* alleles (*curt1a-1*, SGT_3_4785; *curt1a-2,* GT_5_19630) are in the Landsberg *erecta* (L*er*) background, carry insertions of the *Dissociation* (*Ds*) transposon and were obtained from the IMA (Parinov, 1999) and Exotic (Sundaresan, 1995) (www.jic.ac.uk/science/cdb/exotic) collections, respectively. The *curt1b-1* allele (15-2546-1) is from the RIKEN *Ds* collection (Ito, 2002) (http://rarge.psc.riken.jp/dsmutant/index.pl) and was induced in the Noessen (No-0) background, whereas *curt1b-2* (FLAG_218A11) originates from the FLAGdb/FST T-DNA collection (Samson, 2002) and is in the genetic background of the ecotype Wassilewskija (WS). In *curt1c-1* (SALK_052057) from the SALK T-DNA collection (Alonso, 2003) (http://signal.salk.edu; genetic background Col-0), the gene is disrupted by insertion of the ROK2 T-DNA. The *curt1d-1* mutant (SAIL_1240_C05) carries an insertion of the pCSA110 T-DNA and originates from the Syngenta SAIL collection (Sessions, 2002) (Col-0 background). The translated parts of exons (boxes), as well as intron sequences (lines), are depicted. For each insertion mutant used in this study, the site and the orientation of the insertion are indicated. The T-DNA and transposon insertions are not drawn to scale. The presence of gene insertions was confirmed by PCR with gene- and insertion-specific primers (Table 1) and sequencing of the PCR products. Loss of expression of the gene products was confirmed by RT-PCR or Northern analysis, as well as by immunoblot analysis (see Supplementary Fig. 14a). **b**, For *CURT1A*, *C* and *D*, RT-PCR analysis was performed on transcripts from WT and mutant plants with gene-specific primers located 5' (1) or 3' (2) to the insertion and *ACTIN1* primers as positive control. Because no suitable primer pair could be designed for one of the two insertions in *CURT1B,* Northern analysis was performed. Aliquots (30 µg) of total leaf RNA were fractionated on a denaturing agarose gel, transferred to a positively charged nylon membrane and hybridized with a *CURT1B* cDNA probe. Ribosomal RNA was stained with Methylene Blue (M.B.) as a loading control.

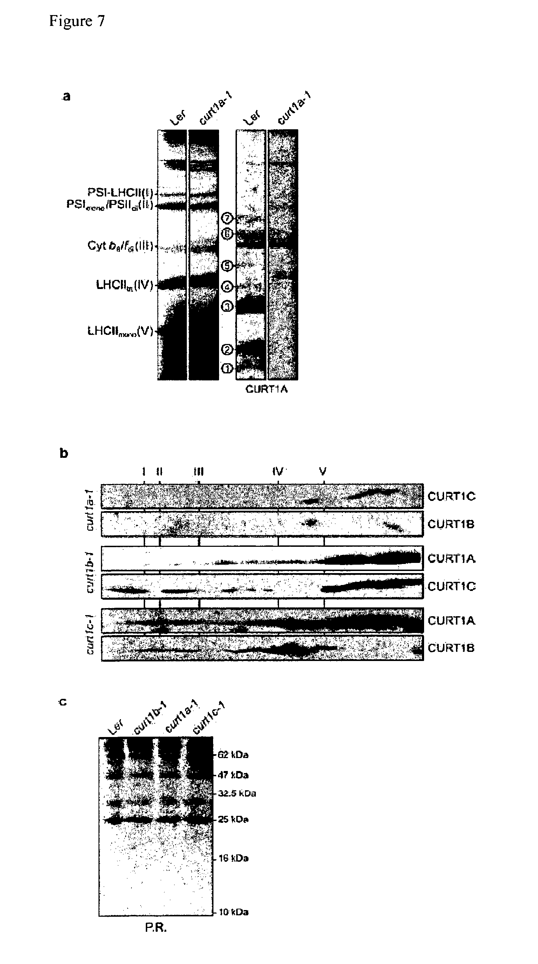

**Figure 5****. CURT1 proteins form oligomers a,** Relative abundance of CURT1 proteins in *curt1* mutants. **b**, 2D-BN/SDS-PAGE of thylakoid multiprotein complexes. See Fig. 7a for designation of multiprotein complexes (roman numerals). Circled Arabic numbers indicate the different heteromeric CURT1-containing complexes (A/B/C, CURT1A/CURT1B/CURT1C). f.p., free protein. **c**, Chemical crosslinking. On the right, the protein composition of crosslinked products is shown. **d**, CoIP analysis of HA-tagged CURT1 proteins. s.n, supernatant; Co-IP HA, co-immunoprecipitated proteins.

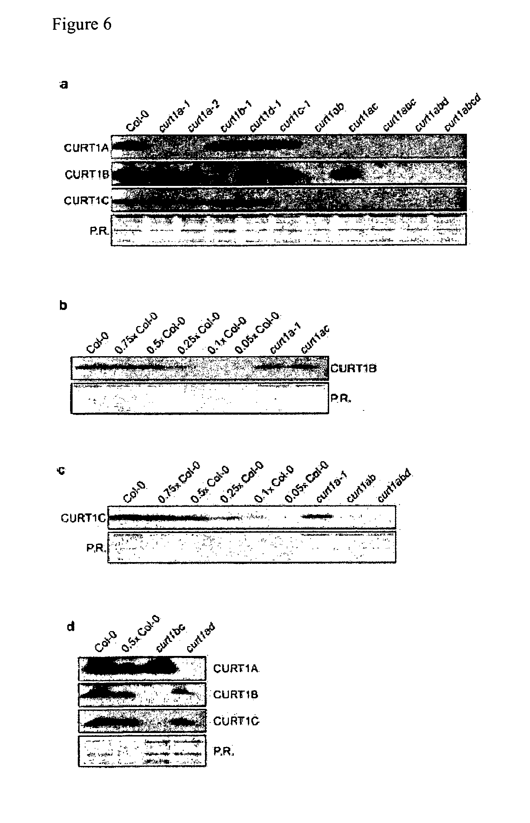

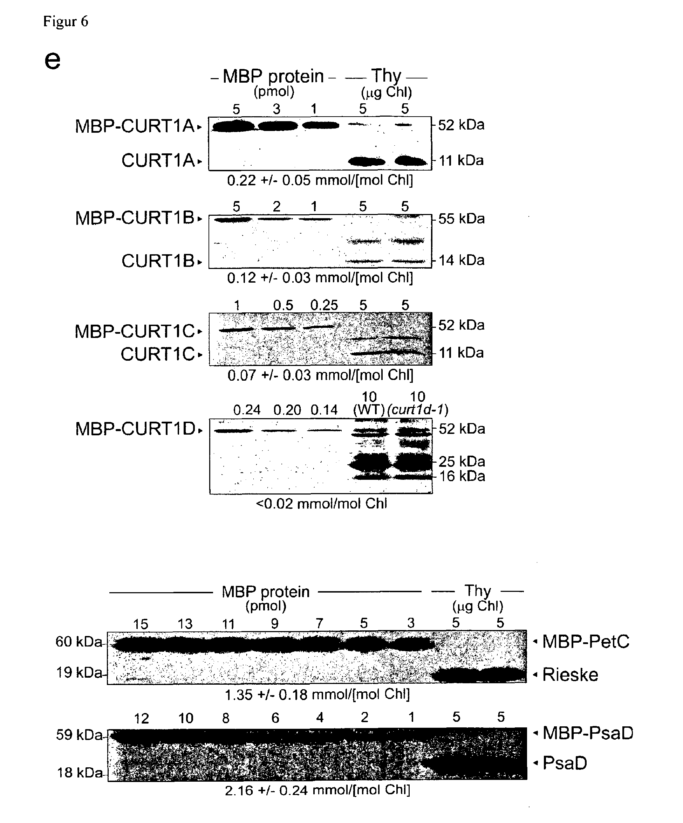

**Figure 6****. Analysis of CURT1 protein abundance** a-d, Total protein extracts from Col-0 (1x corresponds to 3 µg of Chl) or *curt1* mutant plants were fractionated by SDS-PAGE, and blots were probed with antibodies raised against CURT1A, B and C. The Ponceau Red (P.R.)-stained protein blot served as loading control. Note that L*er* and No-0 behaved like Col-0 (see Supplementary Fig. 5a). **e**, The four *Arabidopsis* CURT1 proteins were expressed in *E. coli* as C-terminal fusions to the maltose-binding protein (MBP), purified by affinity chromatography and quantified. Adequate quantities of these four MBP fusions, as well as of the MBP fusions of PetC and PsaD as controls, were titrated against thylakoid membrane protein preparations and subjected to immunoblot analyses. Representative results from five experiments are shown. The calculated concentration of the respective CURT1 protein in thylakoid preparations is given below each panel.

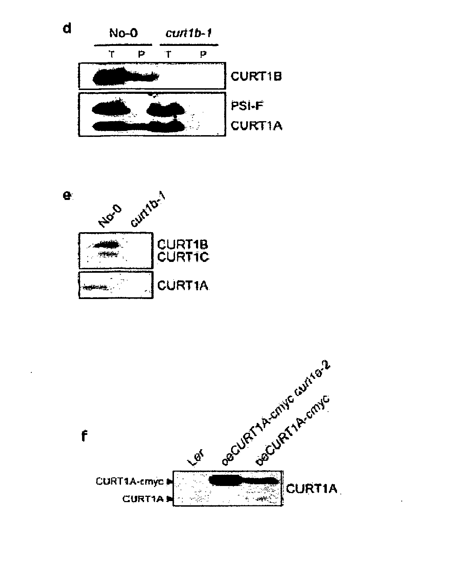

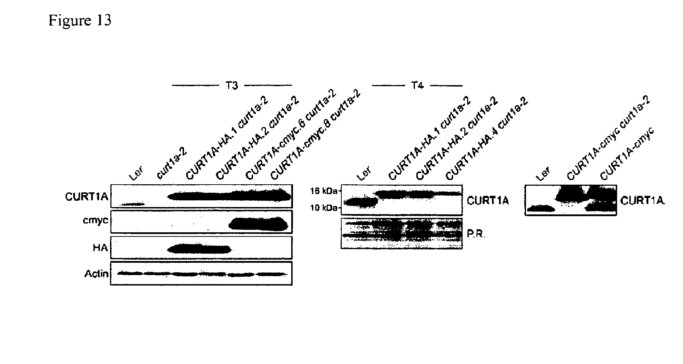

**Figure 7****. CURT1 proteins form oligomers a,** Blue-Native (BN) polyacryamide gel electrophoresis (PAGE) analysis of thylakoid multiprotein complexes. Left: WT (L*er*) and mutant (*curt1a-1*) thylakoids were solubilised with 1.6 % (w/v) digitonin and then fractionated by BN-PAGE. The bands were assigned to the PSI-LHCI-LHCII supercomplex (PSI-LHCII), PSI-LHCI monomers (PSIₘₒₙₒ) and PSII dimers (PSII_{di}), dimeric Cytochrome *b₆*/*f* complexes (Cyt *b₆*/*f_{di}*) trimeric (LHCIItri) and monomeric (LHCIIₘₒₙₒ) LHCII (Armbruster, 2010, Pesaresi, 2009). Right: After immunoblotting, the blots were immunodecorated with CURT1A-specific antibodies. The circled Arabic numbers indicate the different CURT1A containing complexes. The strong signal between bands 5 and 6 derives from chemiluminescence of the hemes contained in the Cyt *b₆*/*f* complex. **b**, Immunoblots of thylakoid proteins from *curt1a-1*, *curt1b-1* and *curt1c-1* separated by 2D gel electrophoresis were analysed as in Fig. 5b. The positions of the major thylakoid multi-protein-pigment complexes are indicated by Roman numerals as in panel a. **c**, As a loading control for Fig. 5c, blots with crosslinked products were stained with Ponceau Red (P.R.). **d**, Identification of interactors of CURT1B by CoIP. Thylakoid membranes from WT (No-0) and *curt1b-1* mutants were co-incubated with a CURT1B-specific antibody. Complexes were solubilised with 1.6% (w/v) digitonin and precipitated with Protein-A agarose before centrifugation. The pellet was repeatedly washed and bound proteins were eluted by adding SDS-PAGE loading dye. Thylakoid membrane (T) and immunoprecipitated (P) proteins were analysed by SDS-PAGE and Western analysis employing antibodies specific for CURT1B, CURT1A and PSI-F. **e**, CoIP analysis of CURT1B was performed as in panel d, except that the immunoprecipitated proteins were also analysed with a CURT1C-specific antibody. **f**, CoIP analysis was carried out with antibodies against cmyc on thylakoid proteins from WT (L*er*) or plants overexpressing CURT1A-cmyc either in the *curt1a-2* or the WT background (see Fig. 13). Immunoprecipitated proteins were detected with an antibody raised against CURT1A.

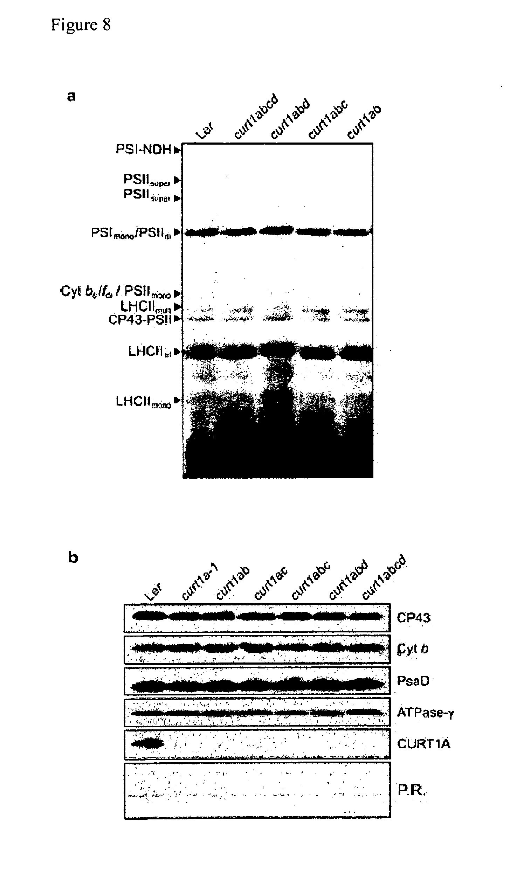

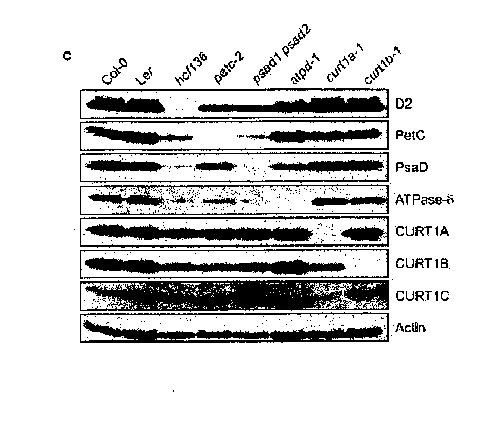

**Figure 8****. CURT1 proteins are not constitutive components of the major thylakoid photosynthesis complexes a**, Blue Native (BN) polyacryamide gel electrophoresis (PAGE) analysis of thylakoid multi-protein complexes. Thylakoids corresponding to 30 µg Chl from WT (L*er*) and *curt1* mutant plants were solubilised with 1.0 % (w/v) dodecyl-β-D-maltoside (β-DM). The extracts were then fractionated by BN-PAGE. The bands detected were identified as specific protein complexes in accordance with previously published profiles (Armbruster, 2010, Granvogl, 2006, Peng, 2008, Schwenkert, 2006). PSI-NDH supercomplex (PSI-NDH), PSII supercomplexes (PSIIₛᵤₚₑᵣ), PSI monomers and PSII dimers (PSIₘₒₙₒ and PSII_{di}), PSII monomers and dimeric Cyt *b₆*/*f* (PSIIₘₒₙₒ and Cyt *b₆*/*f_{di}*), PSII monomers w/o CP43 (CP43-PSII), multimeric (LHCIIₘᵤₗₜ), trimeric (LHCIIₜᵣᵢ) and monomeric (LHCIIₘₒₙₒ) LHCII. **b**, The composition of thylakoid complexes from WT (L*er*) and *curt1* mutants was analysed as in Fig. 14a, except that aliquots corresponding to 5 µg Chl were used. Antibodies specific for the PSII subunit CP43, the D subunit of PSI (PsaD), Cyt *b₆* from the Cyt *b₆*/*f* complex, the γ-subunit of the chloroplast ATP synthase and CURT1A were used. Ponceau Red (P.R.) staining served as the loading control. **c**, Immunoblot analysis of leaf proteins (corresponding to 40 µg of total protein) from WT (Col-0 and L*er*), *curt1a-1* and *curt1b-1* mutants, and mutants devoid of PSII (*hcf136*), PSI (*psad1 psad2*), Cyt *b₆*/*f*(*petc-2*), or cpATPase (*atpd-1*). Antibodies specific for CURT1A, B, C and the respective thylakoid multiprotein complexes, or actin as control, were used.

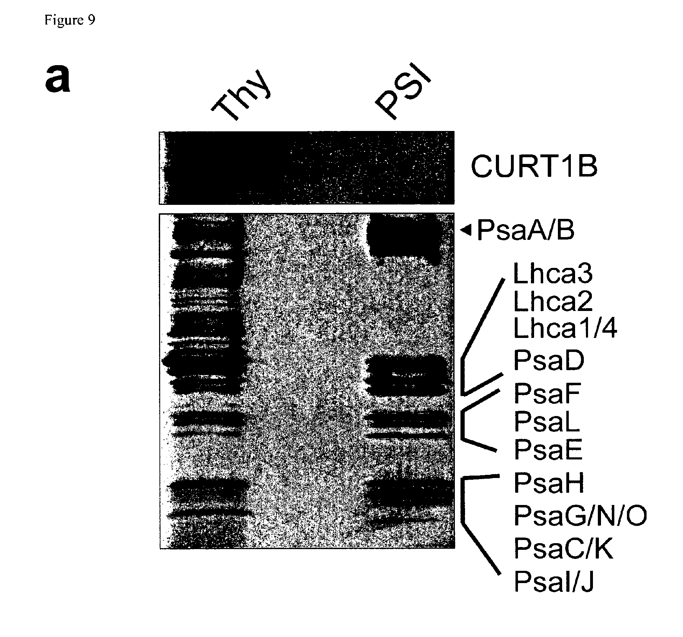

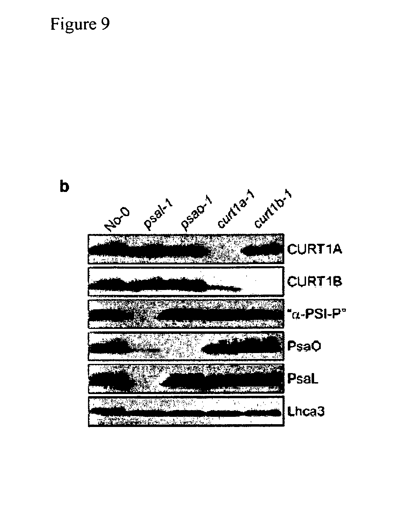

**Figure 9****. CURT1B is not a subunit of PSI a,** Immunoblot analysis of thylakoid and PSI preparations with a CURT1B-specific antibody. PSI was isolated from WT thylakoids by sucrose-gradient centrifugation (DalCorso, 2008). Thylakoid and PSI proteins corresponding to 10 µg Chl were separated by SDS-PAGE and either stained with Coomassie Blue (lower panel) or subjected to immunoblot analysis. Positions of PSI subunits previously identified by immunodetection are indicated. **b**, Immunoblot analysis of thylakoid proteins (5 µg Chl) from WT (No-0) and mutant (*psal-1, psao-1, curt1a-1* and *curt1b-1*) plants. Antibodies directed against CURT1A, CURT1B, PSI-L, PSIO or Lhca3, as well as an antibody ("α-PSI-P") previously raised against a peptide from CURT1B (previously denoted TMP14 and PSI-P) (Khrouchtchova, 2005), were used. Clearly, the "α-PSI-P" antibody does not recognise CURT1B but crossreacts with PSI-L even though the two proteins have no apparent similarity. This led to the erroneous conclusion that CURT1B was a PSI subunit (PSI-P).

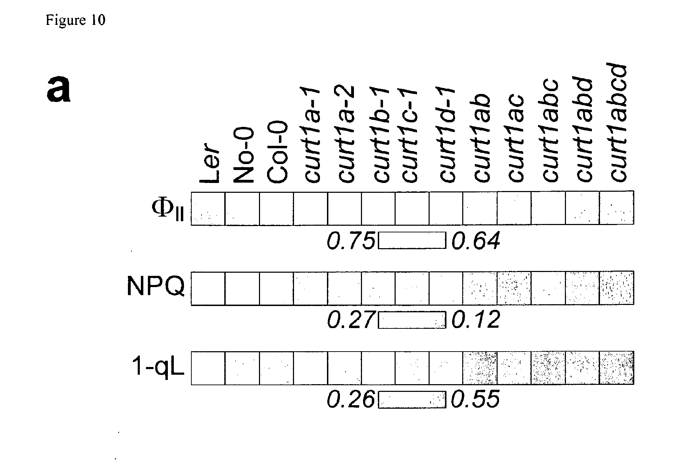

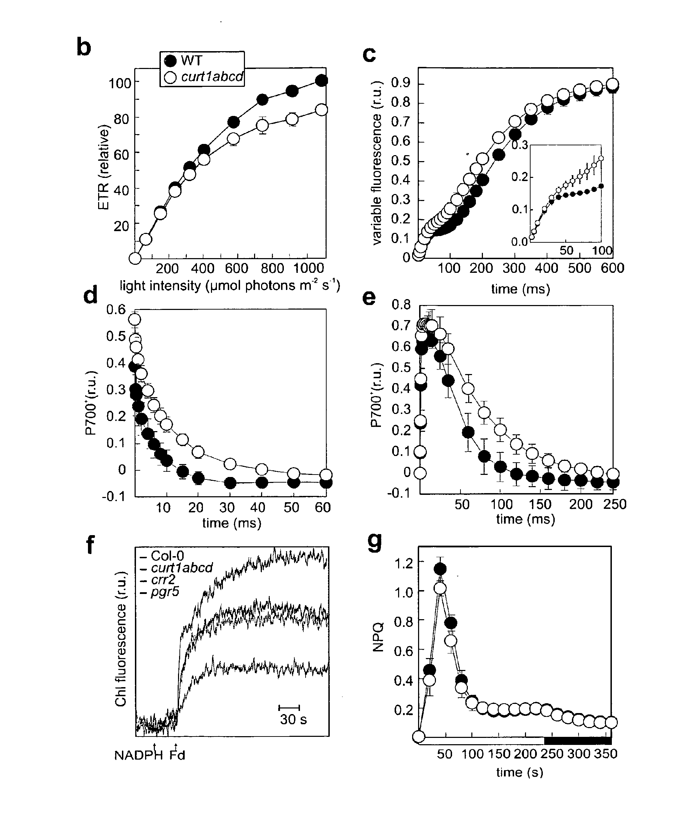

**Figure 10****. Characterisation of photosynthetic electron flow a,** Effective quantum yield (ΦII), NPQ and 1-qL (as listed in Table 2) for WT and *curt1* mutant plants are indicated on a gray scale. **b-e** and **g,** Average values SD (bars) for three to five different plants are indicated by filled (WT) or open (*curt1abcd*) circles. **b**, Dependence of the relative electron transport rate (ETR) on light intensity. ETR is expressed relative to the maximum ETR in the WT (100%). **c**, Time course of Chl a fluorescence during illumination with actinic light at 100 µmol photons m⁻²s⁻¹. **d**, Kinetics of re-reduction of P₇₀₀⁺ following a short saturating light pulse (7000 µmol photons m⁻²·s⁻¹) that led to the complete oxidation of P₇₀₀. **e,** P₇₀₀ oxidation and re-reduction kinetics during a 250-ms saturating light pulse. **f,** Quantification of CET *in situ.* Increases in chlorophyll fluorescence were measured in ruptured chloroplasts after the addition of NADPH and Fd as described (Munekage, 2002). As controls, the mutants *pgr5* (defective in Fd-dependent CET) and *crr2* (defective in NDH-dependent CET) were employed. **g**, Time courses of induction and relaxation of NPQ monitored during dark-to-light (80 µmol photons m⁻²s⁻¹, white bar) transitions. The 4-min light period (white bar) was followed by a 2 min dark period (black bar).

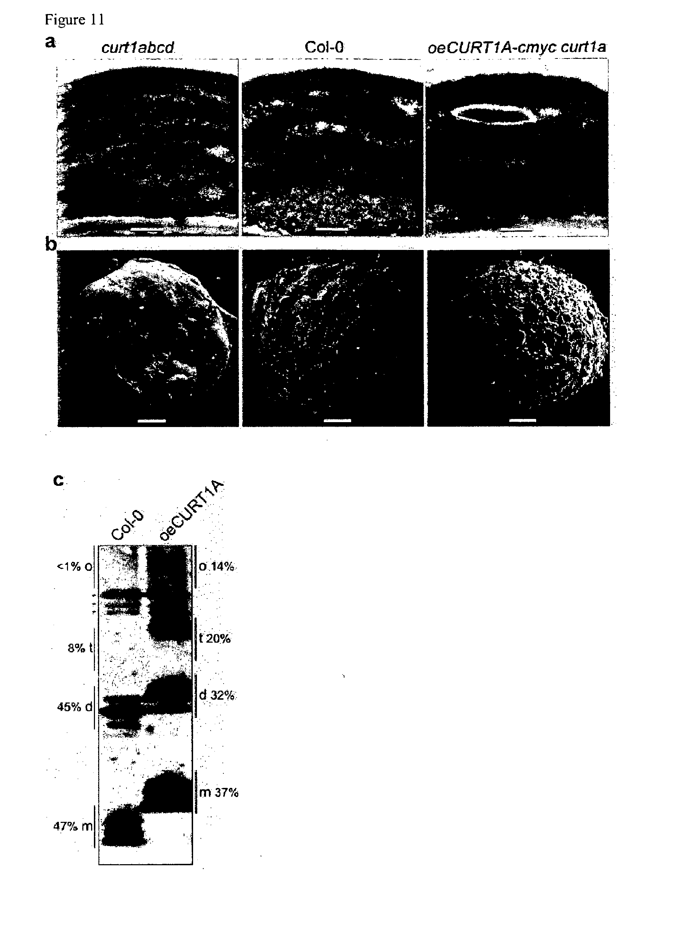

**Figure 11****. Thylakoid architecture depends on CURT1 protein levels a,** TEM micrographs of *curt1abcd* leaves, WT (Col-0) and *CURT1A-cmyc curt1a-2* lines that express tagged CURT1A proteins at - 400% of WT CURT1A levels. **b**, Topographic view of envelope-free chloroplasts from the same genotypes as in panel a obtained by scanning electron microscopy (SEM). **c**, WT and *CURT1A-cmyc curt1a-2* (oeCURT1A) thylakoids were crosslinked, analysed as in Fig. 5c, and CURT1A signals were quantified. Intensities for bands representing monomers (m), dimers (d), tetramers (t) and oligomers (o) are given in % next to the corresponding genotype. Asterisks indicate unspecific bands.

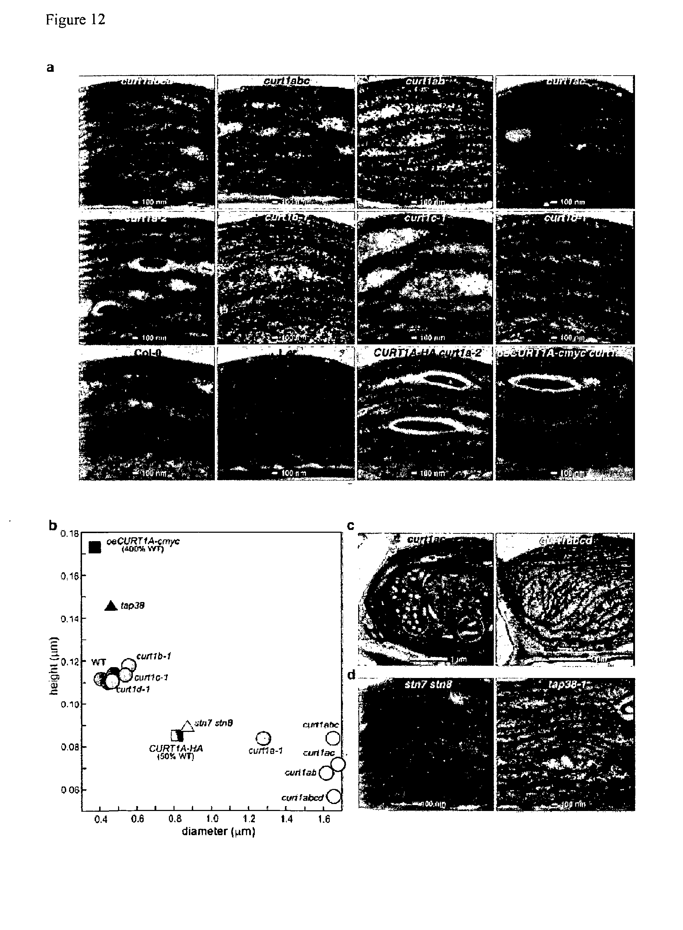

**Figure 12****. The degree of stacking of grana depends on the levels of CURT1 proteins a,** TEM micrographs of ultrathin sections of leaves from WT (L*er*, Col-0), *curt1* mutants, and lines expressing tagged CURT1A proteins (*CURT1A-HA curt1a-2* and *CURT1A-cmyc curt1a-2*). Note that the *CURT1A-HA curt1a-2* and *CURT1A-cmyc curt1a-2* lines express ∼50% and 400% of WT levels of CURT1A, respectively. **b**, Scatter plot indicating the average height (y-axis) and diameter (x-axis) of thylakoid stacks in the genotypes shown in panel a and d. Average values for ≥7 chloroplasts are provided; a complete list including standard deviations is provided in Table 3. **c,d**, Sections of chloroplasts as in panel a from *curt1ab* and *curt1abcd* (**c**) and from *stn7 stn8* and *tap38* mutant chloroplasts.
**Figure 13****. Overview of tagged CURT1A variants used in this study** The *CURT1A* cDNA was inserted into binary vectors between the 35S promoter and the sequences coding for the HA or cmyc tags and introduced into *curt1a-2* plants. In the left panel, transgene expression analysis was carried out on total protein extracts from WT and T3 plants homozygous for the transgenes. CURT1A is overexpressed in all four lines tested and corresponding lines are designated in the following as "oeCURT1A-HA" or "oe-CURT1A-cmyc". In the middle panel, thylakoid proteins from T4 plants expressing CURT1A-HA were analysed by immunoblot analysis. These lines express CURT1A at lower levels than WT and are designated as "CURT1A-HA". In the right panel, expression of oeCURT1A-cmyc in *curt1a-2* plants and WT (T3 generation) is shown.
**Figure 14****. CURT1 proteins are enriched at grana margins** SEM images of envelope-free chloroplasts from WT (Col-0), *CURT1A-HA curt1a-2* and *oeCURT1A*-*cmyc curt1a-2* plants, following immunogold-labelling with antibodies raised against CURT1B, Cyt f, HA and cmyc.

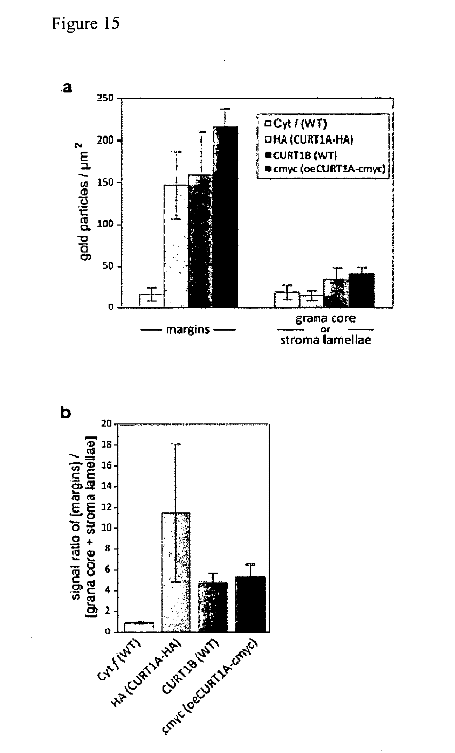

**Figure 15****. Quantification of the distribution of immunogold-labelled CURT1 proteins in envelope-free chloroplasts a,** Protein quantification is based on SEM analysis of envelope-free chloroplasts from WT (Col-0), *CURT1A-HA curt1a-2* and *oeCURT1A-cmyc curt1a-2* plants after immunogold labelling with antibodies raised against CURT1B, Cyt *f*, HA and cmyc, as exemplarily shown in Fig. 4. At least eight independent SEM pictures were analysed for each immunogold-labelling experiment. "Margins" were defined as the area covering around 5 nm in both directions of the visual membrane bending zones. The residual surface was combined to represent the grana core and stroma lamellae regions. Note that the minor amounts of CURT1 proteins in Col-0 and *oeCURT1A*-*cmyc* plants assigned to the grana core or stroma lamellae regions were also mainly detected close the curved membrane borders but just outside of the region arbitrarily defined as "margins". **b**, The distribution ratios of the CURT1 proteins and, as control, Cyt *f* were determined based on the specific accumulation of gold particles in the margins or the remaining membrane area (grana core + stroma lamellae) of the same SEM images analysed in panel a.

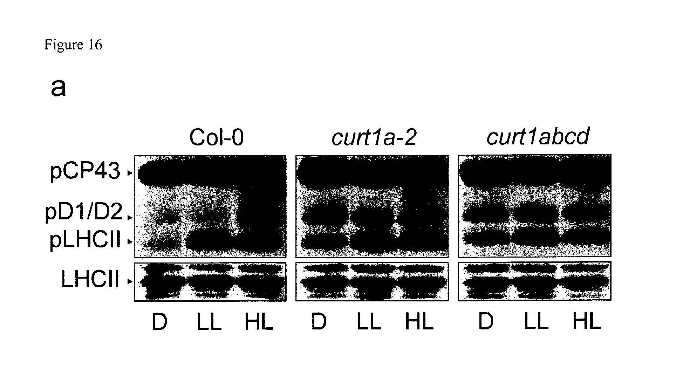

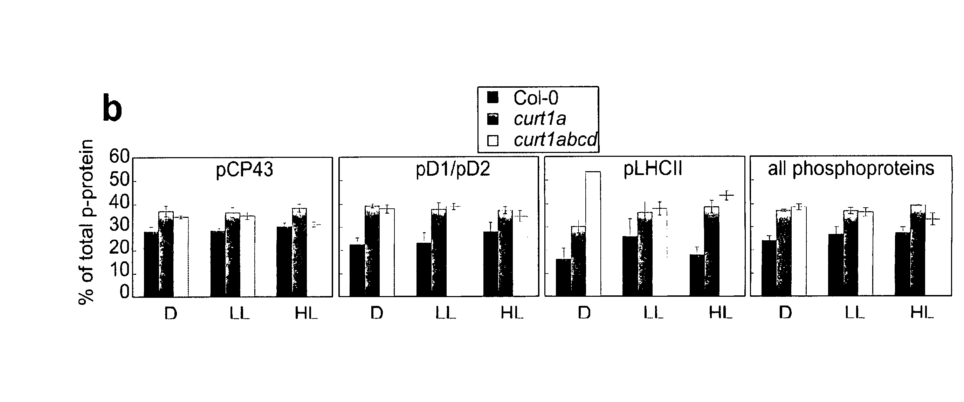

**Figure 16****. Quantification of thylakoid protein phosphorylation a,** Thylakoid proteins were extracted from WT (Col-0) and *curt1* mutant (*curt1a-2* and *curt1abcd*) plants that had been kept for 16 h in the dark (D), subsequently exposed to low light (LL) for 2 h, and then to high light (HL) for 2 h. After fractionation by SDS-PAGE, phosphorylation of thylakoid proteins was detected by immunoblot analysis with a phos-phothreonine-specific antibody. pCP43, phosphorylated CP43; pD1/D2, phosphorylated D1/D2; pLHCII, phosphorylated LHCII. The lower panels show portions of the Ponceau S-stained PA gels as loading control; the position of LHCII is indicated. **b,** Total signals for pCP43, pD1+pD2 and pLHCII and all four phosphoproteins together in panel a and two repetitions of the experiment were quantified. Average values ± SD are provided. The total of the signals measured from the three genotypes for a given phosphoprotein and light condition was set to 100%.

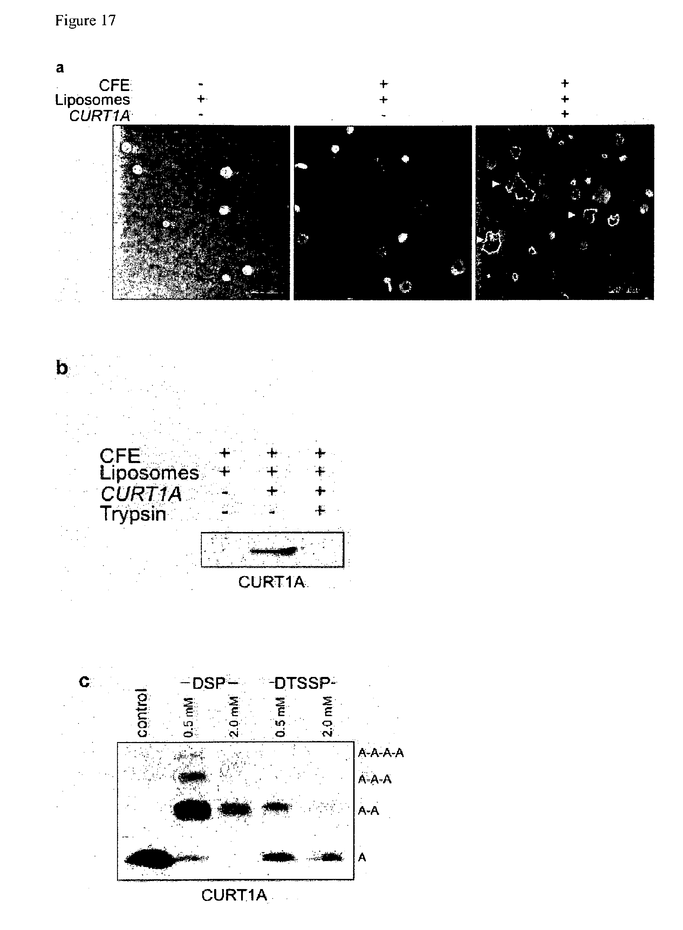

**Figure 17****. CURT1A oligomers tubulate liposomes a,** Liposomes with thylakoid-like lipid composition were incubated with *E. coli* cell-free extracts (CFEs) either containing a template (*CURTIA*) for *de-novo* CURT1A synthesis or not. After purification, liposomes were analysed by TEM. Tubular structures are indicated by white arrowheads. **b,** Liposomal integration of CURT1A. Liposomes from reaction mixtures with indicated composition were purified and subjected to SDS-PAGE and Western analysis. Trypsination of proteoliposomes confirmed the surface-exposed and thylakoid-like topology of CURT1A. **c,** CURT1A proteoliposomes were crosslinked with membrane-permeable (DSP) and -impermeable (DTSSP) crosslinkers. Crosslinking products ("A-A" to "A-A-A-A") were detected by SDS PAGE and Western analysis.

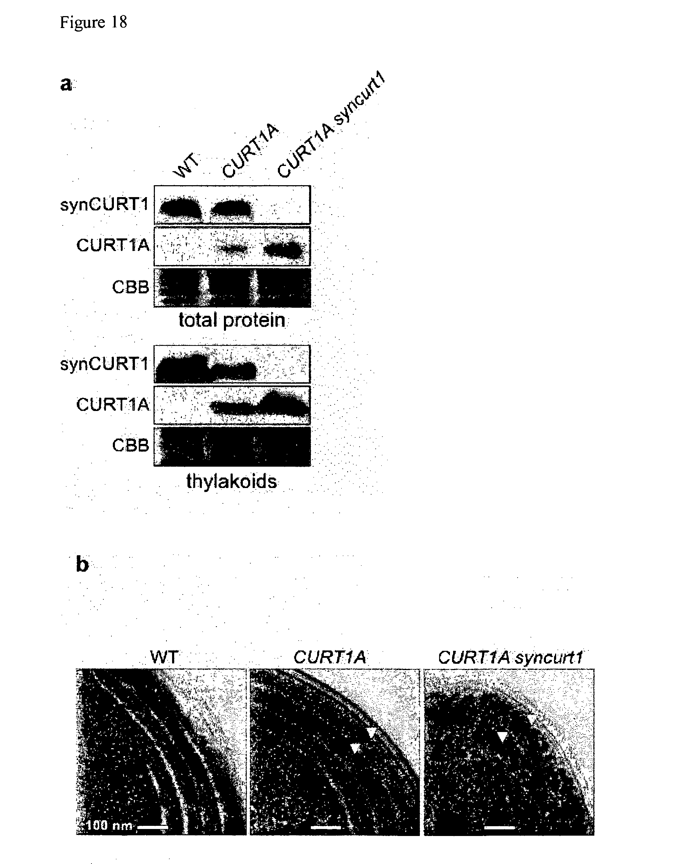

**Figure 18****. Heterologous expression of *Arabidopsis* CURT1A in *Synechocystis* alters thylakoid membrane structure a,** Different *Synechocystis* mutant strains that express CURT1A in the presence (*CURTIA*) or absence (*CURT1A syncurt1*) of endogenous *Synechocystis* CURT1 (synCURT1) were generated. Total proteins and thylakoid proteins of WT and mutant *Synechocystis* strains were analysed by SDS-PAGE and immunoblot analysis, employing antibodies specific for CURT1A and synCURT1. Coomassie staining (CBB) served as loading control. **b,** The same genotypes were analysed regarding their thylakoid structure by TEM. Exemplary detached phycobilisomes are indicated by white arrowheads.

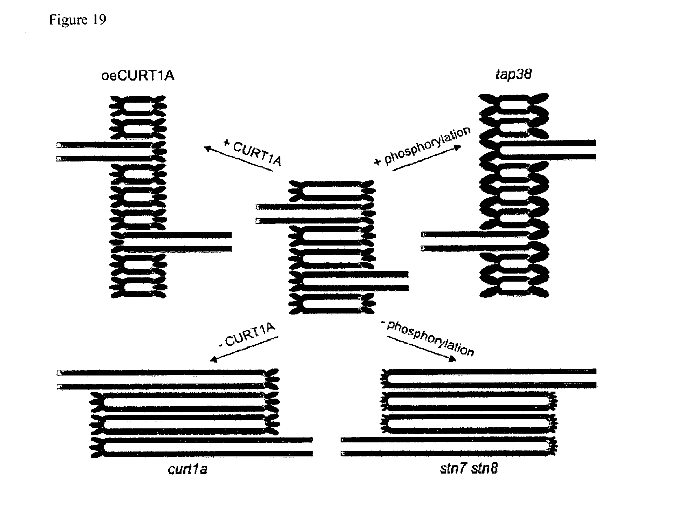

**Figure 19****. Schematic model for the effects of CURT1 proteins and their phosphorylated forms on grana stacking** CURT1 proteins are symbolised by black ellipses. Hypothetical phosphorylation-induced changes in CURT1 structure or oligomerisation state are indicated by changes in ellipse size. Note that changes in grana stacking in the *stn7 stn8* and *tap38* mutants might alternatively be caused by changes in repulsion between membrane layers due to altered PSII phosphorylation (Fristedt, 2009), although this effect is overridden in *curt1* mutants with increased thylakoid phosphorylation.

### EXAMPLES

### Thylakoids contain CURT1 oligomers

Nuclear photosynthetic genes are often transcriptionally coregulated in *Arabidopsis* (Biehl, 2005), and this transcriptional signature can be used to identify novel photosynthetic genes (DalCorso, 2008). Three *Arabidopsis* genes of unknown function but featuring this transcriptional signature (Fig. 1a), here designated *CURT1A, B* and *C* (because of their function in the curvature of thylakoids, see below), belong to a small gene family with four members. Transcripts encoding CURT1D, the fourth member of the family, cluster with transcripts for 4 non-photosynthetic proteins (Fig. 1a). All four CURT1 proteins share a predicted N-terminal chloroplast targeting peptide, two predicted transmembrane domains (TMs), a tentative N-terminal amphipathic helix, and are relatively small (11.0-15.7 kDa; Fig. 2a, Fig. 1b,c). Genes for CURT1 homologues exist in the nuclear genomes of green algae, plants, and, more distantly related, also in cyanobacteria (Fig. 1d).

CURT1B was already shown to be located in thylakoids (Hansson, 2003), and each of the other three *Arabidopsis* CURT1 proteins has been detected at least once in chloroplasts in proteomic studies (Peltier, 2004; Friso, 2004) (Table 4). In fact, *in-vivo* subcellular localisation of full-length N-terminal fusions of CURT1A, C and D to the dsRED protein showed that all three proteins are chloroplast-located (Fig. 1e). When wild-type (WT) chloroplasts were fractionated into thylakoids, stroma and two envelope fractions and subjected to immunoblot analysis, CURT1A, B and C were exclusively detected in thylakoids (Fig. 2b). Overexpression of the low-abundant CURT1D (Fig. 3a) with a C-terminal HA-tag (oe-CURT1D-HA) confirmed that CURT1D is also a thylakoid protein (Fig. 2b). To clarify whether the CURT1 proteins constitute integral or peripheral thylakoid proteins, WT (Col-0) thylakoids were treated with alkaline and chaotropic salts to release membrane-associated proteins (Fig. 2c). In this assay, CURT1A and C behaved like the integral proteins Lhcb1 (with three TMs) and Cyt *b*₆ (four TMs), whereas CURT1B and CURT1D-HA could be partially extracted from thylakoids with NaSCN - similar to the Rieske protein PetC (with one TM).

To determine the membrane topology of the CURT1 proteins, thylakoids of WT plants and of lines expressing HA-tagged versions of CURT1 proteins were subjected to mild digestion with trypsin such that only the stroma-exposed face was accessible to the protease. The fragmentation pattern (Fig. 3b,c) and phosphorylation behaviour (Table 4) of CURT1 proteins, as well as additional experimental evidence (see 5 legend of Fig. 3c), suggest that they are located in the thylakoid membrane with their N- and C-termini facing the stroma. Starting from insertion mutants for the four *Arabidopsis CURT1* genes (Fig. 4), double and triple mutants and the quadruple mutant were generated. Lack of CURT1D and, in particular, CURT1A decreased the abundance of other CURT1 proteins (Fig. 5a, Fig. 6a-d). In the four double mutants analysed, a superadditive effect was observed, whereas in the two triple mutants (*curt1abc* and *curt1abd*) and the quadruple mutant (*curt1abcd*) no CURT1 protein was detectable at all.

To estimate their absolute abundance, known quantities of C-terminal fusions of the four CURT1 proteins to the maltose-binding protein (MBP), as well as of MBP fusions of PetC from the cytochrome *b₆*/*f* complex and the PSI subunit PsaD as controls, were titrated against thylakoid preparations and subjected to immunoblot analysis. Levels of PetC (1.35±0.18 mmol/mol chlorophyll (Chl)) and PsaD (2.16±0.24 mmol/mol Chl) found in thylakoid preparations (Fig. 6e) were in good agreement with published data (Kirchhoff, 2002). CURT1A was the most abundant CURT1 protein (0.22±0.05 mmol/mol Chl), followed by CURT1B (0.12±0.03 mmol/mol Chl) and CURT1C (0.07±0.03 mmol/mol Chl), whereas CURT1D was expressed at very low levels (Fig. 6e).

The interdependence of CURT1 protein accumulation suggests that the proteins physically interact. To further study these interactions, immunoblot analysis of thylakoid protein complexes separated by Blue-Native PAGE (BN-PAGE) were performed. Seven CURT1A-containing complexes, none of which comigrated with the known high-abundance complexes (Armbruster, 2010; Pesaresi, 2009), were identified in WT plants (Fig. 7a). The complexes resolved by BN-PAGE were then separated into their subunits by SDS-PAGE in the second dimension, and subjected to immunoblot analysis. This analysis showed that three complexes contained all three CURT1 proteins tested, while CURT1A and C are both present in a further three complexes (Fig. 5b). In the single mutants *curt1a-1, curt1b-1* and *curt1c-1* the remaining two CURT1 proteins still co-migrate, forming in *curt1b-1* and *curt1c-1* some complexes with an even higher molecular weight than in WT (Fig. 7b).

To corroborate the interactions between CURT1 proteins, thylakoid proteins were subjected to chemical crosslinking. Comparison of the pattern of signals obtained after SDSPAGE and immunoblot analysis from crosslinked WT thylakoid proteins with the ones obtained from *curt1a-1, curt1b-1* and *curt1c-1* mutants showed that CURT1A and B are present in several distinct complexes, forming homodimers, heterodimers, homotrimers (CURT1B) and heterotrimers (Fig. 5c, Fig. 7c). Moreover, coimmunoprecipitation experiments, carried out with thylakoids from plants expressing HAtagged CURT1A (CURT1A-HA *curt1a-2),* cmyc-tagged CURT1A or overexpressing CURT1D-HA, confirmed the existence of multiple interactions between different CURT1 proteins, as well as between the same CURT1 proteins (Fig. 5d, Fig. 7d-f).

### CURT1 depletion affects photosynthesis indirectly

Because of their low abundance, CURT1 proteins are unlikely to be constitutive subunits of the major thylakoid multiprotein complexes. To further test the relationship between CURT1 proteins and other thylakoid proteins, BN- and SDS-PAGE analyses were performed, demonstrating that lack of even all four CURT1 proteins had no effect on the accumulation of the major thylakoid multiprotein complexes (Fig. 8a,b). Vice versa, CURT1 proteins accumulated independently of the major thylakoid protein complexes (Fig. 8c). In addition, CURT1B is not a subunit of PSI - contrary to an earlier suggestion (Khrouchtchova, 2005) (Fig. 9).

Measurement of various photosynthetic parameters showed that CURT1 proteins are not involved in a specific photosynthetic function and that their absence probably perturbs photosynthesis indirectly (see the chapter Effect of CURT1 depletion on photosynthesis, Table 2, Fig. 10).

### CURT1 proteins modify thylakoid architecture

The pleiotropic effects on photosynthesis observed in plants without CURT1 proteins suggest that thylakoids might be affected at a more basic level. We therefore examined the thylakoid lipids and found that their composition was not markedly altered in *curt1* mutants (Table 5). We then investigated the effect of *curt1* mutations on the architecture of the thylakoid system by transmission electron microscopy (TEM) (Fig.11a, Fig. 12). In WT chloroplasts, the average diameter of grana stacks lies between 0.41 µm and 0.47 µm (depending on ecotype) and average height is around 0.11 µm (Fig. 12b, Table 3). The corresponding values for *curt1abcd* plants, on the other hand, were 1.66 and 0.06 µm (Fig. 12b, Table 3), as thylakoids were disorganised with extended stretches of unstacked membranes and broader stacks made up of fewer layers than in WT (Fig. 11a). Moreover, in double, triple and quadruple mutants, thylakoids were occasionally curved instead of forming horizontal layers, and also contained vesicular structures (Fig. 12c). The WT-like grana phenotype of the three single mutants *curt1b-1, curt1c-1* and *curt1d-1* and the intermediate phenotype of *curt1a* plants (average grana stack diameter/height of 1.32/0.08 µm) is in accord with their photosynthetic phenotype (see above). Because thylakoids that lack grana stacks represent the primordial type, and are ubiquitous in cyanobacteria, it is not surprising that *curt1abcd* plants are still capable of performing photosynthesis (see above), although they are virtually devoid of grana. However, the complex perturbations in photosynthesis found in *curt1abcd* plants are compatible with the fact that important features of photosynthesis in land plants are facilitated by the lateral heterogeneity provided by grana (Mullineaux, 2005; Mustardy, 2003: Daum, 2011; Dekker, 2005; Anderson, 2008; Trissl, 1993).

To obtain further evidence for a relationship between grana structure and the abundance of CURT1 proteins, tagged lines expressing varying levels of CURT1A (the major CURT1 8 protein) were investigated by TEM. CURT1A-HA plants with about 50% of WT CURT1A levels (Fig. 13) yielded average values for diameter/height of grana stacks of 0.82/0.09 µm, intermediate between those measured in *curt1a* and WT plants (Fig. 12,b; Table 3). Plants overexpressing CURT1A-cmyc *(oeCURT1acmyc curt1a-2),* with CURT1A levels about four times higher than in WT plants (Fig. 13), had slimmer but higher grana stacks than WT (average diameter/height: 0.37/0.17 µm)(Fig. 11a, Fig. 12b).

To assess the three-dimensional arrangement of thylakoid membranes, envelope-free chloroplasts from WT, the quadruple mutant *curt1abcd* and *oeCURT1a-cmyc curt1a-2* plants were analysed by high-resolution scanning electron microscopy (SEM). Strikingly, the top view of the thylakoid system confirmed that extended sheets of stroma thylakoids predominate in *curt1abcd* chloroplasts (Fig. 11b). Conversely, in thylakoids from CURT1Acmyc overexpressors, the grana stacks were much more abundant but displayed a significantly reduced diameter compared to WT, and stroma lamellae regions adopted more tubular structures. Moreover, quantification of CURT1A crosslinking products from thylakoids of WT and CURT1A-cmyc overexpressors showed that the fraction of interacting CURT1A proteins is increased in the overexpressor (Fig. 11c).

Taken together, the electron microscopy data clearly show a striking association between the dose of CURT1 proteins and the architecture of thylakoid membranes. In the absence of CURT1 proteins, grana consist of fewer but broader layers of membrane, whereas overexpression of CURT1A is characterised by higher stacks of smaller grana discs, tubular stroma lamellae and increased oligomerisation of CURT1 proteins. The negative correlation between the diameter and height of grana must be attributed to the fact that a fixed proportion of thylakoid membrane is incorporated into the grana stacks (Albertsson, 2004). Therefore, an increase in the diameter of grana must be compensated for by a decrease in the number of stacks and *vice versa.*

The localisation of the CURT1A and B proteins was determined by immunogold labelling of envelope-free chloroplasts followed by SEM. To this end, envelope-free chloroplasts of WT, CURT1A-HA and oeCURT1A-cmyc plants were first immunodecorated with specific antibodies and then treated with the appropriate gold-labelled secondary antibodies (Fig. 14). Signals for CURT1B (in WT), HA (in CURT1A-HA) and cmyc (in oeCURT1A-cmyc) were quantified (Fig. 15) and found to be highly enriched at the lateral boundaries of the thylakoid membranes which face the stroma, where the membrane curves to form grana margins. In fact, signals for CURT1A-HA were almost exclusively associated with margins, whereas CURT1B- and cmyc-specific antibodies also labelled a few sites outside of the margins. In particular, signals were also detected in the tubular structures branching from the cylindrical grana stack in oeCURT1A-cmyc plants. When envelope-free chloroplasts from WT were immunolabelled with antibodies recognizing Cyt *f* as control, signals were dispersed over the entire thylakoid system, as expected (Fig. 14, Fig. 15). The predominant, if not exclusive, localisation of the CURT1 proteins in thylakoids regions of high curvature (in particular grana margins), together with the fact that the dose of CURT1 proteins controls the extent of grana stacking, leads us to conclude that CURT1 proteins are directly involved in causing the localised membrane curvature that results in grana margins.

### Intrinsic membrane-bending property of CURT1

The extent of thylakoid protein phosphorylation had previously been associated with changes in grana architecture (Fristedt, 2009). Therefore, TEM analysis was performed on chloroplasts displaying either hypo- *(stn7 stn8* mutant (Bellafiore, 2005; Bonardi, 2005)). or hyperphosphorylation (*tap38* mutant (Pribil, 2010; Shapiguzov, 2010)) of thylakoid proteins. Grana from *stn7 stn8* plants had fewer but broader membrane discs as reported (Fristedt, 2009) and were comparable to grana from CURT1A-HA plants expressing CURT1A at ∼50% of WT 10 levels (Fig. 12b,d). In contrast, grana stacks in *tap38* plants had more membrane layers than seen in WT (Fig. 12b,d).

Because a decrease in thylakoid phosphorylation appears to have a similar effect on grana architecture as decreasing CURT1 levels, we tested whether reduced thylakoid phosphorylation might cause the changes in grana architecture observed in the *curt1a* and *curt1abcd* mutants. However, the levels of PSII core and LHCII phosphorylation were actually higher in the two *curt1* mutants (Fig. 16), implying (i) that modulation of thylakoid membrane curvature by CURT1 proteins does not involve PSII core phosphorylation, and (ii) that loss of CURT1-dependent membrane curvature overrides the influence of PSII core phosphorylation.

To test whether CURT1 proteins can directly induce membrane curvature, CURT1A was translated in a cell-free system in the presence of liposomes with a thylakoid-like lipid composition. After purification of liposomes, TEM analysis allowed to recognise tubulation of CURT1A-containing liposomes (Fig. 17a), demonstrating that CURT1A can directly induce membrane curvature *in vitro.* CURT1A was found to be integrated into liposomes, as shown by Western analysis of purified liposomes (Fig. 17b). Moreover, trypsination experiments, employing an antibody that recognises the N-terminal domain of CURT1A (and which contains a trypsination site; see Fig. 3b), indicated that the N-terminus of CURT1A protrudes from the liposomal surface and is accessible to trypsination (Fig. 17b). Thus, the topology of CURT1A in liposomes is similar to the one of natural CURT1A with a stromal orientation of its N-terminus. To test whether liposomal CURT1A also forms oligomers, CURT1A proteoliposomes were crosslinked. Both membrane-permeable and impermeable crosslinkers were capable to produce crosslinking products, indicating that liposomal CURT1 proteins form oligomers, providing a link between the membrane-bending activity of CURT1 proteins and their capability to spontaneously oligomerise (Fig. 17c).

### CURT1 function is evolutionary conserved

Cyanobacteria have CURT1 proteins (Fig. 1d) but do not form grana. To clarify the impact of CURT1 proteins on cyanobacterial thylakoid structure, *Synechocystis* strains were generated displaying altered levels of the endogenous (synCURTI) or *Arabidopsis* (CURT1A) CURT1 protein. In fact, loss of synCURT had serious consequences for cell viability, because we failed to obtain fully segregating knock-out strains for *synCURT1.* However, we could generate strains which express CURT1A instead of synCURT1 (strain: CURT1A *syncurt1),* indicating that *Arabidopsis* CURT1 can replace *Synechocystis* CURT1 (Fig. 18a). Interestingly, strains that express CURT1A in addition to endogenous synCURT1 (strain: *CURT1A),* express less synCURT1 than WT and less CURT1A than *CURT1A syncurt1* (Fig. 18a), suggesting that mechanisms might operate in *Synechocystis* that prevent over-accumulation of CURT1 proteins. The growth rate of the two mutant strains, in particular the one of *CURT1A syncurt1,* was reduced compared to WT cells, indicating that *Arabidopsis* CURT1A does only partially replace *Synechocystis* CURT1. When thylakoid architecture was studied by TEM, multiple changes were observed in the strains with altered CURT1 levels (Fig. 18b). The thylakoids of the mutant strains had a crumpled appearance with a decreased average lumen width (WT, 23.8 ± 2.9 nm; *CURT1A, 19.5* ± 1.5 nm; CURT1A *syncurt1, 17.3* ± 2.9 nm; n>30 cells). Moreover, phycobilisomes were associated with WT thylakoids, but detached from the thylakoid membranes of the mutant strains. Taken together, these data suggest that already in cyanobacteria - the progenitors of chloroplasts, CURT1 proteins are involved in establishing thylakoid architecture. Strikingly, *Arabidopsis* CURT1A can partially replace *Synechocystis* CURT1.

### Effect of CURT1 depletion on photosynthesis

Mutants lacking one or more CURT1 proteins displayed essentially WT growth behavior and leaf coloration, indicating that thylakoid functions are not drastically impaired. Photosynthetic electron flow was not affected in *curt1b, curt1c* and *curt1d* plants (Table 2, Fig. 10a). However, in *curt1a* and even more so in double, triple and quadruple mutants lacking CURT1A, a decrease in the effective quantum yield (Φ_{II}) and an increase in excitation pressure (1-qL) (Kramer, 2004) and non-photochemical quenching (NPQ) were noted. The impairment in photosynthetic electron flow was pronounced at high light intensities (Fig. 10b). Maximum quantum yield of PSII (Fv/Fm) was WT-like (Fig. 10a, Table 2), indicating that electron flow through PSII is not affected. This interpretation was supported by analyses of light-induced fluorescence changes (Fig. 10c). Here, the initial fluorescence increase was identical in WT and *curt1abcd* plants, but the plateau following the initial rise was less pronounced in the mutant, indicating that the photochemical efficiency of PSII is unaltered but electron transfer to PSI from the plastoquinone pool (Rappaport, 2007) is slightly decreased in *curt1abcd* plants (Fig. 10c). This is consistent with the observation that P700⁺ rereduction was slightly slower in *curt1abcd* (Fig. 10d,e). Yet, the activity of PSI was not affected in *curt1abcd,* because the oxidation kinetics induced by a saturating light pulse were WT-like (Fig. 10e).

To further investigate the perturbation in photosynthesis, cyclic electron transport (CET) was analysed. In *curt1abcd,* CET was also decreased, as determined by *in situ* measurements in ruptured chloroplasts and by monitoring the transient induction of non-photochemical quenching (NPQ) (Fig. 10f,g). Quenching of chlorophyll fluorescence due to state transitions (qT), the reversible association of the mobile pool of LHCII proteins with PSII or PSI, was reduced but not completely abolished in plants lacking CURT1 proteins (WT, 0.13±0.01; *curt1abcd, 0.04±0.01*)*.*

### DISCUSSION

Multiple lines of evidence point to the conclusion that CURT1 proteins control thylakoid architecture by inducing membrane curvature (Fig. 19). 1. In *Arabidopsis,* levels of CURT1 proteins and of the number of membrane layers in grana stacks (and in turn of the area of grana margins, which are the sites of highest membrane curvature) are directly correlated; 2. CURT1 proteins are predominantly located in grana margins; and 3. CURT1 proteins are capable of inducing membrane curvature *in vitro.* Expression of *Arabidopsis* CURT1A in *Synechocystis* cells alters thylakoid structure, although whether reduced levels of endogenous synCURT1 or the action of the *Arabidopsis* protein are responsible remains to be clarified. In either case, the increased membrane curvature revealed by the crumpled appearance of thylakoids in these cells, and the decrease in lumen width, might lead to detachment of phycobilisomes due to steric hindrance of the protruding extrinsic antenna complexes. Moreover, because this steric hindrance due to phycobilisomes does also prevent the direct interaction of thylakoid membranes, grana formation by CURT1 overexpression might be actually feasible in *Synechocystis* strains without phycobilisomes.

A number of eukaryotic proteins are known to share th*e in vitro* vesicle/tubule-forming activity of CURT1A. Many of these possess either an ENTH (epsin N-terminal homology) domain (Kay, 1999; De Camilli, 2002; Ford, 2002) or a BAR (Bin-amphiphysin-Rvs) domain (Zimmerberg, 2006; Farsad, 2003)., both of which include an N-terminal amphipathic helix. Epsin's ability to curve membranes is dependent upon insertion of this helix into one leaflet of the membrane bilayer (Ford, 2002), and formation of highly ordered aggregates via self-association of the ENTH domain (Yoon, 2010). The membrane-deforming activity of N-terminal amphipathic helices in the prokaryotic MinE and the peroxisomal PEW11 proteins has been directly demonstrated (Shih, 2011; Opalinski, 2011).

With about 100 amino acids CURT1 proteins are considerably smaller than canonical ENTH (∼150 amino acids) or BAR (-200 amino acids) domains. However, CURT1 proteins appear to contain an amphipathic helix, which might be involved in its membrane-deforming activity (Fig. 2a, Fig. 1b,c). Moreover, their capacity for oligomerisation *in vivo* in thylakoids and *in vitro* in liposomes suggests that 13 aggregation of CURT1 might play a pivotal role in inducing membrane curvature, analogous to the mechanisms suggested for MinE (Shih, 2011) or epsin (Yoon, 2010).

Taken together, our data identify the CURT1 proteins as a novel family of proteins that form oligomers, can bend membranes *in vitro* and control the architecture of grana in plants. Their function in defining thylakoid structure can be traced back to the endosymbiotic precursor of chloroplasts and, in land plants, the regulation of their activity might involve their posttranslational modification by phosphorylation.

The role of protein phosphorylation in modulating thylakoid architecture remains enigmatic. Our results clearly show that CURT1-dependent membrane curvature overrides the influence of PSII core phosphorylation on grana stacking. Moreover, CURT1 proteins also seem to act in determining thylakoid structure in cyanobacteria, where no PSII core phosphorylation occurs. One possibility is that dephosphorylation of CURT1 proteins (in *stn7 stn8* mutants) might alter the structure of the predicted N-terminal amphipathic helix and/or its oligomerisation behaviour. This, in turn, could modify the membrane-bending activity of CURT1 proteins in the grana margins, leading to more extensive but fewer membrane layers in grana (Fig. 19). This model is supported by the fact that phosphorylated forms of CURT1A, B and C have been found in phosphorylomes (Table 4).

### METHODS

### Plant propagation

*Arabidopsis thaliana* plants were either grown on soil in a growth chamber, or, when non-photoautotrophic, on MS medium supplemented with sucrose (Pesaresi, 2009).

### Chemical crosslinking

Thylakoid preparations (300 µg/ml chlorophyll) were washed five times with 20 mM HEPES/KOH (pH 7.5) to remove EDTA. After addition of bis(sulfosuccinimidyl) suberate (BS3; final concentration 4 mM, Thermo Scientific) and incubation for 1 h at 0°C the reaction was quenched by adding 150 mM Tris/HCl (pH 7.5). CURT1A proteoliposomes were crosslinked for 1 h on ice with either 0.5 mM and 2.0 mM DSP (dithio-bis[succinimidylpropionate]) or 0.5 mM and 2.0 mM DTSSP (3,3'- dithio-bis[sulfosuccinimidylpropionate]) (Pierce, Thermo Scientific). Subsequently the reaction was quenched at a final concentration of 30 mM Tris (pH 7.6). Samples were resuspended in non-reducing Laemmli buffer and heated for 10 min at 100°C prior to Tris-Tricine PAGE.

### Co-immunoprecipitation (CoIP)

For CoIP experiments with HA- or cmyc-tagged proteins, thylakoid membranes were solubilised with digitonin and the supernatant was recovered after centrifugation (60 min, 16,000 g, 4°C). The supernatant was co-incubated with HA or cmyc affinity beads and interactors were eluted after washing. For CoIP experiments with CURT1B, thylakoids were incubated for 2 h with a CURT1B-specific antibody before solubilisation and Protein-A beads were used instead of HA or cmyc beads.

### Electron microscopy

For transmission electron microscopy, micrographs were taken with an EM-912 electron microscope (Zeiss) equipped with an integrated OMEGA energy filter operated in the zeroloss mode. For scanning electron microscopy, envelope-free chloroplasts were examined with a Zeiss AURIGA® high-resolution field-emission scanning electron microscope. Topographic images were taken with the chamber SE detector. For immunogold-labelling detection, backscattered electron images were taken with the energy-selective in-lens (EsB) Detector, with the EsB grid set at 900 V. The immunogold signal was coloured in yellow and superimposed on the green-coloured topographic image of the chloroplasts.

### Plant material

Insertion lines for CURT1 genes originate from six different mutant collections (see Fig. 4a).

### Nucleic acid analysis

DNA and RNA analyses were performed as described (Armbruster, 2010). Primers for PCRs are listed in Table 1.

### Generation of tagged CURT1 proteins

Plants expressing CURT1A and D with a C-terminal HA or cmyc tag were generated by introducing the respective coding region into the vectors pPCV812ΔNotI-Pily (for hemag-glutinin (HA) fusions) or pPCV8124Δ*NotI*-Lola (for cmyc fusions) under control of the 35S promoter, and then transforming *curt1a-2* (CURT1A-HA or CURT1A-cmyc) and WT (CURT1D-HA) flowers with the constructs (Clough, 1998).. Individual transgenic plants were identified as described (Armbruster, 2010).

### Protein isolation, PAGE and immunoblot analyses

Leaves harvested from 4-week-old plants were used for the preparation ofthylakoids (Bassi, 1985) and total proteins (Martinez-Garcia, 999). PSI isolation was performed as described (DalCorso, 2008).

For BN-PAGE, thylakoid samples equivalent to 30 µg (n-dodecyl β-D-maltoside, β-dim; Sigma) or 100 µg (digitonin, Sigma) Chl were solubilised in 750 mM 6-aminocaproic acid, 5 mM EDTA (pH 7), and 50 mM NaCl in the presence of 1.0% (w/v) β-DM or 1.6% (w/v) digitonin for 10 min (β-DM) or 60 min (digitonin) at 4°C. Following centrifugation (15 min (β-DM) or 60 min (digitonin); 21,000 g), the solubilised material was fractionated using nondenaturing BN-PAGE (Schägger, H., 1988). Designation of supercomplexes: PSI-LHCII, PSI-LHCI-LHCII supercomplex; PSIₘₒₙₒ, PSI-LHCI monomers; PSII_{di}, PSII dimers; Cyt b₆/f_{di}, dimeric Cytochrome *b₆*/*f* complexes; LHCII_{mono/tri}, monomeric/trimeric LHCII. 2D-PAGE analysis and immunoblot analysis with appropriate antibodies, signal detection and quantification was performed as described (Armbruster, 2010)

### Generation and characterisation of Synechocystis mutant strains

*Synechocystis sp. PCC 6803* glucose-tolerant wild-type and mutant strains were grown at 30°C in BG11 medium containing 5 mM glucose, under continuous illumination at 30 µmol m⁻² s⁻¹. For growing on plates, 1.5% (w/v) agar and 0.3% (w/v) sodium thiosulfate were added. In both cases, BG-11 was supplemented with antibiotics appropriate for the particular strain.

To generate the *CURT1A* strain, the coding region of *CURT1A* without cTP was introduced into the *Synechocystis slr0168* ORF (Kunert, 2000) together with a nptI-sacB double-selection cassette (Cai, 1990). To generate the CURT1A *syncurt1* strain, the endogenous *synCURT1* gene *(slr0483)* was replaced by the coding region of CURT1A without cTP, together with the double-selection cassette. In both strains, *CURT1A* was put under the control of the *Synechocystis psbA2* promoter (Eriksson, 2000). Vectors used for transformation were assembled with the Golden Gate Shuffling method (Engler, 2009), using the pL2-1 destination vector (Weber, 2011). All inserts were PCR amplified using primers with an additional BsaI restriction site at their 5' termini.

Transformation of *Synechocystis* sp. PCC 6803 was performed as described (Vermaas, 1987), and transformants were selected based on kanamycin resistance. Complete segregation of the introduced gene was confirmed by PCR.

For Western analyses, cells were harvested by centrifugation (5,000 g, 10 min, 4°C). Cells were then resuspended in 50 mM HEPES-NaOH pH 7.8, 10 mM MgCl₂, 5 mM CaCl₂ and 25% glycerol, and ruptured by five disrupting strokes of 20 sec using 0.25/0.3-mm glass beads. After centrifugation (5,000 g, 5 min, 4°C), the supernatant constitutes the total protein fraction. After subsequent centrifugation (45,000 g, 1.5 h, 4°C) the pellet representing the membrane fraction was recovered and suspended in one volume of the above mentioned buffer. Samples were solubilised at 70°C for 10 min in 2% w/v SDS in presence of 100 mM DTT prior to separation via 15% PA SDS Tris-Tricine gel. For Western analysis, antibodies raised against CURT1A or the peptide CTDVGPITTPNPQKS (SEQ ID NO: 11) of synCURT1 (BioGenes) were used.

### Preparation of proteoliposomes

For proteoliposome generation, unilamellar vesicles were prepared as described (Haferkamp, 2008). Briefly, a lipid mixture of 25% MGDG, 42% DGDG, 16% SQDG, and 17% PG dissolved in chloroform:methanol (1:1) (Lipid Products, UK) was dried under a stream of nitrogen and stored under vacuum overnight. The lipid film was slowly dissolved in DEPC-treated ddH2O to a concentration of 20 mg/ml and underwent 5 freeze-thaw cycles in liquid nitrogen. To obtain the final vesicle size, the lipid suspension was extruded 21 times at 250 kPa and 60°C through a 0.1 µm Nuclepore membrane (Whatman Ltd., UK). CURT1A protein was expressed using a S12 cell free expression system as described (Kim, 2006) with minor modifications. The reaction mix contained 20% (v/v) cell extract, no PEG 8000, and 200 µl of the liposome preparation. After 3 h of incubation in a 37°C shaker, the mix was separated by a discontinuous sucrose gradient (100,000 g, 18 h) and the liposome containing fraction extracted. For topology studies, the liposome fraction was mixed with trypsin (Sigma Aldrich, USA) and CaCl₂ to a final concentration of 0.25 mg/ml and 25 mM, respectively, and incubated for 20 min on ice. Samples were then analysed by SDS-PAGE and Western analysis.

### Electron microscopy

Plant material (pieces of tissue from 5-7^{th} true leaves of 28-day-old plants at the beginning of the light period) and *Synechocystis* cells were fixed with 2.5% glutaraldehyde in 75 mM sodium cacodylate, 2 mM MgCl₂ (pH 7.0) for 1 h at 25 °C, rinsed several times in fixative buffer, and postfixed (2 h) with 1% osmium tetroxide in fixative buffer at 25°C. After two washing steps in distilled water, the pieces were stained en bloc with 1% uranyl acetate in 20% acetone (30 min), followed by dehydration and embedding in Spurr's low-viscosity resin. Ultrathin sections of 50-70 nm were cut with a diamond knife and mounted on uncoated copper grids. The sections were poststained with aqueous lead citrate (100 mM, pH 13.0).

Isolated chloroplasts were made envelope-free by one freeze-thaw cycle in liquid nitrogen. Incubation with specific antibodies was carried out in a 1:1 dilution with 50 mM HEPES/KOH (pH 8.0), 330 mM sorbitol, 150 mM NaCl, 0.5% (w/v) BSA (overnight, 4°C). Unbound antibody was removed by washing with the same buffer (centrifugation at 0.2 g for 2.5 min) and gold-coated secondary antibody was added and incubated (60 min, 4°C), followed by removal of unbound secondary antibody by repeated washing.

For scanning electron microscopy, drops of chloroplast suspensions were placed on pieces of conductive silicon wafer, covered with a coverslip and rapidly frozen with liquid nitrogen. The coverslip was removed, and the slide immediately fixed with 2.5% glutaraldehyde in iso-osmotic cacodylate buffer (pH 7.0), postfixed with osmium tetroxide, dehydrated in a graded series of acetone solutions and critical-point dried from liquid CO₂, mounted on stubs, and examined uncoated at 1 kV with the Zeiss AURIGA®.

### Bioinformatic analyses

Amino acid sequences were aligned using ClustalW (v2.0.12; http://mobyle.pasteur.fr/) (Thompson, 1994) and BoxShade (v3.21; http://www.ch.embnet.org). Chloroplast transit peptide sequences were predicted with ChloroP (http://www.cbs.dtu.dk/services/ChloroP/) (Emanuelsson, 1999), transmembrane domains with TMHMM (http://www.cbs.dtu.dk/services/TMHMM-2.0/) (Krogh, 2001). Amphipathic properties of α-helices were illustrated by helical wheel representations using Java Applet (http:// cti.itc.virginia.edu/∼cmg/Demo/wheel/wheelApp.html). Representation of the CURT1A-H1 sequence as a 3D model of an α-helix was computed by I-TASSER (Zhang, 2008) and processed by 3D-Mol Viewer (Invitrogen).

### Determination of the topology of CURT1 proteins

Intact *Arabidopsis* chloroplasts were isolated and purified from leaves of 4- to 5-week-old plants (Aronsson, 2002), broken open in 10 mM Tris/ HCl, pH 8.0; 1 mM EDTA pH 8.0 at a Chl concentration of 2 mg/ml, and loaded onto a three-step sucrose gradient (Li, 1991). After centrifugation (30,000 g; 4°C), the upper phase (containing the stroma), both interphases (with the envelope membranes) and the pellet (thylakoids) were collected, and all three membrane fractions were washed with TE buffer.

Salt washes of thylakoids were performed as described (Karnauchov, 1997). Soluble and membrane proteins were separated by centrifugation for 10 min (10,000 g, 4°C), and immunoblot analysis was performed on both fractions.

For tryptic proteolysis experiments, thylakoid membranes were resuspended in 50 mM HEPES/KOH (pH 8.0) and 300 mM sorbitol at a Chl concentration of 0.5 mg/ml. Trypsin (Sigma) was added to a concentration of 10 µg/ml. Samples were taken 30 min later and proteolysis was stopped by adding soybean trypsin inhibitor (50 µg/ml) (Sigma).

### Measurement of photosynthetic parameters

Maximum (Fv/Fm) and effective (ΦII) quantum yields of PSII, excitation pressure (1-qL), nonphotochemical quenching (NPQ) and state transitions were measured as described (DalCorso, 2008, Kramer, 2004, Pribil, 2010, Varotto 2000).

Fast light-induced absorption and fluorescence changes were measured with a JTS10 (Biologic). Cut plant leaves, dark-adapted for at least 1 h, were used and all experiments were performed with at least three leaves from independent plants. The absorption changes reflecting the redox changes of P700 were measured at 810 nm. The actinic light was provided by an orange LED and the light intensity was 7000 and 100 µmol photons m⁻²s⁻¹ for the absorption and fluorescence experiments, respectively.

### In-situ assay of ferredoxin-dependent plastoquinone reduction activity

Ferredoxin-dependent plastoquinone reduction activity was measured in ruptured chloroplasts as described (Munekage, 2002).

### Generation of MBP fusions and protein titration

Sequences coding for the mature proteins PetC, PsaD1, CURT1A, B, C and D were directionally cloned into pMal-c2 (New England Biolab) downstream of the MBP coding sequence. Fusion proteins were expressed in the *E*. *coli* strain BL21 and purified under native conditions with amylose-resin (New England Biolabs). Purified proteins were quantified (Protein Assay Kit, Bio-Rad) and concentrations of recombinant proteins were calculated. Total amounts of the respective proteins in thylakoid membrane preparations were estimated by quantifying the thylakoid protein signal with respect to signals of dilution series of the recombinant proteins using IMAGE QUANT (Molecular Dynamics). Signals were obtained by protein blot analysis with the appropriate antibodies.

### Leaf lipid quantification

Total lipids were extracted from whole leaves with chloroform/methanol. After removal of solvent with nitrogen gas in a sample concentrator, total leaf lipids were transmethylated with methanolic HCl in the presence of pentadecanoic acid (15:0) as internal standard. After extraction with hexane, fatty acid methyl esters were quantified by gas chromatography as described (Browse, 1986).

### Intracellular localisation of dsRED fusions in Arabidopsis protoplasts

The red fluorescent protein from the reef coral *Discosoma* (dsRED) was used as a reporter to determine the intracellular location of CURT1 proteins as described (DalCorso, 2008, Jach, 2001).

### Electron microscopy of liposomes

For negative staining of liposomes, a drop of the sample was placed on a carbon-coated copper grid, freshly hydrophilized by glow discharge. After incubation for 2 min, the drop was quickly removed with a pasteur pipette and the grid was stained with 2% phosphotungstic acid and 0.05% glucose.

### TABLES

The following primers of Table 1 A designated CURT1A, B, C and D are used with respect to *Arabidopsis thaliana* CURT1 polypeptides.

**Table 1 A. Oligonucleotides used for PCR and RT-PCR**

| Gene | Forward primer (5'→3') | Reverse primer (5'→3') | experiment |
|---|---|---|---|
| CURT1A | CGTCCCGGGATGGCGATATCA | GCCAGATCTTTCGCTTCCTGC | Fusion to HA/cmyc |
| | CGGAATTCATGCCTTCTTCAGAAG | GCTCTAGACTATTCGCTTCCTGCG | Fusion to MBP |
| | CGCCATGGCGATATCAGTAGCAGC | CGCCATGGCGCTTCCTGCGATCTTCT | Fusion to RFP |
| | AACACCACTGAGTTGGATTTC | AACATACAGAGAGAAAGCTGG | Genotyping |
| | AAGAAGAAGTTCTCGGCGTG | GACGAGGTCTCTTCTGAAGA | RT-PCR |
| | GGATGGTCTTGAGAAC | GCTTCCTGCGATCTTCTTCT | RT-PCR |
| | CTCGAATAGTGAAGCTCCTC | CACTCACTATCAGACCCAAG | RT-PCR |
| CURT1B | CGGAATTCATGGCCGCTAAAG | CCTCTAGACTAGCTGCTCCC | Fusion to MBP |
| | ACTCCAAGATCGTCTCTACC | CATGCATGGITAGCTTAGCTT | Genotyping |
| | AAAGCCACTGCTTACTGTCGG | GCTGCTCCCTAATATGTTT | RNA blot analysis |
| CURT1C | CGGAATTCATGGCTAGTGGAGA | GCTCTAGATCACTGGCCAAGTA | Fusion to MBP |
| | CGCCATGGCTTCAATTTCTGCA | CGCCATGGCCTGGCCAAGTATATCC | Fusion to RFP |
| | CAACTTTGCCTTCGCCATTGT | CTGGCCAAGTATATCCGCTA | Genotyping |
| | CAACTTTGCCTTCGCCATTGT | GCTGCTAGGGTTTCGAGTAA | RT-PCR |
| | TGGGCATCATTGAATCTCATC | CTGGCCAAGTATATCCGCTA | RT-PCR |
| CURT1D | CGCCCGGGATGGAGCTCTGCACCA | GCAGATCTCTCACTATCAGACCC | Fusion to HA-cmyc |
| | CGGAATTCATGTGTAGTAGAAAG | GCTCTAGATCACTCACTATCAG | Fusion to MBP |
| | CGCCATGGAGCTCTGCACCA | CGCCATGGCCTCACTATCAGACCC | Fusion to RFP |
| | TGCACCAGGTCTACCACAAT | TTGAGTTTCCTCATCTTCAGC | Genotyping |
| | TGCACCAGGTCTACCACAAT | GCAACAACTACTCCATCACG | RT-PCR |
| | CTCGAATAGTGAAGCTCCTC | CACTCACTATCAGACCCAAG | RT-PCR |
| PetC | GCGAATTCGCGTCGAGTATTCCA | GCTCTAGATTAAGACCACCATGGA | Fusion to MBP |
| PsaD1 | GCGAATTCATGGCCGAGAAAACA | GCTCTAGATCACAAATCATAACTTTG | Fusion to MBP |
| ACTIN1 | TGCGACAATGGAACTGGAATG | AGCATGTGGAAGTGCATACC | RT-PCR |

The following primers of Table 1 B are used for generating Synechocystis constructs (see the experiment regarding Synechocystis).

**Table 1 B. Oligonucleotides used for PCR and RT-PCR**

| Gene | Forward primer (5' → 3') | Reverse primer (3' → 5') | name |
|---|---|---|---|
| CURT1A | TTTGGTCTCTAGGTTGCTCA- GCAGTGACCTATTC | TTTGGTCTCTTGGGGCCACTGT- TATTTTGATTG | PG UR |
| | TTTGGTCTCTCCCATG- GAAAAAACGACAATTAC | TTTGGTCTCTTTGGTTATAATT- CCTTATGTATTTG | psbA2P FW COE_P RV |
| | TTTGGTCTCTCCAAATGGCTT- CTTCAGAAGAGACCTC | TTTGGTCTCTAACGCTATT- CGCTTCCTGCGATCTTC | CURT1A FW |
| | | | CURT1A2 RV |
| | TTTGGTCTCACGTTGGAATT- CGATTGATCCGTCGAC | TTTGGTCTCCCATACTT- TAGGCCCGTAGTCTGCA | selection cassette |
| | TTTGGTCTCTTATGCAATTTCGTT- TGCGAATTTAC | TTTGGTCTCTAAGCATAAAATAC- CTTCCCATC | PG DR2 |
| CURT1A *syncurt1* | TTTGGTCTCTAGGTTTTTTGAC- CTATCTGGGTGAAG | TTTGGTCTCTAACGAGCTTCCCA- TATTGGGGC | CURT UR |
| | TTTGGTCTCACGTTGGAATT- CGATTGATCCGTCGAC | TTTGGTCTCCCATACTT- TAGGCCCGTAGTCTGCA | selection cassette |
| | TTTGGTCTCTTATGCCCATG- GAAAAAACGACAATTAC | TTTGGTCTCTTTGGTTATAATT- CCTTATGTATTTG | COE_P |
| | TTTGGTCTCTCCAAATGGCTT- CTTCAGAAGAGACCTC | TTTGGTCTCTGACACTATT- CGCTTCCTGCGATCTTC | CURT1A |
| | TTTGGTCTCATGTCTCCAGAC- CGCCCCAG | TTTGGTCTCTAAGCCAAAT- GCCATTCCTGGGCG | CURT DR |

The primer sequences have been assigned the SEQ ID NOs: 12 to 79, respectively, in the order of their appearance, starting with CGTCCCGGGATGGCGATATCA and ending with TTTGGTCTCTAAGCCAAATGCCATTCCTGGGCG. The forward primers have an even SEQ ID number, whereas the reverse primers have an uneven SEQ ID number. Each primer pair (adjacent forward and reverse primers) has subsequent SEQ ID numbers. Where the names of the primers of a primer pair are different, the designations FW (forward) and RV (reverse) have been added.

**Table 2. Chlorophyll fluorescence parameters measured in wild-type and curt1 mutant leaves**

| | Fᵥ/Fₘ | Φ_{II} | NPQ | 1-qL |
|---|---|---|---|---|
| *Ler* | 0.83 ± 0.01 | 0.74 ± 0.01 | 0.27 ± 0.01 | 0.26 ± 0.01 |
| No-0 | 0.83 ± 0.01 | 0.74 ± 0.02 | 0.23 ± 0.04 | 0.25 ± 0.04 |
| Col-0 | 0.84 ± 0.01 | 0.74 ± 0.01 | 0.24 ± 0.02 | 0.26 +0.02 |
| *curt1a-1* | 0.83 ± 0.01 | 0.70 ± 0.03 | 0.19 ± 0.03 | 0.43 ± 0.03 |
| *curt1a-2* | 0.84 ± 0.01 | 0.71 ± 0.03 | 0.19 ± 0.03 | 0.40 ± 0.03 |
| *curt1b-1* | 0.84 ± 0.01 | 0.75 ± 0.02 | 0.23 ± 0.02 | 0.22 ± 0.02 |
| *curtlc-1* | 0.83 ± 0.01 | 0.73 ± 0.02 | 0.25 ± 0.04 | 0.26 ± 0.04 |
| *curt1d-1* | 0.83 ± 0.01 | 0.73 ± 0.01 | 0.23 ± 0.02 | 0.24 ± 0.02 |
| *curt1ab* | 0.84 ± 0.01 | 0.65 ± 0.04 | *0.15* ± 0.04 | 0.57 ± 0.04 |
| *curt1ac* | 0.83 ± 0.01 | 0.65 ± 0.05 | 0.15 ± 0.02 | 0.48 ± 0.02 |
| *curt1abc* | 0.83 ± 0.01 | 0.66 ± 0.03 | 0.16 ± 0.04 | 0.55 ± 0.05 |
| *curt1abd* | 0.82 ± 0.01 | 0.65 ± 0.04 | 0.12 ± 0.03 | 0.48 ± 0.03 |
| *curt1abcd* | 0.83 ± 0.02 | 0.64 ± 0.02 | 0.15 ± 0.01 | 0.55 ± 0.01 |

Mean values for five plants (± SD) are shown. Fifth to seventh true leaves were chosen for measurements. To determine FV/FM, 0.8 s pulses of white light (5000 µmol photons m⁻² s⁻¹) were used. A 10 min illumination with actinic light (40 µmol photons m⁻² s⁻¹) was used to drive electron transport between PSII and PSI before measuring Φ_{II}, NPQ and 1-qL.

**Table 3. Diameter and height of grana in curt1 mutants and wild-type plants**

| Genotype | Diameter | Height |
|---|---|---|
| Col-0 | 0.446 ± 0.116 | 0.110 ± 0.039 |
| *Ler* | 0.408 ± 0.114 | 0.112 ± 0.041 |
| No-0 | 0.474 ± 0.114 | 0.114 ± 0.031 |
| *curt1d-1* | 0.466 ± 0.121 | 0.110 ± 0.030 |
| *curt1b-1* | 0.540 ± 0.142 | 0.113 ± 0.038 |
| *curt1c-1* | 0.558 ± 0.086 | 0.118 ± 0.036 |
| *curt1a-1* | 1.282 ± 0.375 | 0.084 ± 0.022 |
| *curt1a-2* | 1.357 ± 0.211 | 0.079 ± 0.027 |
| *curt1ac* | 1.678 ± 0.479 | 0.072 ± 0.050 |
| *curt1ab* | 1.615 ± 0.461 | 0.068 ± 0.020 |
| *curt1abc* | 1.652 ± 0.299 | 0.084 ± 0.022 |
| *curt1abcd* | 1.656 ± 0.414 | 0.057 ± 0.023 |
| *CURT1A-cmyc curt1a-2* | 0.374 ± 0.057 | 0.173 ± 0.050 |
| *CURT1A-HA curt1a-2* | 0.817 ± 0.233 | 0.085 ± 0.021 |
| *tap38* | 0.460 ± 0.080 | 0.146 ± 0.055 |
| *stn7 stn8* | 0.872 ± 0.192 | 0.089 ± 0.028 |

| | | |
|---|---|---|
| Mean values (± SD are given in µm. At least seven plants were analysed for each genotype. | | |

**Table 4. Proteomics-based data on subcellular localisation and phosphorylation of CURT1 proteins**

| | total chloroplast | | | thylakoids | | envelope | | phosphorylated |
|---|---|---|---|---|---|---|---|---|
| | 1 | 2 | 3 | 4 | 5 | 6 | 7 | PhosPhAt 3.0 |
| CURT1A | + | + | + | + | + | + | + | + |
| CURT1B | + | + | + | + | + | + | + | + |
| CURT1C | | + | + | + | + | | + | + |
| CURT1D | | | | + | | | | |

Data on the subcellular localisation of proteins were derived from proteomics studies by Rutschow, 2008 (1), Zybailov, 2008 (2), Kleffmann, 2004 (3), Peltier, 2004 (4), 2004 (5), Froehlich, 2003 (6) and Ferro, 2010 (7). Protein phosphorylation data are from the PhosPhAt 3.0 database (http://phosphat.mpimp-golm.mpg.de/). Note that mature CURT1A and B have phosphorylation sites N-terminal to the two TMs and sites C-terminal to the two TMs are found in CURT1C. Because the vast majority of thylakoid proteins are phosphorylated in their stroma-exposed regions (Pesaresi, 2011), this argues in favour of a topology of CURT1 proteins in which both N- and C-termini face the stroma (see Fig. 3).

**Table 5 Fatty acid composition of Arabidopsis leaf lipids**

| Fatty Acid | Col-0 | Ler | No-0 | *curt1a* | *curt1ab* | *curt1abcd* |
|---|---|---|---|---|---|---|
| 16:0 | 19.6 ± 1.0 | 18.5 ±1.9 | 17.9 ± 0.5 | 19.6 ± 0.8 | 21.5 ± 2.3 | 20.5 ± 1.9 |
| 16:1 | 3.3 ± 0.1 | 3.0 ± 0.2 | 3.0 ± 0.1 | 3.1 ± 0.2 | 3.6 ± 0.4 | 3.2 ± 0.5 |
| 16:3 | 11.8 ± 0.3 | 13.5 ± 1.6 | 12.9 ± 1.4 | 11.8 ± 1.8 | 10.4 ± 1.4 | 11.6 ± 1.2 |
| 18:0 | 9.5 ± 1.1 | 8.2 ± 1.1 | 8.2 ± 0.4 | 9.6 ± 0.6 | 11.0 ± 1.6 | 11.1 ± 1.9 |
| 18:1 | 2.9 ± 0.1 | 3.0 ± 0.3 | 2.9 ± 0.3 | 3.0 ± 0.3 | 3.1 ± 0.1 | 2.9 ± 0.1 |
| 18:2 | 12.6 ± 0.4 | 10.6 ± 0.3 | 11.4 ± 1.1 | 11.0 ± 1.3 | 11.1 ± 0.2 | 10.5 ± 0.5 |
| 18:3 | 40.3 ± *1.5* | 43.4 ±1.9 | 43.8 ± 0.8 | 41.9 ± 1.4 | 39.4 ± 3.1 | 40.3 ± 2.5 |

| | | | | | | |
|---|---|---|---|---|---|---|
| Mean values (± SD) of three measurements are shown and expressed as mol%. | | | | | | |

### REFERENCES

Albertsson, P. A. & Andreasson, E. Physiol Plant 121, 334-342 (2004).
Alonso, J. M. et al. Science 301, 653-657 (2003)
Anderson, J. M. Ann Rev Plant Physiol Plant Mol Biol 37, 93-136 (1986).
Anderson, J. M. et al. Photosynth Res 98, 575-587 (2008).
Armbruster, U. et al. Plant Cell 22, 3439-3460 (2010).
Aronsson, H. & Jarvis, P. FEBS Lett 529, 215-220 (2002)
Bassi, R. et al. Eur J Biochem 146, 589-595 (1985).
Bellafiore, S. et al. Nature 433, 892-895 (2005).
Biehl, A. Gene 344, 33-41 (2005).
Bonardi, V. et al. Nature 437, 1179-1182 (2005).
Browse, J., et al. Anal Biochem 152, 141-145 (1986)
Cai, Y. P. & Wolk, C. P. J Bacteriol 172, 3138-3145 (1990).
Clough, S. J. & Bent, A. F. Plant J 16, 735-743 (1998)
Cole, C., et al. Nucleic Acids Res 36, W197-20 (2008)
DalCorso, G. et al. Cell 132, 273-285 (2008)
Daum, B. & Kühlbrandt, W. J Ex.pBot (2011)
De Camilli, P. et al. FEBS Lett 513, 11-18 (2002)
Dekker, J. P. & Boekema, E. J. Biochim Biophys Acta 1706, 12-39 (2005)
Emanuelsson, O., et al.. Protein Sci 8, 978-984 (1999)
Engler, Cet al. PLoS One 4, e5553 (2009)
Eriksson, J., et al. Mol Cell Biol Res Commun 3, 292-298 (2000)
Farsad, K. & De Camilli, P. Curr Opin Cell Biol 15, 372-381 (2003)
Ferro, M. et al.. Mol CellProteomics 9, 1063-1084 (2010)
Ford, M. G. et al. Nature 419, 361- 366 (2002).
Friso, G. et al.. Plant Cell 16, 478-499 (2004)
Fristedt, R. et al. Plant Cell 21, 3950-3964 (2009)
Froehlich, J. E. et al. J Proteome Res 2, 413-425 (2003)
Gatz et al., Plant J 2, 397-404 (1992)
Granvogl, B., et al.. Proteomics 6, 3681-3695 (2006)
Haferkamp, S. & Kirchhoff, H. Photosynth Res 95, 129-134 (2008)
Hansson, M. & Vener, A. V. Mol Cell Proteomics 2, 550-559 (2003)
Holtorf S. et al. Plant Mol. Biol. 29, 637-646 (1995)
Ito, T. et al. Plant Physiol 129, 1695-1699 (2002)
Jach, G., et al. Plant J 28, 483-491 (2001)
Karnauchov, I., et al. FEBS Lett 408, 206-210 (1997)
Kay R. et al. Science 236, 1299-1307 (1987)
Kay, B. K., et al. Protein Sci 8, 435-438 (1999)
Khrouchtchova, A. et al. FEBS Lett 579, 4808-4812 (2005)
Kim, T. W. et al. J Biotechnol 126, 554-561 (2006)
Kirchhoff, H., et al. Biochemistry 41, 4872-4882 (2002)
Kleffmann, T. et al. Curr Biol 14, 354-362 (2004)
Kramer, D. M., et al.. Photosynths Res 79, 209-218 (2004
Krogh, A., et al. Journal of molecular biology 305, 567-580 (2001)
Kunert, A. et al. J Microbiol Methods 41, 185-194 (2000)
Li, H. M., Moore, T. & Keegstra, K. Plant Cell 3, 709-717 (1991)
Martinez-Garcia, J. F. et al. Plant J 20,251-257 (1999)
Mullineaux, C. W. Trends Plant Sci 10, 521-525 (2005)
Munekage, Y. et al. Cell 110, 361-371 (2002)
Mustardy, L. & Garab, G Trends Plant Sci 8, 117-122 (2003)
Nain V. et al., Indian Journal of Experimental Biology 46, 207-211 (2008)
Obayashi, T. et al. Nucleic Acids Res 35, D863-869 (2007)
Opalinski, L., et al. EMBO J 30, 5-16 (2011)
Parinov, S. et al. Plant Cell 11, 2263-2270 (1999)
Peltier, J. B., et al. J Biol Chem 279, 49367-49383 (2004)
Peng, L., et al. J Biol Chem 283, 34873-34879 (2008)
Pesaresi, P. et al. Mol Plant 2, 236-248 (2009)
Pesaresi, P. et al. Plant Cell 21, 2402-2423 (2009)
Pesaresi, P., et al. Biochim Biophys Acta 1807, 887-896 (2011).
Pribil, M., et al. PLoS Biol 8, e1000288 (2010)
Rappaport, F., et al. Biochim Biophys Acta 1767, 56-65 (2007)
Roy, A., et al. Nat Protoc 5, 725-738 (2010)
Rutschow, H., et al. Plant Physiol 148, 156-175 (2008)
Sambrook et al., Molecular Cloning: A Laboratory Manual Cold Spring Harbor Laboratory Press (2001)
Samson, F. et al. Nucleic Acids Res 30, 94-97 (2002)
Schägger, H., et al. Anal Biochem 173, 201-205 (1988).
Schwenkert, S. et al. J Biol Chem 281, 34227-34238 (2006)
Sessions, A. et al. Plant Cell 14, 2985-2994 (2002)
Shapiguzov, A. et al. Proc Natl Acad Sci U S A 107, 4782-4787 (2010)
Shih, Y. L. et al. PLoS One 6, e21425 (2011)
Song P. et al., The Journal of Cotton Science 4, 217-223 (2000)
Song, G.-q. et al. Journal of the American Society for Horticultural Science 132, 551-556 (2007)
Sundaresan, V. et al. Genes Dev 9, 1797-1810 (1995)
Thiel, T., Chapter 19: Genetic analysis of cyanobacteria, in "The Molecular Biology of Cyanobacteria" edited by D.A. Bryant. P. 582-606, Kluwer Academic Publishers (1994).
Thompson, J. D., et al. Nucleic Acids Res 22, 4673-4680 (1994)
Trissl, H. W. & Wilhelm, C. Trends Biochem Sci 18, 415-419 (1993)
Yoon, Y. et al. J Biol Chem 285, 531-540 (2010)
Varotto, C. et al. PlantJ22, 115-124 (2000)
Vermaas, W. F. J., et al. J. Plant Mol Biol 8, 317-326 (1987)
Weber, E. et al. PLoS One 6, e16765 (2011)
Zhang, Y. I-TASSER BMC Bioinformatics 9, 40 (2008)
Zimmerberg, J. & Kozlov, M. M. Nat Rev Mol Cell Biol 7, 9-19 (2006)
Zybailov, B. et al. PLoS ONE 3, e1994 (2008)

## Claims

1. A method of enhancing photosynthesis in a cell of a photosynthetic eukaryote or in a photosynthetic eukaryote comprising the step of transforming the cell of the photosynthetic eukaryote or the photosynthetic eukaryote with a polynucleotide encoding a CURT1 polypeptide, wherein the cell of the photosynthetic eukaryote or the photosynthetic eukaryote expresses the polynucleotide.

2. The method of claim 1, wherein the cell of a photosynthetic eukaryote is a regenerable cell and wherein the photosynthetic eukaryote is regenerated from the transformed regenerable cell of the photosynthetic eukaryote.

3. A method of enhancing photosynthesis in a cell of a photosynthetic eukaryote or in a photosynthetic eukaryote comprising the step of increasing the expression of a CURT1 polynucleotide endogenous to the cell of a photosynthetic eukaryote or to the photosynthetic eukaryote in said cell or photosynthetic eukaryote.

4. The method according to any one of claims 1 to 3, wherein the enhancement of photosynthesis results in an increase of growth and/or yield of the photosynthetic eukaryote.

5. An organism having an enhanced CURT1 expression as compared to a control organism.

6. The organism according to claim 5, wherein the expression of an endogenous CURT1 polynucleotide in a cell is enhanced or wherein a cell is transformed with a polynucleotide encoding a CURT1 polypeptide.

7. The organism according to claim 6, wherein the cell is stably transformed with a CURT1 polynucleotide.

8. The organism according to any one of claims 5 to 7 which is a photosynthetic eukaryote, preferably a plant or an alga, or is a cyanobacterium.

9. A cyanobacterium comprising a polynucleotide encoding a CURT1 polypeptide.

10. The organism according to claim 8, wherein the plant is a dicotyledonous, monocotyledonous or eudicotyledonous plant.

11. The method according to any one of claims 1 to 4 or the organism according to claim 8 or 10, wherein the plant is selected from the group consisting of
(a) Leguminosae, particularly pea, alfalfa, soybean, bean or peanut;
(b) Umbelliferae, particularly carrot or celery;
(c) Solanaceae, particularly tomato, potato, aubergine, tobacco, or pepper;
(d) Cruciferae, particularly the genus Brassica, for example oilseed rape, beet, cabbage, cauliflower, broccoli or thale cress *(Arabidopsis thaliana);*
(e) Compositae, particularly lettuce;
(f) Malvaceae, particularly cotton;
(g) Amaranthaceae, particularly sugar beet or beet root;
(h) Cucurbitaceae, particularly zucchini, squash, melon or cucumber;
(i) wheat, barley, sorghum, millet, rye, triticale, maize, rice, oats and sugarcane;
(ji) apple, pear, quince, plum, cherry, peach, nectarine, apricot, papaya, mango, citrus species, poplar, pine, sequoia, cedar or oak;
(k) herb species, particularly basil, thyme, sage, chives or parsley; and
(1) berry species, particularly strawberries, Ribes species, Vaccinium species or Rubus species.

12. A method of producing the organism according to any one of claims 5 to 8, 10 or 11 or the cyanobacterium according to claim 9, comprising transforming a cell of the organism or the organism or the cyanobacterium with a polynucleotide encoding a CURT1 polypeptide, wherein the organism or cyanobacterium expresses the polynucleotide.

13. The method of claim 12, further comprising generating from the cell a whole organism, whereby the organism is a multicellular organism.

14. A seed with a stably transformed polynucleotide encoding a CURT1 polypeptide, wherein the seed is produced by the plant according to any one of claims 8, 10 or 11, wherein a cell of the plant is stably transformed with a polynucleotide encoding a CURT1 polypeptide.

15. Use of a polynucleotide encoding a CURT1 polypeptide for enhancing photosynthesis of a cell of a photosynthetic eukaryote or of a photosynthetic eukaryote.

16. The method according to any one of claims 1 to 4 or 11 to 13, the organism according to any one of claims 5 to 8, 10 or 11, the cyanobacterium according to claim 9, the seed according to claim 14 or the use according to claim 15, wherein the CURT1 polypeptide is at least one of CURT1A, CURT1B, CURT1C or CURT1D, preferably wherein the CURT1 polypeptide is CURT1A, CURT1B, CURT1C and CURT1D.

17. The method according to any one of claims 1 to 4, 11 to 13 or 16, the organism according to any one of claims 5 to 8, 10, 11 or 16, the cyanobacterium according to claim 9 or 16, the seed according to claim 14 or 16 or the use according to claim 15 or 16, wherein the CURT1 polypeptide is from the genus *Arabidopsis,* in particular *Arabidopsis thaliana.*

18. The method according to any one of claims 1 to 4, 11 to 13, 16 or 17, the organism according to any one of claims 8, 10, 11, 16 or 17, the seed according to any one of claims 14, 16 or 17 or the use according to any one of claims 15 to 17, wherein the cell of a photosynthetic eukaryote or the photosynthetic eukaryote is grown under conditions of shade or low light.

19. The method, organism, seed or use according to any of the preceding claims, wherein the polynucleotide encoding the CURT1 polypeptide is under the control of an inducible or tissue specific promoter.
